# EUROPEAN PATENT APPLICATION

(11) **EP 1 489 172 A1**
(43) Date of publication of application: **22.12.2004**
(21) Application number: 03712914.5
(22) Date of filing: 25.03.2003
(51) Int. Cl.: C12N 15/09, A01K 67/027, A61K 45/00, A61P 3/06, A61P 9/00, A61P 9/10, A61P 19/10, A61P 25/14, A61P 25/28, A61P 35/00, A61P 37/02, C07K 14/72, C12P 21/02, C12Q 1/02, C12Q 1/68, G01N 33/15, G01N 33/50, G01N 33/53, G01N 33/566, A61K 31/711

(54) **NUCLEAR RECEPTOR ERR GAMMA 3**

(30) Priority: 25.03.2002 JP 2002084560
(71) Applicant: FUJISAWA PHARMACEUTICAL CO., LTD., Osaka-shi Osaka 541-8514 (JP)
(72) Inventor: KOJO, H., Berumezon-kamogawa 105, 262, Demizu-cho, Kamigyo-ku Kyoto-shi, Kyoto 602-0862 (JP); TAJIMA, Kaoru, Yorii-cho, Oozato-gun, Saitama 369-1203 (JP); FUKAGAWA, M, c/o Fujisawa Pharmaceutical Co., Ltd., Chuo-ku, Osaka-shi, Osaka 541-8514 (JP); NISHIMURA, S., c/o Fujisawa Pharmaceutical Co. Ltd, Chuo-ku, Osaka-shi, Osaka 541-8514 (JP); ISOGAI, Takao, Ami-machi, Inashiki-gun, Ibaraki 300-030 (JP)
(74) Representative: Gille Hrabal Struck Neidlein Prop Roos
(86) International application number: PCT/JP2003/003611
(87) International publication number: WO 2003/080831

(57) **Abstract**

The present invention relates to novel nuclear receptor ERRγ3. Although ERRγ3 itself lacks a DNA binding domain, it comprises the function of enhancing the transcriptional activation function of arbitrary nuclear receptors, such as ERR, ER, or TR. Moreover, the present inventors found that, like ERRγ3, the known proteins ERRγ1 and ERRγ2 also comprise the function of enhancing the transcriptional activation function of other nuclear receptors. Thus, the present invention provides methods for evaluating the regulatory function of these ERRγ subtypes in enhancing the transcriptional activation function of other nuclear receptors, and screening methods based on these evaluation methods.

## Description

### Technical Field

The present invention relates to novel nuclear receptors, and their use.

### Background Art

Nuclear receptors constitute a superfamily of a group of transcription factors whose activities are induced in a ligand-dependent manner. Nuclear receptors have specific structures, including a DNA-binding domain and a ligand-binding domain, which are highly homologous among family members.

By taking advantage of this high sequence homology, a number of novel nuclear receptor genes have been discovered. To date, forty or more nuclear receptor genes have been reported in humans. These include genes whose ligands have not yet been identified, and those that do not require a ligand to function. Estrogen receptor-related receptor (ERR) was first discovered as a gene encoding a novel nuclear receptor highly homologous to estrogen receptor (ER). There are three known subtypes of ERR: α, β, and γ. Of these, human ERRγ is known to have two isoforms, generated from differential splicing and giving rise to proteins with or without 23 amino acid residues at the N-terminal. These isoforms are defined as hERRγ1 (the short form) and hERRγ2 (the long form) (1. Eudy J.D., Yao S.F., Weston M.D., Edmonds M.M., Talmadge C.B., Cheng J.J., Kimberling W.J., and Sumegi J. "Isolation of a gene encoding a novel member of the nuclear receptor superfamily from the critical region of Usher syndrome type IIa at 1q41." Genomics 50: 382-384 (1998); Nagase T., Ishikawa K., Suyama M. , Kikuno R., Hirosawa M. , Miyaj ima N., Tanaka A. , Kotani H., Nomura N., and Ohara O. "Prediction of the coding sequences of unidentified human genes. XII. The complete sequences of 100 new cDNA clones from brain which code for large proteins *in vitro*." DNA Res. 5:355-364 (1998); Chen F., Zhang Q., McDonald T., Davidoff M.J., Bailey W., Bai C., Liu Q., and Caskey T. "Identification of two hERR2-related novel nuclear receptors utilizing bioinformatics and inverse PCR." Gene 228:101-109 (1999)).

In the case of human ERRγ subtypes, 5'-RACE revealed that at least six transcripts encoding the two isoforms, ERRγ1 and ERRγ2, exist tissue-dependently (Heard D.J., Norby P. L., Holloway J., and Vissing H. "Human ERRγ, a third member of the estrogen receptor-related receptor (ERR) subfamily of orphan nuclear receptors: tissue-specific isoforms are expressed during development and in the adult." Mol. Endocrinol. 14:382-392 (2000)).

The DNA binding domains of the three ERR subtypes, α, β, and γ, are 93% homologous or more. Each can recognize the estrogen response element (ERE) or its extended half site, and induce the transcription of downstream genes ligand-independently (Heard D.J., Norby P.L., Holloway J., and Vissing H. "Human ERR gamma, a third member of the estrogen receptor-related receptor (ERR) subfamily of orphan nuclear receptors: tissue-specific isoforms are expressed during development and in the adult." Mol. Endocrinol. 14:382-392 (2000) ; Hong H. , Yang L., and Stallcup M.R. "Hormone-independent transcriptional activation and coactivator binding by novel orphan nuclear receptor ERR3." J. Biol. Chem. 274:22618-22626 (1999)). Although ERRγ comprises the activity of controlling transcription ligand-independently, its ligand-binding domain is essential to this activity (Heard D.J., Norby P.L., Holloway J., and Vissing H. "Human ERRγ, a third member of the estrogen receptor-related receptor (ERR) subfamily of orphan nuclear receptors: tissue-specific isoforms are expressed during development and in the adult." Mol. Endocrinol. 14:382-392 (2000); Hong H., Yang L., and Stallcup M.R. "Hormone-independent transcriptional activation and coactivator binding by novel orphan nuclear receptor ERR3." J. Biol. Chem. 274:22618-22626 (1999)). Diethylstilbestrol, an ER agonist, was found to inhibit the transcriptional activation function of all three subtypes at concentrations of 10⁻⁶ M or higher (Tremblay G.B., Kunath T., Bergeron D., Lapointe L., Champigny C., Bader J.A., Rossant J., and Giguere V. "Diethylstilbestrol regulates trophoblast stem cell differentiation as a ligand of orphan nuclear receptor ERR beta." Genes & Dev. 15:833-838 (2001)), 4-hydroxytamoxifen, a partial agonist of ER, was found to inhibit the transcriptional activation function of ERRγ only at concentrations of 10⁻⁶ M or higher (Coward P., Lee D., Hull M.V., and Lehmann J.M. "4-hydroxytamoxifen binds to and deactivates the estrogen-related receptor gamma." Proc. Natl. Acad. Sci. U.S.A. 98:8880-8884 (2001)). Although the physiological function of ERR remains obscure, ERR has been suggested to play a role in controlling the function of ER since it cross-reacts with the ER response element (Eudy J.D., Yao S.F., Weston M.D., Edmonds M.M., Talmadge C.B., Cheng J.J., Kimberling W.J., and Sumegi J. "Isolation of a gene encoding a novel member of the nuclear receptor superfamily from the critical region of Usher syndrome type IIa at 1q41." Genomics 50:382-384 (1998); Yang N., Shigeta H., Shi H., and Teng C.T. "Estrogen-related receptor, hERR1, modulates estrogen receptor-mediated response of human lactoferrin gene promoter." J. Biol. Chem. 271:5795-5804 (1996)).

### Disclosure of the Invention

An objective of the present invention is to provide novel nuclear receptors capable of controlling the activities of other nuclear receptors. In addition, another objective of the present invention is to identify factors that regulate the transcription-controlling activity of nuclear receptors, and to provide methods for evaluating the activity of controlling the functions of such factors.

The present inventors attempted to find novel nuclear receptors by searching for genes that encode proteins containing a DNA binding domain (DBD) and a ligand-binding domain (LBD), which are characteristic to nuclear receptors. The inventors then analyzed the function of genes narrowed down in these searches. They found that some ERRγ isoforms were capable of regulating the transcription-controlling activity of other nuclear receptors, despite lacking part of the DBD themselves.

The present inventors then established assay systems for evaluating the function of regulating the transcription-controlling activity of the complexes formed between a nuclear receptor of every kind and an ERRγ isoform having a function of controlling the receptor's activity. The inventors also demonstrated that test substances capable of regulating the transcription-controlling activity of the aforementioned complexes can be selected using these systems, thus accomplishing the present invention.

Thus, the present invention relates to polynucleotides as described below, and proteins encoded by these polynucleotides. The present invention also relates to methods for measuring the transcription-controlling activity of complexes that comprise a second nuclear receptor, and ERRγ isoforms that can control the activity of this second nuclear receptor when coexisted. Furthermore, the invention relates to methods for evaluating the effect of test substances on the activity of the above complexes, based on these measuring methods.
[1] A polynucleotide of any one of the following (A) to (E):
   (A) a polynucleotide comprising a coding region of the nucleotide sequence of SEQ ID NO: 1;
   (B) a polynucleotide encoding a protein comprising the amino acid sequence of SEQ ID NO: 2;
   (C) a polynucleotide comprising an amino acid sequence wherein one or more amino acids are replaced, deleted, inserted, and/or added to the amino acid sequence of SEQ ID NO: 2, encoding a protein that:
      (a) controls the transcriptional activation function of a nuclear receptor when co-existed with the nuclear receptor; and
      (b) lacks at least part of a DNA binding domain;
   (D) a polynucleotide that hybridizes under stringent conditions with a polynucleotide comprising the nucleotide sequence of SEQ ID NO: 1, encoding a protein that:
      (a) controls the transcriptional activation function of a nuclear receptor when co-existed with the nuclear receptor; and
      (b) lacks at least part of a DNA binding domain;
   (E) a polynucleotide comprising a nucleotide sequence in which a sequence corresponding to nucleotides 599 to 715 of SEQ ID NO: 5 is deleted or replaced with another nucleotide sequence, and comprising a nucleotide sequence that comprises homology of 70% or more to the above nucleotide sequence in which the sequence corresponding to nucleotides 599 to 715 is deleted;
[2] the polynucleotide of [1], wherein the nuclear receptor in (a) belongs to a member selected from the group consisting of group A of subfamily 3, group B of subfamily 3, group C of subfamily 3, and group C of subfamily 1;
[3] the polynucleotide of [2], wherein the nuclear receptor in (a) is a nuclear receptor selected from the group consisting of estrogen receptor related receptor, estrogen receptor, and thyroid hormone receptor, wherein the polynucleotide encodes a protein that enhances the transcriptional activation function of the nuclear receptor;
[4] the polynucleotide of [2] that encodes a protein that reduces the transcriptional activation effect of a nuclear receptor, wherein the nuclear receptor is a glucocorticoid receptor;
[5] a protein encoded by the polynucleotide of [1];
[6] a vector comprising the polynucleotide of [1];
[7] a transformant carrying the polynucleotide of [1] or the vector of [5];
[8] a method for producing the protein of [5], comprising a step of culturing the transformant of [7], and recovering an expression product;
[9] a method of detecting the activity of a test substance in regulating the transcription-controlling activity of a nuclear receptor complex, wherein the complex comprises an arbitrary nuclear receptor and a protein encoded by the polynucleotide of any one of the following (A) to (E):
   (A) a polynucleotide comprising the coding region of the nucleotide sequence of any of SEQ ID NO: 1, 3, or 5;
   (B) a polynucleotide encoding a protein comprising the amino acid sequence of any of SEQ ID NO: 2, 4, or 6;
   (C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
   (D) a polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
   (E) a polynucleotide comprising homology of 80% or more with the nucleotide sequence of any of SEQ ID NO: 1, 3, or 5, encoding a protein that is functionally equivalent to a protein comprising the amino acid sequence of any of SEQ ID NO: 2, 4, or 6;
   wherein the method comprises the following steps:
   (1) contacting the test substance with a cell expressing the arbitrary nuclear receptor and the protein encoded by the polynucleotide of any one of (A) to (E) , where the cell carries an expression cassette in which a reporter gene is inserted downstream of a response element for the nuclear receptor;
   (2) culturing the above cell under conditions that allow expression of the nuclear receptor and the protein encoded by the polynucleotide of any one of (A) to (E), and measuring the expression level of the reporter gene in the cell; and
   (3) detecting the activity of the test substance in controlling the transcription-controlling activity of the above nuclear receptor, using the measurement result of step (2) as an index;
[10] the method of [9], wherein the nuclear receptor belongs to a member selected from the group consisting of group A of subfamily 3, group B of subfamily 3, group C of subfamily 3, and group C of subfamily 1;
[11] the method of [10], wherein the nuclear receptor is a nuclear receptor selected from the group consisting of estrogen receptor related receptor, estrogen receptor, and thyroid hormone receptor;
[12] the method of [10], wherein the nuclear receptor is a glucocorticoid receptor;
[13] a method of evaluating a substance having an activity of regulating the transcription-controlling activity of a nuclear receptor complex, comprising the followings steps:
   (1) detecting the activity of a test substance in regulating the transcription-controlling activity of the nuclear receptor complex using the method of [9]; and
   (2) selecting a test substance capable of suppressing or enhancing the transcriptional activation function of the nuclear receptor complex, by comparison with a control;
[14] a method for detecting the activity of a test substance in binding to a nuclear receptor, wherein the nuclear receptor is a protein that is encoded by the polynucleotide of any one of the following (A) to (E):
   (A) a polynucleotide comprising a coding region of the nucleotide sequence of SEQ ID NO: 1;
   (B) a polynucleotide encoding a protein comprising the amino acid sequence of SEQ ID NO: 2;
   (C) a polynucleotide comprising an amino acid sequence wherein one or more amino acids are replaced, deleted, inserted, and/or added to the amino acid sequence of SEQ ID NO: 2, encoding a protein that:
      (a) controls the transcriptional activation function of a nuclear receptor when co-existed with the nuclear receptor; and
      (b) lacks at least part of a DNA binding domain;
   (D) a polynucleotide that hybridizes under stringent conditions with a polynucleotide comprising the nucleotide sequence of SEQ ID NO: 1, encoding a protein that:
      (a) controls the transcriptional activation function of a nuclear receptor when co-existed with the nuclear receptor; and
      (b) lacks at least part of a DNA binding domain;
   (E) a polynucleotide comprising a nucleotide sequence in which a sequence corresponding to nucleotides 599 to 715 of SEQ ID NO: 5 is deleted or replaced with another nucleotide sequence, and comprising a nucleotide sequence that comprises homology of 70% or more to the above nucleotide sequence in which the sequence corresponding to nucleotides 599 to 715 is deleted;
   wherein the method comprises the following steps:
   (1) contacting the nuclear receptor, its ligand, and the test substance in any of the following orders i) to iii):
      i) contacting the nuclear receptor and the test substance first, and then contacting them with the ligand;
      ii) contacting the nuclear receptor and the ligand in the presence of the test substance; or
      iii) contacting the nuclear receptor and the ligand first, and then contacting them with the test substance;
   (2) measuring the amount of the ligand or the test substance bound to the nuclear receptor; and
   (3) detecting the activity of binding to the nuclear receptor, using the measurement result according step (2) as an index;
[15] the method of [14], wherein the ligand is a compound selected from the group consisting of ligands, agonists, and antagonists of estrogen receptor-related receptors;
[16] the method of [14], wherein the nuclear receptor coexists with another arbitrary second nuclear receptor;
[17] the method of [16], wherein the second nuclear receptor belongs to a member selected from the group consisting of group A of subfamily 3, group B of subfamily 3, group C of subfamily 3, and group C of subfamily 1;
[18] the method of [17], wherein the second nuclear receptor is a nuclear receptor selected from the group consisting of estrogen receptor-related receptors, estrogen receptors, and thyroid hormone receptors;
[19] the method of [17], wherein the nuclear receptor is a glucocorticoid receptor;
[20] a method of evaluating a substance capable of binding to a nuclear receptor, comprising the following steps:
   (1) detecting the activity of a test substance in binding to a nuclear receptor, using the method of [14]; and
   (2) selecting a test substance capable of binding to the nuclear receptor;
[21] an agent for controlling the activity of a nuclear receptor, comprising as an effective ingredient a substance selected by the method of [13] or [20];
[22] an agent for controlling the activity of a nuclear receptor, comprising as an effective ingredient a polynucleotide of any one of the following (A) to (E), or a protein encoded by the polynucleotide:
   (A) a polynucleotide comprising a coding region of the nucleotide sequence of any one of SEQ ID NO: 1, 3, or 5;
   (B) a polynucleotide encoding the amino acid sequence of any one of SEQ ID NO: 2, 4, or 6;
   (C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
   (D) a polynucleotide that hybridizes under stringent conditions with a polynucleotide comprising a nucleotide sequence of any one of SEQ ID NO: 1, 3, or 5, encoding a protein functionally equivalent to a protein comprising the amino acid sequence of any one of SEQ ID NO: 2, 4, or 6;
   (E) a polynucleotide comprising a sequence with homology of 80% or more to the nucleotide sequence of any one of SEQ ID NO: 1, 3, or 5, encoding a protein functionally equivalent to a protein comprising the amino acid sequence of any one of SEQ ID NO: 2, 4, or 6;
[23] an agent for controlling the activity of a nuclear receptor, comprising as an effective ingredient any of the following (1) to (4) :
   (1) An antisense polynucleotide of the polynucleotide of the above (A) to (E);
   (2) An antibody capable of recognizing a protein encoded by the polynucleotide of the above (A) to (E);
   (3) A protein conferring a dominant negative effect on a protein encoded by a polynucleotide of the above (A) to (E); and
   (4) A double-stranded RNA of 21 to 23 base pairs, comprising a sense RNA corresponding to a partial sequence of a polynucleotide of the above (A) to (E), and its antisense RNA;
[24] a model animal in which the activity of a nuclear receptor is controlled, where the animal is a transgenic non-human animal in which the expression of a protein of any one of the following (A) to (D) is controlled:
   (A) A protein comprising the amino acid sequence of any of SEQ ID NO: 2, 4, or 6;
   (B) A protein encoded by a DNA that hybridizes under stringent conditions with a DNA comprising the nucleotide sequence of any of SEQ ID NO: 1, 3, or 5, and which is functionally equivalent to a protein comprising the amino acid sequence of any one of SEQ ID NO: 2, 4, or 6;
   (C) A protein comprising an amino acid sequence wherein one or more amino acids are replaced, deleted, inserted, and/or added to the amino acid sequence of any one of SEQ ID NO: 2, 4, or 6, and which is functionally equivalent to a protein comprising the amino acid sequence of any one of SEQ ID NO: 2, 4, or 6; and
   (D) A protein comprising an amino acid sequence with homology of 80% or more to the amino acid sequence of any one of SEQ ID NO: 2, 4, or 6, and which is functionally equivalent to a protein comprising the amino acid sequence of any one of SEQ ID NO: 2, 4, or 6;
[25] a method for diagnosing a disease that is caused by an abnormal activity of a nuclear receptor, comprising the steps of measuring the expression level of a polynucleotide in a biological sample collected from a subject, and determining that the subject is developing a disease caused by abnormal nuclear receptor activity when the expression level of that polynucleotide is elevated or decreased compared with a healthy subj ect, wherein the polynucleotide is any of the following (A) to (E) :
   (A) A polynucleotide comprising a coding region of the nucleotide sequence of any of SEQ ID NO: 1, 3, or 5;
   (B) A polynucleotide encoding a protein comprising the amino acid sequence of any of SEQ ID NO: 2, 4, or 6;
   (C) A polynucleotide encoding a protein comprising an amino acid sequence wherein one or more amino acids are replaced, deleted, inserted, and/or added to the amino acid sequence of any of SEQ ID NO: 2, 4, or 6, encoding a protein functionally equivalent to a protein comprising the amino acid sequence of any of SEQ ID NO: 2, 4, or 6;
   (D) A polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
   (E) a polynucleotide comprising a sequence with homology of 80% or more to a nucleotide sequence of any of SEQ ID NO: 1, 3, or 5, encoding a protein functionally equivalent to a protein comprising the amino acid sequence of any of SEQ ID NO: 2, 4, or 6.

To discover novel nuclear receptors, the present inventors targeted full-length cDNA libraries, searching for genes that encode proteins comprising a DNA binding domain (DBD) and a ligand-binding domain (LBD) that are specific for a nuclear receptor. Of the genes screened, the present inventors focused on C-BRAWH2017250, which was confirmed to be highly homologous to ERRγ.

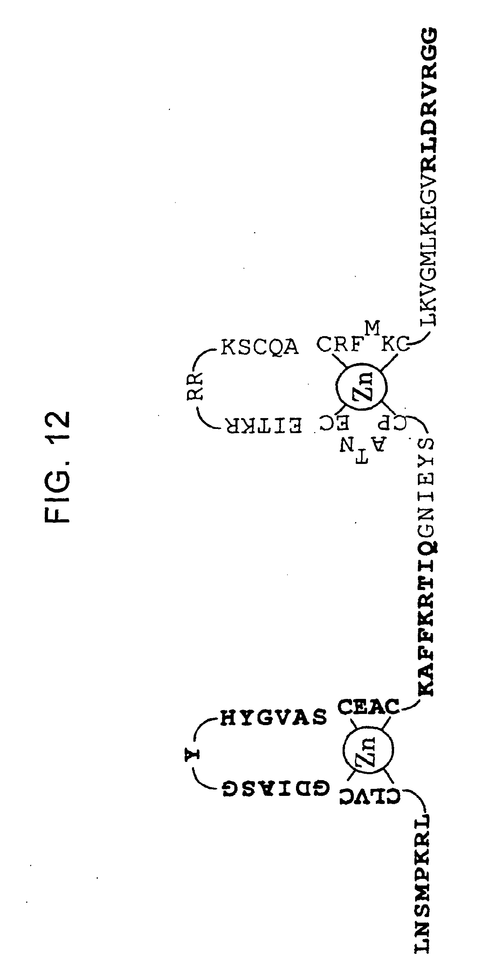

C-BRAWH2017250 comprises a region of 1188 base pairs (684-1874), encoding a protein of 396 amino acids. The nucleotide sequence of ERRγ3 is shown in SEQ ID NO: 1, and the amino acid sequence it encodes is shown in SEQ ID NO: 2. The protein contains a DNA binding domain (DBD) and a ligand-binding domain (LBD), which are common to the nuclear receptor family. The two ERRγ isoforms, ERRγ1 (the short form) and ERRγ2 (the long form), showed the highest homology with the C-BRAWH2017250 cDNA. Comparison of their amino acid sequences showed that the N-terminal sequence of the protein encoded by C-BRAWH2017250 was 23 amino acids shorter than that of ERRγ2. Furthermore, a deletion of 39 amino acids in length was observed with regard to the common DBD region that exists in both ERRγ1 and ERRγ2. The deleted region corresponds to the downstream zinc finger structure of the two in the DBD (Fig. 12).

The nucleotide sequences comprising C-BRAWH2017250, ERRγ1 and ERRγ2 were then correlated with the genomic sequence. The results indicated that C-BRAWH2017250 is a splice variant, lacking an exon that composes part of the DBD. Based on structural features, the present inventors concluded that C-BRAWH2017250 was a novel isoform of ERRγ, naming it ERRγ3. Thus, the present invention relates to polynucleotides comprising the coding region of the nucleotide sequence of SEQ ID NO: 1, which encodes ERRγ3, and polynucleotides that encode proteins comprising the amino acid sequence of SEQ ID NO: 2.

The present invention also comprises a polynucleotide encoding a protein functionally equivalent to a protein comprising the amino acid sequence of SEQ ID NO: 2. Herein, a "functionally equivalent" protein is defined as being functionally equivalent to the ERRγ3 proteins of the present invention if the protein of interest comprises the following (a) and (b):
(a) controlling the transcriptional activation function of a nuclear receptor when co-existed with the nuclear receptor; and
(b) lacking at least a part of a DNA binding domain;

For example, the present invention comprises a polynucleotide encoding a protein which comprises the aforementioned (a) and (b), and which is encoded by the following polynucleotide:
(C) a polynucleotide encoding a protein comprising an amino acid sequence wherein one or more amino acids are replaced, deleted, inserted, and/or added to the amino acid sequence of SEQ ID NO: 2; or
(D) a polynucleotide that hybridizes under stringent conditions with a polynucleotide comprising the nucleotide sequence of SEQ ID NO: 1.

Herein, controlling transcriptional activation function of a nuclear receptor when a protein co-existed with the nuclear receptor can be examined as follows: In the following explanation, proteins to be examined for functional equivalence are described as test proteins. For example, a reporter gene assay may be used to examine the aforementioned attribute (a). Specifically, an arbitrary nuclear receptor and a test protein examined for attribute (a) is co-expressed in cells. The cells used as hosts are introduced with an expression vector for a reporter gene, under the regulation of a responsive element for the nuclear receptor. If the test protein comprises attribute (a) , an change in reporter gene expression level are more significant compared to expression of a nuclear receptor only. Therefore, based on a comparison of both reporter gene expression levels, a test protein can be determined to comprise attribute (a).

Herein, controlling the transcriptional activation function means enhancing or reducing the transcriptional activation function of the above-mentioned second nuclear receptor. Thus, test proteins comprise the function of enhancing the transcriptional activation function of a second nuclear receptor when the expression level of the aforementioned reporter gene is increased. On the other hand, a decrease in the expression level indicates that a tested protein comprises the function of reducing the transcriptional activation function of the second nuclear receptor. Control of transcriptional activation function can be said to be the interaction between the tested protein and the second nuclear receptor.

Herein, a nuclear receptor's transcription-controlling activity means a nuclear receptor's activity in initiating gene transcription via a transcriptional unit. In general, nuclear receptors are considered to initiate transcription by recruiting multiple factors called transcriptional cofactors. Transcriptional activation brought by the transcription-controlling activity of a nuclear receptor is called transcriptional activation function.

In addition, test protein binding to a nuclear receptor can be easily confirmed according to the principles of a variety of binding assays. For example, either the nuclear receptor or the test protein can be tagged, and the other labeled. The two are then contacted, and the tagged nuclear receptor or test protein can be recovered using a ligand with affinity for the tag. If a labeled molecule is recovered in association with the tagged molecule, the two molecules are confirmed to comprise binding affinity.

Attribute (b) , "lacking at least a part of a DBD", is used herein as an index of functional equivalence, and may be tested by analyzing amino acid sequences. As well as the deletion of at least a part of the DBD, attribute (b) herein includes the loss of DNA binding ability due to a DBD mutation. For example, as shown in Fig. 12, ERRγ3 of SEQ ID NO: 2 lacks one of the two zinc fingers present in ERRγ2.

Herein, the arbitrary second nuclear receptor may be a nuclear receptor belonging to a group such as group A of subfamily 3, group B of subfamily 3, group C of subfamily 3, and group C of subfamily 1. The definitions and nomenclature of nuclear receptor subfamilies and groups are in accordance with those of the Nuclear Receptors Nomenclature Committee (Nuclear Receptors Nomenclature Committee. "A unified nomenclature system for the nuclear receptor superfamily." Cell 97:161-163 (1999)). Nuclear receptors have been also analyzed from an evolutionary point of view (Laudet V. "Evolution of the nuclear receptor superfamily: early diversification from an ancestral orphan receptor." J. Mol. Endocrinol. 19:207-226 (1997)).

The nuclear receptor superfamily is classified into six subfamilies and 26 groups, mainly by comparing similarities in DBD and LBD amino acid sequences, which are evolutionally well conserved among receptor proteins (http://www.ens-lyon.fr/LBMC/laudet/NucRec/nomenclature_table.ht ml). The respective receptors of each subfamily and group can be searched using the NUREBASE database (Duarte J. et al. "NUREBASE: database of nuclear hormone receptors." Nucleic Acids Research 30(1):364-368 (2002)). For example, these nuclear receptors are classified into the following groups:
Subfamily 1, Group A: Thyroid hormone receptor (TR)
Subfamily 3, Group B: Estrogen receptor related receptor (ERR)
Subfamily 3, Group A: Estrogen receptor (ER)
Subfamily 3, Group C: Glucocorticoid receptor (GR)

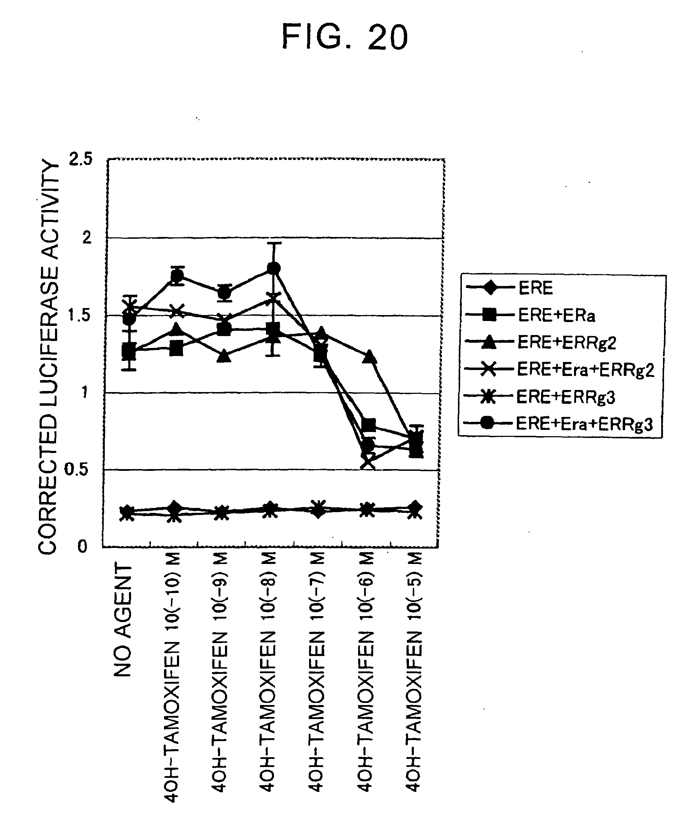

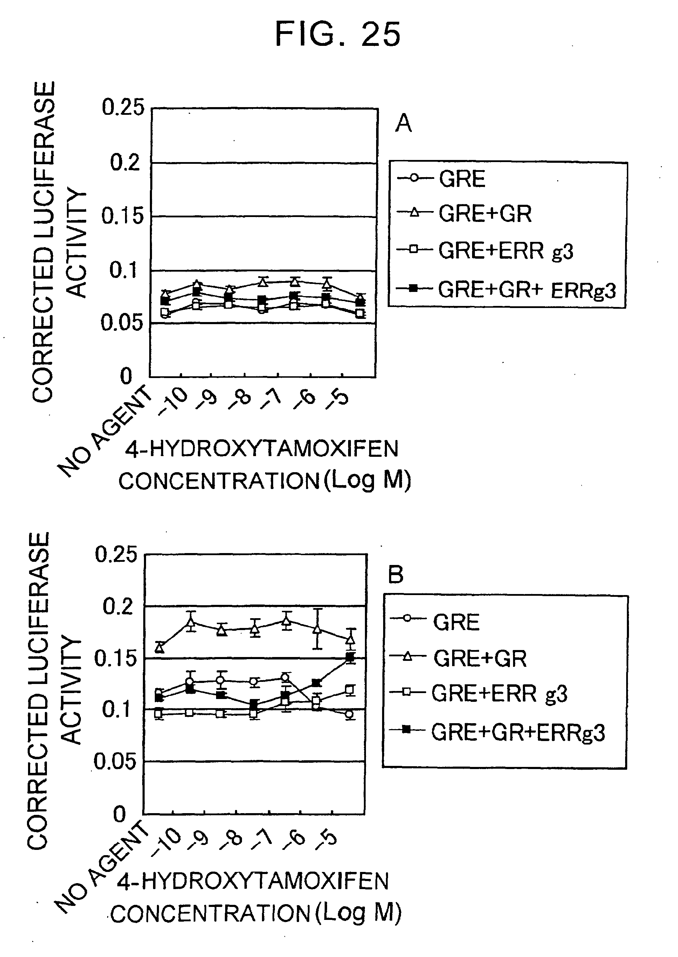

Proteins comprising the amino acid sequence of SEQ ID NO: 2 have the effect of enhancing the transcriptional activation function of ERR, ER, or TR, as shown in the Examples below (Fig. 20). In addition, proteins comprising the amino acid sequence of SEQ ID NO: 2 can inhibit the activity of GR, also shown in the Examples below (Fig. 25).

The present invention also comprises polynucleotides that comprise a nucleotide sequence whereby 1) a sequence corresponding to nucleotides 599 to 715 of SEQ ID NO: 5 is deleted or replaced with another sequence; and 2) the sequence comprises 70% or more homology to a nucleotide sequence in which the sequence corresponding to nucleotides 599 to 715 of SEQ ID NO: 5 is deleted. The nucleotide sequence shown in SEQ ID NO: 5 is that of known ERR isoform, ERRγ2. The sequence corresponding to nucleotides 599 to 715 of SEQ ID NO: 5 encodes a part of the DBD. Thus, polynucleotides comprising a nucleotide sequence that lacks the above region, yet also comprising 70% or more homology to the rest of the sequence, are preferred polynucleotides in the present invention.

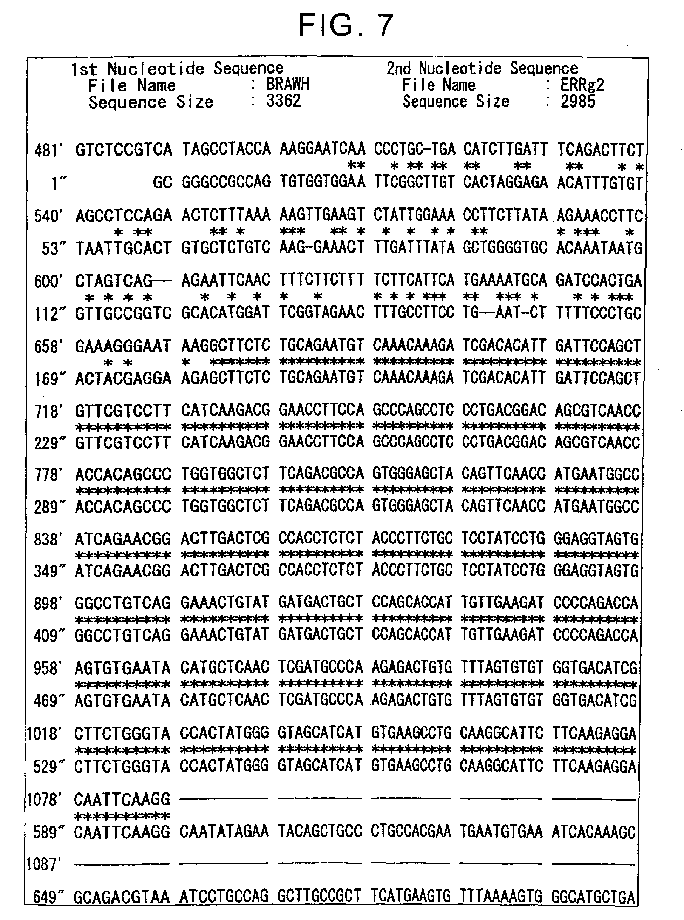

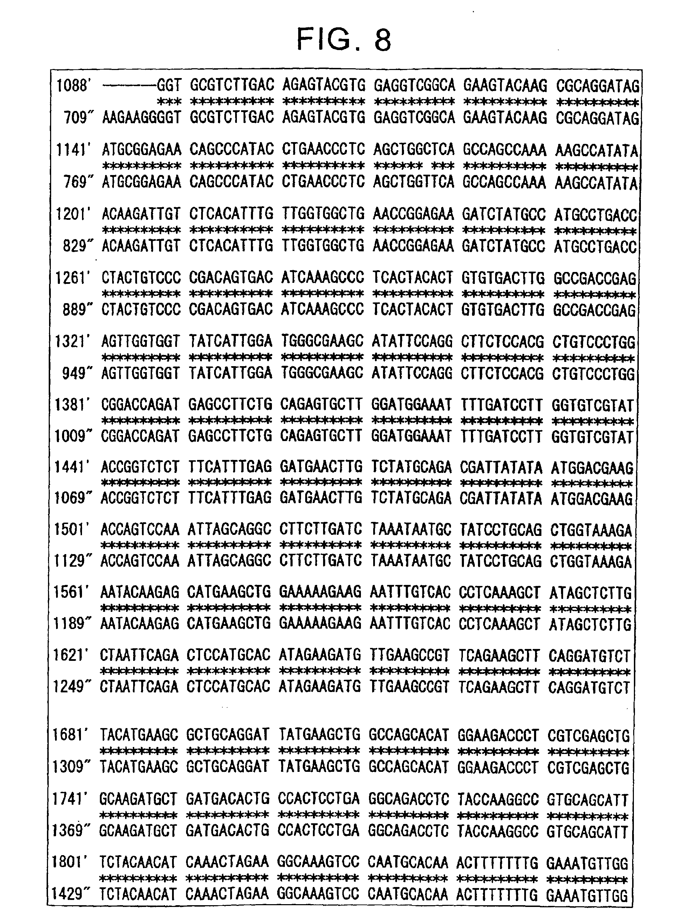

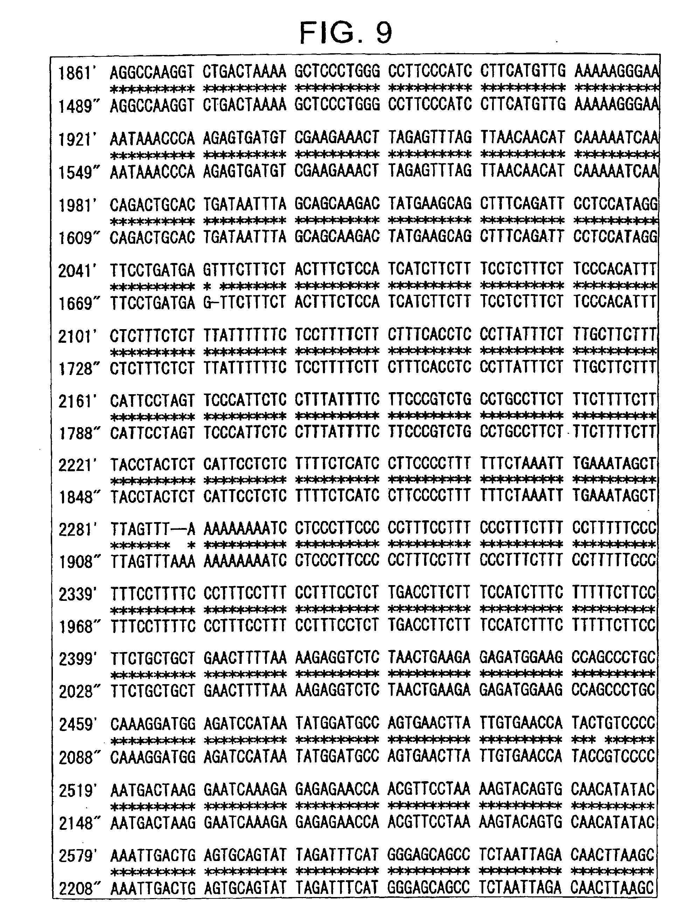

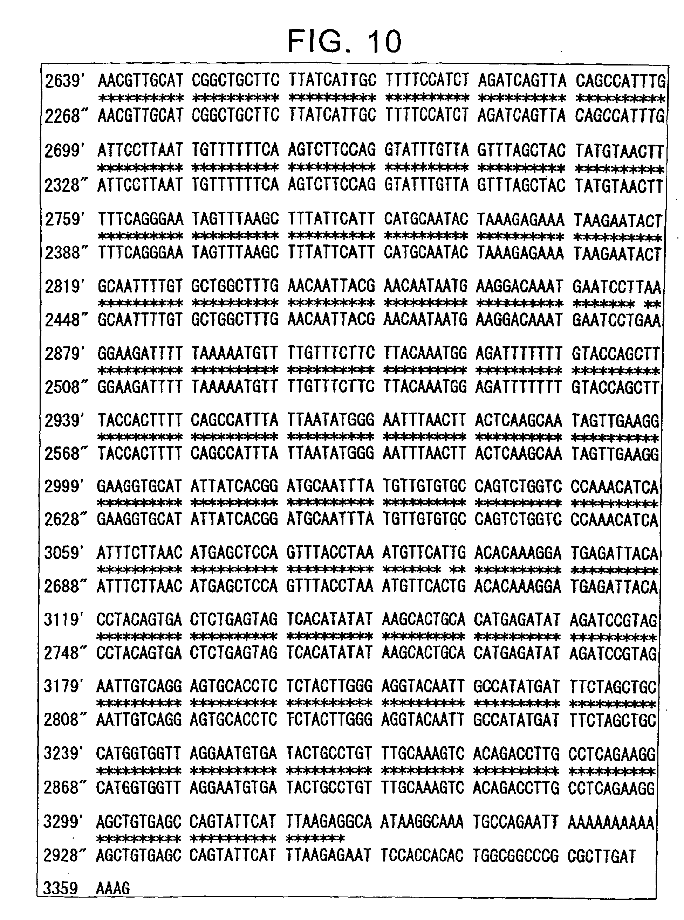

Nucleotide sequences corresponding to nucleotides 599 to 715 means nucleotides in a sequence located in a position that corresponds to the nucleotides from 599 to 715 of SEQ ID NO: 5, when the first sequence is aligned with that of SEQ ID NO: 5. Thus, the nucleotides are not always located at positions 599 to 715. For example, in the nucleotide sequence of SEQ ID NO: 1, which is a favorable polynucleotide of the present invention, nucleotides 1088 to 1094 correspond to those of 599 to 715 of SEQ ID NO: 5. Nucleotide sequences may be checked for their lack of nucleotides corresponding to those from 599 to 715 of SEQ ID NO: 5 by aligning both sequences. Algorithms for aligning different sequences are commonly known to those skilled in the art. For example, Figs. 7 to 10 align the nucleotide sequence of SEQ ID NO: 1 with that of SEQ ID NO: 5.

Furthermore, the present invention comprises polynucleotides that comprise nucleotide sequences in which a nucleotide sequence corresponding to nucleotides 599 to 715 of SEQ ID NO: 5 is replaced with another sequence. Any nucleotide sequence may be used as a replacement. However, the DBD should be deleted in the polynucleotides of the present invention. In addition, when the replacement sequence is connected to the nucleotide sequences adjacent to the region of interest, it must constitute a single translation frame. Thus, the replacement nucleotide sequence is required to be a nucleotide sequence by which any frame shifts or stop codons are not introduced, and which does not encode the same amino acid sequence as that of the DBD encoded by the nucleotide sequence prior to replacement.

Proteins functionally equivalent to the proteins identified in the examples of the present invention can be prepared by those skilled in the art, for example, by using a method of introducing mutations to the amino acid sequence of a protein. An example of such method for introducing mutations is the method of site-directed mutagenesis (Current Protocols in Molecular Biology, edit. Ausubel et al. , Publish. John Wiley & Sons, Section 8.1-8.5 (1987)). In addition, this kind of protein may also be generated as a result of naturally occurring amino acid mutations. The present invention comprises proteins wherein one or more amino acids are replaced, deleted; inserted, and/or added to the amino acid sequence of SEQ ID NO: 2, as long as these proteins are functionally equivalent to the proteins identified in the examples of the present invention.

The number of mutated amino acids in a protein, or the site of mutation, is not limited, as long as the protein retains its function. Typically, the number of mutated amino acids is 10% or less of the total number of the amino acids in a protein sequence, preferably 5% or less, and more preferably 1% or less. To maintain protein function, amino acids are preferably replaced with amino acids comprising similar characteristics. For example, since Ala, Val, Leu, Ile, Pro, Met, Phe and Trp are all grouped into non-polar amino acids, they are considered to comprise similar characteristics. Uncharged amino acids include Gly, Ser, Thr, Cys, Tyr, Asn and Gln. Acidic amino acids include Asp and Glu, and basic amino acids include Lys, Arg and His.

Alternatively, proteins functionally equivalent to the proteins of the present invention can be isolated using techniques known to those skilled in the art, such as hybridization or gene amplification. Those skilled in the art can conventionally carry out hybridization (Current Protocols in Molecular Biology, edit. Ausubel et al., Publish. John Wiley & Sons, Section 6.3-6.4 (1987)) using the nucleotide sequence of SEQ ID NO: 1, which encode the proteins of the present invention, or their partial sequences, to isolate polynucleotides comprising high homology to these nucleotides and obtain functionally equivalent proteins. The present invention comprises proteins encoded by polynucleotides that hybridize with polynucleotides encoding the proteins of the present invention, as long as these proteins are functionally equivalent.

Stringent hybridization conditions for isolating polynucleotides that encode functionally equivalent proteins are normally washing in "1x SSC, 0.1% SDS at 37°C". More stringent conditions are "0.5x SSC, 0.1% SDS at 42°C", and the most stringent conditions are "0.1x SSC, 0.1% SDS at 65°C". By increasing the stringency of the conditions for hybridization, one can expect to isolate DNAs comprising greater homology to the probe sequence. While the above sets of SSC, SDS, and temperature conditions are given as examples, one skilled in the art can readily achieve stringencies similar to the above by appropriately combining these or other conditions that determine hybridization stringency. The other conditions influencing stringency include probe concentration, probe length, and incubation time for hybridization.

The amino acid sequences of the proteins isolated using the above hybridization, or the nucleotide sequences encoding these proteins, normally comprise high homology with the proteins of the present invention of SEQ ID NO: 2. High homology means sequence identity of for example 80% or higher, preferably 85% or higher, and more preferably 90% or higher (for example, 95% or higher). Homology can be determined using BLAST2 homology search algorithm (Altschul, S.F. et al. "Gapped BLAST and PSI-BLAST: a new generation of protein database search programs." Nucleic Acids Res. 25: 3389-3402 (1997)).

The amino acid sequence of ERRγ3 of the present invention, shown in SEQ ID NO: 2, is 82.6% homologous to each sequence of the known isoforms, ERRγ1 and ERRγ2. The amino acid sequences of the present invention preferably comprise an amino acid sequence with homology of 80% or more to the sequence of SEQ ID NO: 2.

In addition, proteins functionally equivalent to the proteins of the present invention can be obtained by performing PCR (Current protocols in Molecular Biology, edit. Ausubel et al., Publish. John Wiley & Sons, Section 6.1-6.4 (1987)) using primers designed based on the parts of the nucleotide sequences identified in the Examples of the present invention (SEQ ID NO: 1), and isolating DNA fragments comprising high homology to the nucleotide sequences or their parts. The proteins of the present invention, and functionally equivalent proteins, can be used in the methods for evaluating a compound that modifies a protein's function, as described below. In addition, the polynucleotides encoding these proteins are useful for producing the proteins, or as primers or probes for examining expression level.

The present invention also relates to polynucleotides that encode proteins comprising a dominant negative form against the proteins of the present invention. When expressed, the polynucleotide encoding a protein capable of conferring a dominant negative effect exerts the function of eliminating or reducing the activity of endogenous wild type proteins originally contained in cells. For example, an inactive nuclear receptor protein, without transcriptional activation function, can be obtained by altering the part of an amino acid sequence of a protein of the present invention that corresponds to a region considered to influence its activity, such as a region required for interaction with another nuclear receptor, or a ligand binding domain. By expressing in cells a gene that encodes such an amino acid sequence, the expression of ERRγ3 of the present invention can be suppressed by dominant negative effect (competitive inhibition by an inactive form). Thus, the activity of a nuclear receptor can be controlled by using gene transfection technologies such as viral vectors to introduce cells with a gene that encodes the inactive form.

In addition, the present invention provides partial peptides of the proteins of the invention. The partial peptides are useful as antigens for obtaining antibodies against the proteins of the present invention. In particular, partial peptides comprising amino acid sequences unique to proteins of the present invention, and showing little homology to other proteins, are promising antigens for giving rise to antibodies highly specific to a protein of the present invention. An example of such an amino acid sequence is a region comprising an amino acid sequence corresponding to the missing zinc finger of ERRγ3, as in the structure shown in Fig. 12.

The partial peptides of the present invention comprise sequences of at least seven amino acids, preferably nine amino acids or more, more preferably 12 amino acids or more, and most preferably 15 amino acids or more. The partial peptide may be produced, for example, by genetic engineering techniques, common methods of peptide synthesis, or by digesting the proteins of the present invention with appropriate peptidases.

The present invention also provides expression vectors comprising any of the above polynucleotides. In addition, the present invention relates to transformants carrying the above polynucleotides, or any of the above expression vectors, and methods of producing the proteins of the present invention or their partial peptides, which comprise culturing the transformant and isolating a protein of the present invention from the culture. Moreover, the present invention provides proteins produced by the above methods, or their partial peptides.

Those skilled in the art know that in using recombinant technology to produce polypeptides, the desired polypeptides can be obtained with different types or degrees of glycosylation, depending on the type of host cell. Those skilled in the art also know that when producing desired polypeptides as secreted proteins, the proteins comprise different kinds of terminal amino acid sequences (N- and/or C-termini), since the precursor polypeptides expressed in host cells undergo processing by signal peptidases and such. Therefore, those skilled in the art should easily understand that these polypeptides are comprised in the scope of the proteins of the present invention.

The Examples below illustrate an example of the construction of an expression vector capable of functioning in mammalian cells. On disclosure of a DNA encoding a protein of the present invention herein, those skilled in the art can readily construct expression vectors capable of expressing and producing the protein when introduced into host cells, including fungi such as yeast, and prokaryotic cells. Therefore, the present invention comprises expression vectors that can be constructed using methods known in the art, based on the nucleotide sequences of the present invention.

Microorganisms that can be used for expressing the polynucleotides that encode the proteins of the present invention include prokaryotic bacteria such as *Escherichia* coli and *Bacillus subtilis*, and eukaryotic yeast such as *Saccharomyces cerevisiae*. Mammalian cells include tissue-cultured human cells, and cultured animal cells. Furthermore, cultured plant cells may be used.

Examples of microorganism host cells are bacteria belonging to *Escherichia*, and *Saccharomyces cerevisiae*. More specifically, the following strains are examples of microorganism hosts:
Bacteria belonging to *Escherichia:*
   *E*.*coli* HB101 (ATCC 33694)
   *E.coli* HB101-16 (FERM BP-1872)
   *E*.*coli* MM294 (ATCC 31446)
   *E*.*coli* DH1 (ATCC 33849)
Yeast:
   *S*.*cerevisiae* AH22 (ATCC 38626)

Examples of mammalian host cells include human embryonic kidney-derived HEK293 cells, mouse L929 cells, and Chinese hamster ovary (CHO) cells.

Normally, when the host cells are prokaryotic bacteria cells, particularly *E*.*coli*, the expression vectors comprise at least a promoter, an initiation codon, a polynucleotide encoding an amino acid sequence of a protein of the present invention, a termination codon, and an autonomously replicable unit. When used in eukaryotic host cells such as yeast and mammalian cells, the expression vector preferably comprises at least a promoter, an initiation codon, a polynucleotide encoding an amino acid sequence of a protein of the present invention, and a termination codon. It may further comprise insertion of an enhancer sequence, 5'- and 3'-untranslated regions of the protein of the present invention, a polyadenylation site, and an autonomously replicable unit.

The above autonomously replicable units preferably comprise a transformant selection marker (e.g. ampicillin resistance). In an expression vector for a microorganism host cells, a promoter means a promoter/operator region, comprising a promoter, operator, and Shine-Dalgarno (SD) sequence (e.g. AAGG). Conventional promoter operator region are examples of such promoters. Specifically, the lactose operon, PL-promoter, trp-promoter, or such can be used. An example of an expression vector for use in yeast host cells is the pho5 promoter. In addition, basic amino acids with affinity for metal ion chelates may be added to either end of the proteins of the present invention to facilitate purification. When adding basic amino acids, oligopeptides can be added to an arbitrary end of a gene of interest by performing PCR using a primer to which is attached at the 5'-end a sequence comprising a consecutive nucleotide sequence encoding the desired amino acids. Basic amino acids include histidine, lysine or arginine.

Promoters used in expression vectors in mammalian cells include the HTLV-LTR promoter, SV40 early and late promoters, CMV promoter, and mouse metallothionein-I (MMT) promoter. Preferable initiation codons include the methionine codon (ATG).

The termination codons include conventional termination codons (e.g. TAG, TGA and such). Autonomously replicable units are DNA sequences capable of autonomously replicating the entire DNA of the expression vector that comprises them within a host cell, and these include natural plasmids, artificially modified plasmids, and synthetic plasmids. Artificially modified plasmids include DNA fragments prepared from natural plasmids. When using *E*. *coli* as a host, preferred examples of such desired plasmids include plasmid pBR322 and its artificial modifications. The artificially modified plasmids include DNA fragments obtained by treating pBR322 with an appropriate restriction enzyme. When using yeast host cells, pJDB219, and pJDB207 may be used as examples. Further, plasmids used in animal cells include plasmid pcDNA3.1 (Invitrogen), pMM324, pSV2dhfr (ATCC 37145), pdBPV-MMTneo (ATCC 37224), and pSV2neo (ATCC 37149).

An enhancer sequence may be the SV40 enhancer sequence, for example. The polyadenylation sites include the SV40 polyadenylation site.

Polynucleotides encoding the amino acid sequences of the proteins of the present invention are obtainable by synthesizing the entire or partial sequence using a DNA synthesizer, for example. Alternatively, they can be obtained from human cDNA libraries by using probes or primers whose design is based on the nucleotide sequence of SEQ ID NO: 1. Furthermore, genomic DNAs that encode the proteins of the present invention may be prepared by processing genomic DNAs of standard methods. For example, genomic DNAs may be processed by methods that comprise the steps of digestion with restriction enzymes, dephosphorylation with bacterial alkaline phosphatase, phosphorylation with T4 polynucleotide kinase, and ligation with T4 DNA ligase. Furthermore, genomic DNAs obtained as above can be used to identify transcription initiation sites for the genes of the present invention that occur in the genome, and to specify the expression regulatory regions located further upstream. Regulatory regions, such as promoters and enhancers that regulate the expression of genes encoding the proteins of the present invention, are useful as target regions for detecting abnormal protein expression. Alternatively, for example, decoy nucleic acid medicines targeted at such regions may be used to achieve expression regulation.

Methods known to those skilled in art or in the literature may be used to perform those procedures required to implement the present invention, such as DNA cloning, construction of each plasmid, transfection into host cells, culture of transformants, and recovery of proteins from the culture (Molecular Cloning, 2nd. edition, Sambrook J. et al. , Cold Spring Harbor Laboratory (1989) ; DNA Cloning, Glover D.M., IRL PRESS (1985) ; Molecular Cloning, 3rd. edition, Sambrook J. et al., Cold Spring Harbor Laboratory (2001)).

The host cells of the present invention also comprise cells used for the functional analysis of ERRγ3 of the present invention, or in the methods for evaluating function-inhibiting and function-enhancing agents which use these proteins. Vector transfection into host cells may be performed by calcium phosphate precipitation, electroporation (Current protocols in Molecular Biology, edit. Ausubel et al. , Publish. John Wiley & Sons., Section 9.1-9.9 (1987)), by using LipofectAMINE (Gibco BRL) , microinjection, or such. The proteins of the present invention may be prepared from transformants using methods of protein separation or purification commonly known to those skilled in the art. The present invention relates to substantially pure proteins that are purified as above. Herein, "substantially pure" means free from contamination with other human-derived proteins. Alternatively, substantially pure proteins of the present invention are proteins comprising a purity of, for example, 80% or more, normally 90% or more, 95% or more, preferably 98% or more, and more preferably 99% or more.

The present invention also provides polynucleotides comprising a nucleotide sequence of SEQ ID NO: 1, or a polynucleotide comprising at least 15 nucleotides that are complementary to the complementary strand of the polynucleotide. Herein, the "complementary strand" means the other strand to one strand of a double-stranded polynucleotide comprising A:T (A:U) and G:C base pairs. In addition, "complementary" refers not only to complete complementarity to a region of at least 15 consecutive nucleotides, but also to homology of at least 70% or more, preferably at least 80% or more, more preferably 90% or more, and further preferably 95% or more. Homology may be determined by the algorithms described in the present invention.

Such polynucleotides may be used as probes for detecting or isolating DNAs or RNAs that encode the proteins of the present invention, or as primers for amplifying the polynucleotides of the present invention. When used a primer, the polynucleotide length is normally from 15 to 100 base pairs, and preferably from 15 to 35 base pairs. In addition, when used as a probe, the polynucleotides comprise at least a part or an entire sequence of a polynucleotide of the present invention, and comprise at least 15 base pairs. When used as a primer, the 3'-region must be complementary, but a recognition site for a restriction enzyme, tag or such can be added to the 5'-region.

In particular, regions within the nucleotide sequence of SEQ ID NO: 1, and which comprise deletion in ERRγ2, are useful for detecting DNAs that comprise the nucleotide sequence of SEQ ID NO: 1. An oligonucleotide capable of hybridizing with the nucleotide sequence of an above region or its complementary sequence may be useful as a probe or primer for specifically detecting EERγ3.

Alternatively, primers that can anneal to a region adjacent to a deleted region in the nucleotide sequence of SEQ ID NO: 1 may be useful for distinguishing ERRγ3 from other isoforms. For example, sets of primers, whereby one primer anneals to a region within the deleted nucleotide sequence, and the other comprises a nucleotide sequence adjacent to the deleted sequence, are capable of amplifying known isoforms ERRγ1 and ERRγ2, whereas they cannot amplify ERRγ3, which comprises deletion. Moreover, primers that can anneal to a region that corresponds to nucleotides 1 to 670 of ERRγ3 and comprises the exons listed below, which are not shared with other isoforms, may be useful for the specific amplification of ERRγ3:
Exon A (1-80)
Exon B (81-552)
Exon C (553-670)

The polynucleotides of the present invention may be used for testing or diagnosing abnormalities of the proteins of the present invention. For example, Northern hybridization or RT-PCR that uses the polynucleotides of the present invention as probes or primers may be performed to test for abnormal expression. In addition, genomic DNA PCR and RT-PCR may be performed by carrying out polymerase chain reactions (PCR) using the polynucleotides of the present invention as primers. Furthermore, by amplifying genes that encode the proteins of the present invention, or their expression regulatory regions, sequence abnormalities may be examined or diagnosed using RFLP analysis, SSCP, sequencing, and such.

Furthermore, since the ERRγ isoforms of the present invention are capable of stimulating the transcriptional activation effect of nuclear receptors, polynucleotides encoding the isoforms may be used for gene therapy of diseases related to these isoforms. Thus, diseases related to the ERRγ3 isoforms of the present invention can be prevented or treated by expressing these isoforms, by introducing polynucleotides that encode the isoforms such that they can be appropriately expressed in the host disease sites. Herein, "expressed" means transcribed and/or translated. The polynucleotides of the present invention may be used in expression analyses to test or diagnose gene expression at the transcription level. Moreover, antibodies against the proteins of the invention may be used to test or diagnose the gene expression at the translation level.

ERRγ3 can regulate the transcription-controlling activity of ERR, which is involved in estrogen signal transduction, and hence may be useful for diagnosing or treating diseases caused by abnormal estrogen signal transduction. For example, if the expression level of ERRγ3 is lower than that in healthy subjects, estrogen signal transduction may be defective. To normalize estrogen signal transduction levels, patients with such conditions can be administered with ERRγ3, or vectors for expressing ERRγ3.

Examples of diseases caused by abnormal estrogen signal transduction are breast cancer, cervix cancer, osteoporosis, hyperlipidemia, arteriosclerosis, angina, myocardial infarction, stroke, systemic lupus erythematosus, or Alzheimer's disease. Elevated ER activity is found in breast cancer and cervix cancer. In contrast, impaired ER function is considered a primary cause of osteoporosis, hyperlipidemia, arteriosclerosis, angina, myocardial infarction, stroke, systemic lupus erythematosus, or Alzheimer's disease. Therefore, ERRγ3 of the present invention may be useful for treatment or diagnosis of the above diseases.

Furthermore, the ERRγ3 of the present invention can regulate the transcription-controlling activity of TR as well as ER. Thus, ERRγ3 may be also useful for diagnosing or treating diseases caused by abnormal thyroid hormone signal transduction. Examples of such diseases are attention deficit hyperactivity disorder (ADHD) , which accompanies elevated TR function.

In addition, ERRγ3 of the present invention is capable of inhibiting the transcription-controlling activity of GR. Thus, ERRγ3 may be also useful for diagnosing or treating diseases caused by abnormal glucocorticoid signal transduction. Examples of such diseases are as follows: chronic rheumatism, hyperlipidemia, asthma, glucocorticoid resistance, inflammatory enteritis, hypertension, lymphoma, arteriosclerosis, leukemia, renal failure, hyperglycemia, multiple sclerosis, and such.

In addition, a "polynucleotide comprising the nucleotide sequence of SEQ ID NO: 1, or a polynucleotide comprising at least 15 nucleotides that are complementary to the complementary strand of the polynucleotide" comprises an antisense polynucleotide capable of suppressing the expression of a protein of the present invention. Herein, the antisense polynucleotide means a polynucleotide that suppresses the expression of a protein comprising the amino acid sequence of SEQ ID NO: 2.

A "double-stranded RNA of 21 to 23 base pairs, comprising a sense RNA corresponding to a partial sequence of the nucleotide sequence of SEQ ID NO: 1, and its antisense RNA" comprises small interfering RNA (siRNA) for suppressing the expression of a protein of the present invention. The double-stranded RNAs of the present invention comprise not only double-stranded RNAs with two strands, but also single-stranded RNAs with hairpin loop structures comprising sense and antisense sequences.

Antisense or siRNAs of the present invention that suppress the function of ERRγ3 on stimulation activity of ERR may be used as agents to control ERR activity. Alternatively, antisense or siRNAs that suppress the inhibitory function of ERRγ3 on activity of GR may be used as agents to control GR activity.

The antisense polynucleotides may be antisense DNAs, or antisense oligonucleotides. Antisense DNAs are at least 15 base pairs or longer, preferably 100 base pairs or longer, more preferably 500 base pairs or longer, and normally 3000 base pairs or shorter, preferably 2000 base pairs or shorter. The antisense DNAs may be used alone, or by insertion in the antisense orientation into appropriate vectors, such as retrovirus vectors, or adenovirus vectors. The antisense oligonucleotides are at least ten base pairs or longer, and normally 100 base pairs or shorter; preferably 20 base pairs or longer and 50 base pairs or shorter. The antisense oligonucleotides may be prepared, for example, based on the nucleotide sequence information of SEQ ID NO: 1 by using the phosphorothioate method (Stein "Physicochemical properties of phosphorothioate oligodeoxynucleotides." Nucleic Acids Res. 16: 3209-3221 (1988)).

In addition, siRNAs may be introduced into an living organism by administering a vector that carry DNAs for both their sense and antisense strands. Vectors for siRNA expression are known.

The antisense or siRNAs may be used for gene therapy of diseases caused by abnormalities of proteins comprising an amino acid sequence of SEQ ID NO: 2. Protein abnormalities include abnormal protein function, abnormal protein expression, and such. When used for gene therapy, patients may be administered using *ex vivo* or *in vivo* methods, by using viral vectors such as retrovirus vectors, adenovirus vectors, and adeno-associated virus vectors, or non-viral vectors such as liposomes.

When used for gene therapy, the polynucleotides and antisense polynucleotides may be administered to patients using ex vivo or in vivo methods, by using viral vectors such as retrovirus vectors, adenovirus vectors, and adeno-associated virus vectors, or non-viral vectors such as liposomes.

Antisenses capable of suppressing the expression of ERRγ3, which comprises the function of regulating the transcription-controlling activity of ERR, which is involved in estrogen signal transduction, may be useful for diagnosing or treating diseases caused by elevated estrogen signal transduction. For example, when the transcription level of ERRγ3 is elevated compared with the level in healthy subjects, elevated estrogen signal transduction may be suspected. Patients with such conditions may be administered with vectors for expressing an ERRγ3 antisense to normalize estrogen signal transduction.

For example, diseases caused by elevated estrogen signal transduction can include breast cancer and cervix cancer. Thus, the ERRγ3 of the present invention is useful for diagnosing and treating such diseases.

The present invention also relates to antibodies that recognize the antigenicity determining residues of the ERRγ3-specific amino acid sequences in the amino acid sequence of SEQ ID NO: 2. Such ERRγ3-specific amino acid sequences may be, for example, a unique amino acid sequence generated by deleting a zinc finger in the sequence of SEQ ID NO: 2. ERRγ2 comprises an amino acid sequence comprising 39 amino acids that correspond to the zinc finger between G (135) and V (136) in the sequence of SEQ ID NO: 2. Thus, regions comprising amino acid sequences that comprise amino acids 135 and 136 of SEQ ID NO: 2 are considered to be sequences unique to ERRγ3. Therefore, the present invention comprises antibodies that recognize the antigenicity determining residues comprised by amino acid sequences that comprise amino acids 135 and 136. Furthermore, the present invention also comprises antibodies that recognize the antigenicity determining residues comprised in the tertiary structure of proteins comprising the amino acid sequence of SEQ ID NO: 2. These antibodies, which are capable of specifically recognizing antigenicity determining residues, are useful for detecting or purifying ERRγ3.

Such antibodies may be provided in any form: they may be polyclonal antibodies or monoclonal antibodies, or parts of antibodies that are capable of binding to an antigen. These antibodies include all classes of antibodies. Moreover, special antibodies such as humanized antibodies and such are also comprised by the present invention.

Polyclonal antibodies may be obtained by immunizing rabbits with proteins of the present invention or their partial peptides (Current protocols in Molecular Biology, edit. Ausubel et al., Publish. John Wiley & Sons., Section 11.12-11.13 (1987)). Monoclonal antibodies may be prepared by immunizing mice with the proteins of the present invention or their partial peptides, generating hybridoma cells by fusing spleen cells with myeloma cells, and recovering the antibodies from the hybridoma cells (Current protocols in Molecular Biology, edit. Ausubel et al., Publish. John Wiley & Sons., Section 11.4-11.11 (1987)).

Antibodies capable of binding to a protein of the present invention may be useful not only for purification of that protein, but also for testing or diagnosing abnormal protein expression or irregular protein structure. Specifically, the presence or absence of abnormal protein expression or irregular structure may be tested or diagnosed by, for example, extracting protein from tissue, blood, cells, or such, and detecting the protein by western blotting, immunoprecipitation, ELISA, and such.

Antibodies capable of binding to proteins of the present invention may be also useful for treating diseases related to the proteins. Human antibodies or humanized antibodies are preferred when treating patients because of their low immunogenicity. Human antibodies may be prepared by immunizing mice whose immune system is replaced with a human system (for an example see "Functional transplant of megabase human immunoglobulin loci recapitulates human antibody response in mice." Mendez, M.J. et al., Nat. Genet. 15:146-156 (1997)). Humanized antibodies may be prepared using recombinant DNA techniques and the hypervariable regions of monoclonal antibodies. (Methods Enzymol. 203:99-121 (1991)).

The present invention also relates to methods of detecting the activity of a test substance in regulating the transcription-controlling activity of a nuclear receptor complex, wherein the nuclear receptor complex comprises an arbitrary nuclear receptor and a protein encoded by a polynucleotide of any one of the following (A) to (E):
(A) a polynucleotide comprising the coding region of the nucleotide sequence of any of SEQ ID NO: 1, 3, or 5;
(B) a polynucleotide encoding a protein comprising the amino acid sequence of any of SEQ ID NO: 2, 4, or 6;
(C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
(D) a polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
(E) a polynucleotide comprising homology of 80% or more with the nucleotide sequence of any of SEQ ID NO: 1, 3, or 5, encoding a protein that is functionally equivalent to a protein comprising the amino acid sequence of any of SEQ ID NO: 2, 4, or 6;
wherein the method comprises the following steps:
(1) contacting the test substance with a cell expressing the arbitrary nuclear receptor and the protein encoded by the polynucleotide of any one of (A) to (E) , where the cell carries an expression cassette in which a reporter gene is inserted downstream of a response element for the nuclear receptor;
(2) culturing the above cell under conditions that allow expression of the nuclear receptor and the protein encoded by the polynucleotide of any one of (A) to (E) , and measuring the expression level of the reporter gene in the cell; and
(3) detecting the activity of the test substance in controlling the transcription-controlling activity of the above nuclear receptor, using the measurement result of step (2) as an index.

In the methods of the present invention, nuclear receptor complexes are defined as complexes that comprise an arbitrary nuclear receptor and a protein encoded by a polynucleotide of any one of the above (A) to (E). Such complexes may be obtained by simultaneously expressing, in the same cell, DNAs that encode the proteins that compose the complex. Alternatively, these complexes may be obtained by mixing individually purified proteins.

In the above detection methods, proteins functionally equivalent to proteins comprising an amino acid sequence of SEQ ID NO: 2, 4, or 6 are defined as those proteins capable of enhancing the function of an aforementioned nuclear receptor by binding it. SEQ ID NO: 2 shows the amino acid sequence of ERRγ3, a novel protein discovered by the present inventors. SEQ ID NOs: 4 and 6 show the amino acid sequences of ERRγ1 and ERRγ2, respectively. The ERRγ1 and ERRγ2 sequences are known to those skilled in the art. Although ERRγ1 or ERRγ2 themselves were known to comprise transcription-controlling activity, they were not known to comprise the function of enhancing the transcriptional activation function of other nuclear receptors.

Methods for confirming the activity of a given protein in controlling the transcriptional activation function of another nuclear receptor by coexisting with that nuclear receptor molecule are described above.

In the detection methods of the present invention, proteins functionally equivalent to those proteins comprising the amino acid sequence of SEQ ID NO: 2 comprise the above attributes (a) and (b). Thus, proteins comprising the function of controlling the transcriptional activation function of another nuclear receptor, and preferably lacking at least a part of the DBD, are favorable as proteins functionally equivalent to those proteins comprising the amino acid sequence of SEQ ID NO: 2.

Herein, the cells of the present invention which express an arbitrary nuclear receptor and a protein encoded by a polynucleotide of any one of (A) to (E), and which carry an expression cassette in which a reporter gene is connected downstream of a response element for the nuclear receptor, may be obtained as in the following example:

First, an arbitrary nuclear receptor and a protein encoded by a polynucleotide of any one of (A) to (E) may be expressed in cells by transfecting DNAs encoding these nuclear receptors. Herein, the arbitrary nuclear receptor may be estrogen receptor-related receptor (ERR), estrogen receptor (ER), thyroid hormone receptor (TR), glucocorticoid receptor (GR), and such. Moreover, any isoform of these nuclear receptors may be used herein, as long as it comprises transcription-controlling activity. The DNAs which encode these nuclear receptors are publicly known. The GenBank accession numbers of nuclear receptor genes that may be used in the present invention are as follows: human ERα: NM_000125; human TRα: M24748; human GR: NM_000176; human ERβ: NM_001437; and human PPARγ1: NM_005037.

DNAs encoding nuclear receptors capable of controlling the transcriptional activation function of arbitrary nuclear receptors may be DNAs that comprise a coding region of a nucleotide sequence of SEQ ID NO: 1, 3, or 5, for example. In addition to such DNAs, DNAs that encode proteins functionally equivalent to proteins comprising an amino acid sequence of SEQ ID NO: 2, 4, or 6 may also be used herein. Such proteins, which comprise an amino acid sequence of SEQ ID NO: 2, 4, or 6, comprise the function of enhancing the activity of ERR, ER, or TR, as shown in the Examples. Such proteins also comprise the function of inhibiting GR activity.

Such DNAs are obtainable by screening cDNA libraries prepared from humans or other animals, such as nematodes, and yeast using PCR or hybridization based on respective nucleotide sequence information. Alternatively, DNAs comprising the essential nucleotide sequences may be synthesized based on nucleotide sequence information.

The DNAs are cloned in distinct or shared expression vectors, and transfected into cells. The expression vectors include pcDNA3.1 (Invitrogen), pMM324, pSV2dhfr (ATCC 37145), pdBPV-MMTneo (ATCC 37224) , and pSV2neo (ATCC 37149). pcDNA3.1(+) is a vector enabling high level expression of a foreign gene in mammalian cells. When cloning the genes for an arbitrary nuclear receptor and a nuclear receptor that comprises the function of enhancing the transcriptional activation function of that arbitrary receptor into separate vectors, both vectors may be introduced into a cell by co-transfection. The respective vectors may carry different selection markers to confirm the introduction of each vector. Alternatively, the two genes may be cloned into a single vector, and introduced into the cell.

Herein, cells transfected with a DNA encoding a nuclear receptor are those cells capable of translating the DNA into a protein, and comprising an expression cassette wherein a reporter gene is linked down stream of a responsive element for the introduced the arbitrary nuclear receptor. Herein, expression cassettes represent a responsive element and reporter gene set, in which reporter genes are located such that they are transcribed under the regulation of the responsive element. Expression cassettes may be introduced into cells as plasmids, or integrated into the cell genome.

Responsive elements are nucleotide sequences capable of binding to the DBD of a nuclear receptor, and are necessary for the initiation of transcription to mRNA by the transcription unit. For example, responsive elements of the arbitrary nuclear receptors are already known.

For example, the estrogen response element (ERR) comprises the nucleotide sequence of SEQ ID NO: 15 (Lu D., Kiriyama Y., Lee K.Y., and Giguere V. "Transcriptional regulation of the estrogen-inducible pS2 breast cancer marker gene by the ERR family of orphan nuclear receptors." Cancer Res. 61:6755-6761 (2001)). Other response elements for nuclear receptors, such as those for ER, TR, retinoic acid X receptor (RXR), and peroxisome proliferator-activated receptor (PPAR), are also reported in the following literature:
ER: See reference, Klein-Hitpass L., Schorpp M., Wagner U., and Ryffel G.U. "An estrogen-responsive elements derived from the 5' flanking region of the Xenopus vitellogenin A2 gene functions in transfected human cells." Cell 46:1053-1061(1986).
TR: See reference, Brent G.A., Harney J.W., Chen Y., Warne R.L., Moore D.D., and Larsen P.R. "Mutations of the rat growth hormone promoter which increase and decrease response to thyroid hormone define a consensus thyroid hormone response element." Mol. Endocrinol. 3:1996-2004 (1989).
RXR: See reference, Mangelsdorf D.J., Umesono K., Kliewer S.A., Borgmeyer U., Ong E.S., and Evans R.M. "A direct repeat in the cellular retinol-binding protein type II gene confers differential regulation by RXR and RAR." Cell 66:555-561 (1991).
PPAR: See reference, Tugwood J.D., Issemann I., Anderson R.G., Bundell K.R., McPheat W.L., and Green S. "The mouse peroxisome proliferator activated receptor recognizes a response element in the 5' flanking sequence of the rat acyl CoA oxidase gene." EMBO J. 11:433-439 (1992).

Expressing reporter genes under the regulation of these responsive elements may be achieved by replacing a gene under the regulation of a responsive element with a reporter gene. Plasmids comprising an expression cassette that comprises a reporter gene downstream of a responsive element are called reporter plasmids.

Reporter plasmids may be readily constructed using reporter vectors. Reporter vectors comprise reporter genes, and upstream of that, cloning sites for inserting given responsive elements. For example, the reporter vector PGVP2, which comprises the luciferase gene as a reporter, is commercially available. A reporter plasmid for use in the present invention may be obtained by inserting an above responsive element into the cloning site of a reporter vector. Multiple responsive elements may be inserted in tandem to enhance the response to a nuclear receptor. Thus, increased sensitivity in detecting transcriptional activation is expected. The responsive elements to be inserted may be chemically synthesized.

Herein, the reporter genes can be any reporter genes for which signals can be easily measured. Reporter genes for analyzing the gene expression regulatory mechanisms are known. Specifically, genes encoding luciferase, catalase, β-galactosidase, green fluorescent protein (GFP), and such may be used as reporter genes. The cells transfected with the vectors may be, for example, animal cells in which that gene is deleted.

The cells transfected with the different kinds of genes may be animal cells, yeast cells, insect cells, or such. One example of preferable cells in the present invention are the monkey-kidney-derived CV-1 cell line used in the Examples.

Herein, the cells are cultured in the presence of a test substance, under conditions enabling expression of the arbitrary nuclear receptor and the protein encoded by the polynucleotide of any one of (A) to (E). The expression level of the reporter gene in the cells is then measured. Herein, conditions enabling the expression of two nuclear receptors refer to conditions where a nuclear receptor gene comprised in an introduced vector is expressed in the host cells. For example, if the vector utilizes an inducible promoter, the cells are cultured under conditions required to induce expression.

If the arbitrary nuclear receptor functions in a ligand-dependent manner, the cells are preferably provided with the ligand. On the other hand, if the nuclear receptor exhibits the transcription-controlling activity ligand-independently, the above methods may be performed without providing a ligand. For example, ERR is a ligand-independent nuclear receptor, which is used as arbitrary nuclear receptor in Examples.

In the methods of detection of the present invention, test substances are added to the culture to make contact with the above cells. Alternatively, if the test substances are proteins, the genes encoding them may be transfected into the same cells, and contacted with the nuclear receptors. Furthermore, cells expressing a test substance may also be contacted by co-culturing with cells expressing an above nuclear receptor.

Once a nuclear receptor is expressed, the reporter gene is transcribed, and the protein is expressed. The expression level of the reporter gene can be determined by measuring the intensity of the signal originating from this protein. For example, when using the luciferase gene as the reporter gene, luciferase activity can be measured as chemiluminescent intensity. Alternatively, if the reporter gene is GFP, the fluorescent intensity of GFP can be measured.

If a coexisting test substance is capable of regulating the transcription-controlling activity, the transcription level of the reporter gene will change. If the test substance inhibits transcription, reporter gene transcription is suppressed and the signal will be reduced, based on the extent of this change. In contrast, a test substance comprising the function of enhancing transcriptional activation function will increase the reporter gene signal. Therefore, the influence of test substances on transcription-controlling activity can be evaluated using reporter gene transcription level as an index.

The activity of regulating the transcription-controlling activity of a complex of an arbitrary nuclear receptor and a nuclear receptor that enhances its transcriptional activation function can be evaluated by a detection method of the present invention. The method of detection may be used to evaluate substances capable of regulating the transcription-controlling activity. Thus, the present invention relates to methods for evaluating substances capable of regulating a transcription-controlling activity, wherein the methods comprise the following steps: (1) using a method for evaluating the activity of regulating the transcription-controlling activity of a complex of an arbitrary nuclear receptor and a nuclear receptor that enhancing its transcriptional activation function to detect an activity of regulating the transcription-controlling activity of the complex, and
(2) selecting those test substances capable of suppressing or enhancing the transcription-controlling activity of the nuclear receptor, by comparison with a control.

The cells introduced with vectors for a nuclear receptor produce a signal generated from the reporter gene, in line with nuclear receptor expression. The obtained cells are seeded into a 96-well plate, and cultured under conditions that induce expression of the introduced genes. By adding a test substance to be evaluated into each well, the activity of suppressing or enhancing the expression of the nuclear receptor may be readily detected, using reporter gene as an index.

For example, when GFP is used as the reporter gene, it is possible to evaluate influence on the expression regulatory region by comparing the fluorescence intensities of GFP in conditions with or without the test substance. A significant difference may be judged from this comparison when the intensity ratio is, for example, two fold or more, or 1/2 or less, preferably five fold or more, or 1/5 or less, and more preferably ten fold or more, or 1/10 or less. The methods may be applicable not only to animal cells, but to any host cells, regardless of whether they are eukaryotic or prokaryotic cells, as long as the transcriptional activation function of an introduced nuclear receptor can be reproduced. The cells are cultured under conditions that enable reporter gene expression.

The test substances for the evaluation methods of the present invention are not limited. Specifically, they include cell extracts, products expressed from DNA libraries, synthetic low molecular weight compounds, synthetic peptides, and natural compounds, for example. Alternatively, antibodies that recognize a protein encoded by a polynucleotide of any one of the above (A) to (E) may be useful as candidate substances for screening.

Compounds known for their effect on transcription-controlling activity can be used as controls for the evaluation methods of the present invention. More specifically, compounds that clearly have no effect on transcription-controlling activity, for example, may be used as controls. Data measured without any test substance influence can be used as a control for cases without any effect on transcription-controlling activity.

Alternatively, by using substances already known to comprise a target function as controls, substances that induce a larger change in signal may be searched for selecting substances comprising relatively greater function.

The evaluation methods of the present invention may be used, for example, for screening for compounds that comprise desired activities, or for characterizing compounds. For example, screening may be performed by applying the evaluation methods to a broad range of compounds, and repeatedly selecting compounds that excel in the activity of interest. Alternatively, these detection methods may be used to analyze specific compound attributes.

Furthermore, the present invention relates to methods of detecting the activity of test substances in binding to a nuclear receptor, wherein the nuclear receptor is a protein encoded by the polynucleotide of any one of the following (A) to (E):
(A) a polynucleotide comprising a coding region of the nucleotide sequence of SEQ ID NO: 1;
(B) a polynucleotide encoding a protein comprising the amino acid sequence of SEQ ID NO: 2;
(C) a polynucleotide comprising an amino acid sequence wherein one or more amino acids are replaced, deleted, inserted, and/or added to the amino acid sequence of SEQ ID NO: 2, encoding a protein that:
   (a) controls the transcriptional activation function of a nuclear receptor when co-existed with the nuclear receptor; and
   (b) lacks at least part of a DNA binding domain;
(D) a polynucleotide that hybridizes under stringent conditions with a polynucleotide comprising the nucleotide sequence of SEQ ID NO: 1, encoding a protein that:
   (a) controls the transcriptional activation function of a nuclear receptor when co-existed with the nuclear receptor; and
   (b) lacks at least part of a DNA binding domain;
(E) a polynucleotide comprising a nucleotide sequence in which a sequence corresponding to nucleotides 599 to 715 of SEQ ID NO: 5 is deleted or replaced with another nucleotide sequence, and comprising a nucleotide sequence that comprises homology of 70% or more to the above nucleotide sequence in which the sequence corresponding to nucleotides 599 to 715 is deleted;
wherein the method comprises the following steps:
(1) contacting the nuclear receptor, the ligand, and a test substance in any of the following orders i) to iii):
   i) contacting the nuclear receptor and the test substance first, and then contacting with the ligand;
   ii) contacting the nuclear receptor and the ligand in the presence of the test substance; or
   iii) contacting the nuclear receptor and the ligand first, and then contacting with the test substance;
(2) measuring the amount of the ligand or test substance bound to the nuclear receptor; and
(3) detecting the activity of the test substance in binding to the nuclear receptor, using the measurement result of (2) as an index.

In contrast to the aforementioned methods of detecting effect on transcription-controlling activity using behavior within cells as an index, these methods detect the activity of binding to a nuclear receptor based on the interaction between substances. ERRγ3 or proteins functionally equivalent to ERRγ3, which were discovered by the present invention, are the nuclear receptors used in the present invention's methods of detecting a test substance's ability to bind a nuclear receptor. The activity of binding a nuclear receptor can be detected by monitoring competition between ligands.

Herein, ligands, agonists, antagonists or substances known to bind to estrogen receptor-related receptors, for example, can be used as ligands for the above nuclear receptors. Ligands capable of binding to ERRγ1 and ERRγ2 include diethylestradiol (diethylstilbestrol; DES), tamoxifen, 4-hydroxytamoxifen (4-OHT), or such. Tamoxifen comprises the activity of binding to ERR, but does not affect its transcription-controlling activity. 4-OHT and DES are capable of binding ERRγ1 and ERRγ2 and inhibiting their transcriptional activation function. Thus, these ligands may be considered ERRγ1 and ERRγ2 inactivating agents or inhibitors.

In addition, ERRγ3-specific ligands may be used. Such ligands may be identified based on reporter gene assays or measurements of direct binding to nuclear receptors using physicochemical methods. This latter includes methods for measuring competitive binding inhibition using radio isotope-labeled ligands, methods for detecting binding using surface plasmon resonance (SPR) , methods for detecting the bound complex using mass spectrometry (MS), and such.

Thus, candidate substances for ERRγ3 ligands may be evaluated by adding them to cells transfected with ERRγ3 and a reporter plasmid carrying a response element, and detecting the expression level of the reporter gene. Candidate substances that increase reporter gene expression level in a concentration-dependent manner are considered ERRγ3 ligands.

In searching for ligands using physicochemical methods, the function of a candidate substance in binding to a purified ERRγ3 protein can be observed. Binding between substances may be detected rapidly and with high sensitivity using the above analytical methods. Substances capable of binding to ERRγ3 are useful as ligands, or as agonist or antagonist candidates.

Herein, a nuclear receptor, its ligand, and test substance may be contacted with each other in any of the following orders i) to iii): First, i) The function of a test substance in inhibiting ligand binding to a nuclear receptor can be evaluated by contacting the nuclear receptor with a test substance, and then with the ligand. Next, ii) the function of a test substance in competing with a ligand for a nuclear receptor can be evaluated by contacting the nuclear receptor and the ligand in the presence of the test substance. Furthermore, iii) the function of a test substance in replacing a ligand bound to a nuclear receptor can be evaluated by contacting the nuclear receptor with the ligand, and then with the test substance. Compounds identified by these methods are useful as candidates for ERRγ3 agonists or antagonists.

Furthermore, the detection methods of the present invention comprise measuring the amount of ligands or test substances bound to nuclear receptors. The amount of ligand or test substance bound to a nuclear receptor may be measured by labeling these substances. Ligands or test substances may be labeled with radioisotopes, substances with chemiluminescent, fluorescent, or enzymatic active substances, or tags. The amount of ligand or test substance bound to a nuclear receptor may be measured by isolating the nuclear receptor from the reaction mixture, and measuring the label in the isolated fraction. The nuclear receptor may first be immobilized onto a solid phase to facilitate its isolation from the reaction mixture. Such solid phases commonly include magnetic beads, or the inside wall of reaction containers.

In the methods of detection of the present invention, the amount of ligand bound to a nuclear receptor is correlated to the activity of a test substance in binding to that nuclear receptor: when the amount of ligand bound to a nuclear receptor decreases compared with the amount in the absence of the test substance, the test substance is judged capable of binding to the nuclear receptor. Alternatively, when the amount of test compound bound to a nuclear receptor is measured, the test substance is judged capable of inhibiting the interaction between the ligand and the nuclear receptor if the binding of the substance decreases in the presence of the ligand, compared with the amount in the absence of the ligand.

Methods for evaluating compounds capable of binding to nuclear receptors are provided based on the methods for detecting binding activity of the present invention. Specifically, compounds that comprise the ability to bind nuclear receptors can be screened by measuring the ability to bind nuclear receptors, the results of the above detection methods, and then selecting compounds with strong binding ability compared to a control. Substances obtained by the evaluation methods are very likely to function as agonists or antagonists of the nuclear receptors, since these substances are capable of binding to the receptors. Such substances are useful as agents for controlling the activity of ERRγ3, the nuclear receptors of the present invention.

Herein, in the evaluation methods of the present invention, test substances similar to those used in the methods for evaluating the function of regulating transcription-controlling activity may be used. In addition, cases where test substances are not contacted may be used as controls. Alternatively, substances that comprise a binding ability greater that these compounds may be screened by using substances that clearly comprise the ability to bind a nuclear receptor of the present invention as a control.

Compounds isolated by the evaluation methods of the present invention may be candidates for compounds that enhance or inhibit the transcriptional activation function of the nuclear receptors (i.e. agonists and antagonists). They may also be candidates for compounds that enhance or inhibit in vivo the interaction between nuclear receptors, the interaction between a nuclear receptor and a responsive element, or the interaction between a nuclear receptor and a co-activator. These compounds may be applied as medicines for preventing or treating diseases associated with nuclear receptors.

ERRγ3 agonists or antagonists comprising the function of regulating the transcription-controlling activity of ERR, which is involved in estrogen signal transduction, may be useful in treating diseases caused by abnormal estrogen signal transduction. For example, if the ERRγ3 expression level of is lower than that of healthy subjects, estrogen signal transduction is suspected to be defective. Patients with such conditions can be administered with an ERRγ3 agonist to normalize estrogen signal transduction. On the other hand, a patient with elevated estrogen signal transduction may be administered with a compound comprising a function as an ERRγ3 antagonist to return estrogen signal transduction to a normal level.

Examples of diseases with elevated ER activity include breast cancer and cervix cancer. Thus, the ERRγ3 antagonists obtained by the present invention are useful for treating these diseases. Examples of diseases with reduced ER activity include osteoporosis, hyperlipidemia, arteriosclerosis, angina, myocardial infarction, stroke, systemic lupus erythematosus, or Alzheimer's disease. Thus, ERRγ3 agonists of the present invention are useful for treating such diseases.

The kits of the present invention may be further packaged with a medium for cell culture, and culture vessels. The culture vessels are preferably formed in a shape for application in high-throughput screening. The kits may further comprise an instruction manual illustrating the assay method, and a control that is a substance tested for a certain level of effect on the transcription-controlling activity of the nuclear receptor.

Herein, components required for carrying out the present methods for evaluating the activity of regulating the transcription-controlling activity of complexes which comprise an arbitrary nuclear receptor and another nuclear receptor capable of enhancing the transcriptional activation function of that nuclear receptor, can be provided in kits. The kits of the present invention comprise cells that express a polynucleotide encoding an arbitrary nuclear receptor and a polynucleotide of any one of the following (A) to (E), wherein a reporter gene can be expressed under the regulation of a response element of the above arbitrary nuclear receptor.
(A) a polynucleotide comprising a coding region of the nucleotide sequence of SEQ ID NO: 1;
(B) a polynucleotide encoding a protein comprising the amino acid sequence of SEQ ID NO: 2;
(C) a polynucleotide comprising an amino acid sequence wherein one or more amino acids are replaced, deleted, inserted, and/or added to the amino acid sequence of SEQ ID NO: 2, encoding a protein that:
   (a) controls the transcriptional activation function of a nuclear receptor when co-existed with the nuclear receptor; and
   (b) lacks at least part of a DNA binding domain;
(D) a polynucleotide that hybridizes under stringent conditions with a polynucleotide comprising the nucleotide sequence of SEQ ID NO: 1, encoding a protein that:
   (a) controls the transcriptional activation function of a nuclear receptor when co-existed with the nuclear receptor; and
   (b) lacks at least part of a DNA binding domain;
(E) a polynucleotide comprising a nucleotide sequence in which a sequence corresponding to nucleotides 599 to 715 of SEQ ID NO: 5 is deleted or replaced with another nucleotide sequence, and comprising a nucleotide sequence that comprises homology of 70% or more to the above nucleotide sequence in which the sequence corresponding to nucleotides 599 to 715 is deleted.

Polynucleotides encoding arbitrary nuclear receptors and polynucleotides of any of the following (A) to (E) can be provided such that one is supplied as a component of the kits, while the other is freely combined by the user of the kit. For this purpose, a vector for expression of a nuclear receptor may be comprised in a kit. If a user chooses an arbitrary nuclear receptor, a reporter vector may be packed in the kit, enabling the user themselves to construct a reporter plasmid carrying a response element for the nuclear receptor of interest.

In addition, components required for carrying out the present methods for evaluating the activity of binding to the nuclear receptor ERRγ3 of the present invention, can be provided as kits. For example, the kits of the present invention may comprise the nuclear receptor protein ERRγ3 of the present invention (or a protein functionally equivalent to it) , and a labeled ligand. The nuclear receptor protein ERRγ3 (or proteins functionally equivalent) may be provided in a pre-immobilized form. As a positive control, the kit may also comprise a substance known for its ability to bind to the nuclear receptor protein ERRγ3 (or a functionally equivalent protein). Furthermore, the kits may comprise other components required for measuring labeled components.

The present invention further relates to the medicinal use of substances obtained by the evaluation methods. Thus, the invention relates to the use of compounds selected by the evaluation methods of the present invention in the regulation of the transcription-controlling activity of a nuclear receptor. The invention also relates to therapeutic agents for diseases characterized by nuclear receptor abnormalities, particularly comprising the above compounds as effective ingredients. In addition, the invention relates to methods of regulating the transcription-controlling activity of a nuclear receptor, comprising the step of administrating compounds selected by the evaluation methods. The invention also relates to the use of compounds selected by the evaluation methods for producing agents that regulate the transcription-controlling activity of nuclear receptors.

Herein, diseases characterized by nuclear receptor abnormalities refer to diseases caused by increased or decreased levels of nuclear receptor expression or activity. Such diseases include a breast cancer, cervix cancer, osteoporosis, hyperlipidemia, arteriosclerosis, angina, myocardial infarction, stroke, systemic lupus erythematosus, or Alzheimer's disease, and attention deficit hyperactivity disorder (ADHD).

Furthermore, the present invention relates to agents that regulate the transcription-controlling activity of the nuclear receptors, comprising a polynucleotide of any one of the following (A) to (E), or a protein encoded by that polynucleotide as the effective ingredient. In addition, the present invention relates to methods of controlling the activity of a nuclear receptor, comprising the step of administrating a polynucleotide any one of the following (A) to (E) , or a protein encoded by that polynucleotide. The invention also relates to the use of a polynucleotide any one of the following (A) to (E), or a protein encoded by that polynucleotide, in the production of agents that regulating the transcription-controlling activity of a nuclear receptor.
(A) a polynucleotide comprising the coding region of the nucleotide sequence of any of SEQ ID NO: 1, 3, or 5;
(B) a polynucleotide encoding a protein comprising the amino acid sequence of any of SEQ ID NO: 2, 4, or 6;
(C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
(D) a polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
(E) a polynucleotide comprising a sequence with homology of 80% or more to the nucleotide sequence of any of SEQ ID NO: 1, 3, or 5, encoding a protein that is functionally equivalent to a protein comprising the amino acid sequence of any of SEQ ID NO: 2, 4, or 6.

The present invention also relates to agents that inhibit the activity of a nuclear receptor, comprising any one of the following (1) to (4) as an effective ingredient. In addition, the invention relates to methods of inhibiting the activity of a nuclear receptor, comprising the step of administrating any one of the following (1) to (4). The invention also relates to the use of any one of the following (1) to (4) in producing agents that inhibit the activity of a nuclear receptor.
(1) An antisense polynucleotide of a polynucleotide of the above (A) to (E);
(2) an antibody that recognizes a protein encoded by a polynucleotide of the above (A) to (E);
(3) a protein conferring a dominant negative effect on a protein encoded by a polynucleotide of the above (A) to (E); and
(4) a double-stranded RNA of 21 to 23 base pairs, comprising a sense RNA corresponding to a partial sequence of a polynucleotide of the above (A) to (E), and its antisense RNA.

The agents that regulate or inhibit the activity of a nuclear receptor are used for treating diseases characterized by inhibiting or enhancing transcriptional activation function of nuclear receptors.

The proteins, polypeptides, antibodies and proteins with dominant negative form of the present invention, and the substances isolated by the evaluation methods, are useful for regulating the transcription-controlling activity of nuclear receptors. Herein, these may be used as medicines by themselves, or formulated as drugs by using common pharmaceutical methods. For example, they may be formulated by appropriately mixing with a pharmacologically acceptable carrier or vehicle; specifically, sterile water, saline, plant oils, emulsions, suspensions and such.

Administration to patients may be performed by standard methods known to those skilled in the art, such as arterial injection, intravenous injection, and subcutaneous injection. Dosages may vary depending on patient body weight and age, or the method of administration; however, one skilled in the art can appropriately select a suitable dose. If the compound can be encoded by DNA, the DNA may be incorporated into a gene therapy vector to perform gene therapy.

The dosages and methods of administration may vary depending on patient body weight, age, or symptoms. Those skilled in the art can appropriately select suitable dosages and methods of administration.

Furthermore, the present invention relates to model animals in which the activity of a nuclear receptor is regulated, comprising transgenic non-human animals in which the expression of a protein of any one of the following (A) to (D) is controlled:
(A) A protein comprising the amino acid sequence of any of SEQ ID NO: 2, 4, or 6;
(B) A protein encoded by a DNA that hybridizes under stringent conditions with a DNA comprising the nucleotide sequence of any of SEQ ID NO: 1, 3, or 5, and which is functionally equivalent to a protein comprising the amino acid sequence of any one of SEQ ID NO: 2, 4, or 6;
(C) A protein comprising an amino acid sequence wherein one or more amino acids are replaced, deleted, inserted, and/or added to the amino acid sequence of any one of SEQ ID NO: 2, 4, or 6, and which is functionally equivalent to a protein comprising the amino acid sequence of any one of SEQ ID NO: 2, 4, or 6; and
(D) A protein comprising an amino acid sequence that compriseshomology of 80% or more to the amino acid sequence of any one of SEQ ID NO: 2, 4, or 6, and which is functionally equivalent to a protein comprising the amino acid sequence of any one of SEQ ID NO: 2, 4, or 6.

For example, transgenic animals over-expressing a protein of any of the above (A) to (D) may be useful as model animals in which diseases of elevated transcription activation function of nuclear receptors can be induced. Examples of such diseases include breast cancer, cervix cancer, and attention deficit hyperactivity disorder (ADHD). Thus, the model animals of the present invention can be used to evaluate therapeutic agents for the above diseases.

Furthermore, the present invention relates to methods for diagnosing diseases that arise from abnormal nuclear receptor activity, where the methods comprise the steps of measuring the expression level of a polynucleotide of any of the below (A) to (E) in a biological sample collected from a subject, and correlating this with diseases arising from abnormal nuclear receptor activity.
(A) a polynucleotide comprising the coding region of the nucleotide sequence of any of SEQ ID NO: 1, 3, or 5;
(B) a polynucleotide encoding a protein comprising the amino acid sequence of any of SEQ ID NO: 2, 4, or 6;
(C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
(D) a polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
(E) a polynucleotide comprising a sequence with homology of 80% or more to the nucleotide sequence of any of SEQ ID NO: 1, 3, or 5, encoding a protein that is functionally equivalent to a protein comprising the amino acid sequence of any of SEQ ID NO: 2, 4, or 6.

The expression level of the polynucleotides may be measured by, for example, quantifying mRNA, or protein, which is the translated product, or using the protein activity as an index. Methods for quantifying mRNA or protein are commonly known. For example, cells can be collected from a target organ for a disease of a test subject by biopsy, an RNA fraction can be prepared using standard methods, and quantitative RT-PCR using a sequence specific to ERRγ3 as a primer can be performed, and the expression level of ERRγ3 in the disease organ can thus be measured.

Proteins encoded by the above polynucleotides of any one of (A) to (E) are capable of enhancing transcriptional activation function of the arbitrary nuclear receptors. Thus, by using this effect as an index, expression levels may be determined based on the activity of these proteins.

If a significant difference is found on comparing the obtained data with that of normal subjects, the transcription-controlling activity of the nuclear receptor is suspected to be abnormal. Alternatively, diseases caused by abnormal ERRγ3 activity can be diagnosed using the mutation or polymorphism of the ERRγ3 genes.

For example, breast cancer and cervix cancer are considered to be caused by elevated ER activity. Osteoporosis, hyperlipidemia, arteriosclerosis, angina, myocardial infarction, stroke, systemic lupus erythematosus, and Alzheimer's disease are considered to be caused by deficient ER function. ADHD is thought to result from elevated TR activity. ERRγ3 comprises the function of enhancing ER or TR activity. Thus, diseases caused by elevated activity may be carrying a genetic mutation that leads to enhanced ERRγ3 activity. In contrast, diseases caused by functional deficit may comprise a genetic mutation that causes decreased ERRγ3 activity. On proving a correlation between an ERRγ3-related genetic mutation, such as a SNP, and sensitivity to an above-mentioned disease, blood samples can be collected from subjects, the mutation can be identified using a method for measuring ERRγ3 gene mutations (PCR-SSCP, and such) , and this information can be utilized to predict the disease's development or to analyze its mechanism.

### Brief Description of the Drawings

Fig. 1 is a schematic map of the pME18SFL3 vector.
Fig. 2 shows the nucleotide sequence of the cDNA for ERRγ3 (C-BRAWH2017250), and its deduced amino acid sequence.
Fig. 3 shows the nucleotide sequence of the cDNA for ERRγ3 (C-BRAWH2017250), and its deduced amino acid sequence. (Continued from Fig. 2)
Fig. 4 shows the nucleotide sequence of the cDNA for ERRγ3 (C-BRAWH2017250), and its deduced amino acid sequence. (Continued from Fig. 3)
Fig. 5 shows the nucleotide sequence of the cDNA for ERRγ3 (C-BRAWH2017250). (Continued from Fig. 4)
Fig. 6 shows the nucleotide sequence of the cDNA for ERRγ3 (C-BRAWH2017250). (Continued from Fig. 5)
Fig. 7 shows the results of comparing the nucleotide sequences of the cDNAs for ERRγ3 (C-BRAWH2017250) and ERRγ2. The nucleotide sequence of ERRγ3 (C-BRAWH2017250) is shown at the top (3479 bp) , and that of ERRγ2 is shown at the bottom (2985 bp). Matched bases are shown by a "*".
Fig. 8 shows the results of comparing the nucleotide sequences of the cDNAs for ERRγ3 (C-BRAWH2017250) and ERRγ2. (Continued from Fig. 7)
Fig. 9 shows results of comparing the nucleotide sequences of the cDNAs for ERRγ3 (C-BRAWH2017250) and ERRγ2. (Continued from Fig. 8)
Fig. 10 shows results of comparing the nucleotide sequences of the cDNAs for ERRγ3 (C-BRAWH2017250) , and ERRγ2. (Continued from Fig. 9)

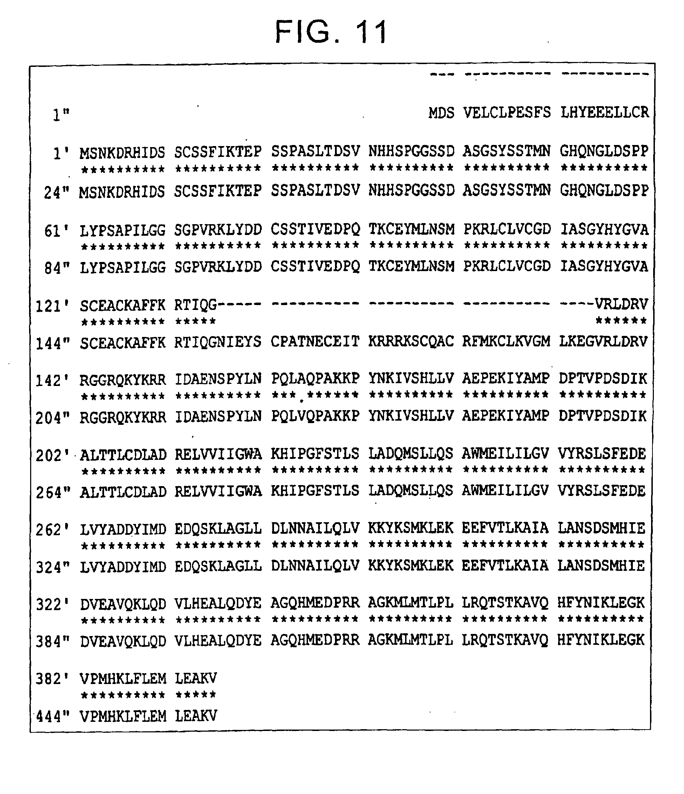

Fig. 11 shows the results of comparing the deduced amino acid sequence of the cDNAs for ERRγ3 (C-BRAWH2017250), and ERRγ2. The amino acid sequence on the top is that of ERRγ3 (C-BRAWH2017250) (396 a.a.), and on the bottom is that of ERRγ2 (458 a.a.). "-" indicates missing amino acids, and "*" indicates matching amino acids.
Fig. 12 schematically shows the deleted zinc finger in ERRγ3. The deleted zinc finger in ERRγ3 (C-BRAWH2017250) is shown in plain font.
Fig. 13 shows the results of a genomic sequence search for ERRγ3 (C-BRAWH2017250) and ERRγ2, and predicted exons based on these results. BRAWH represents the exons predicted for ERRγ3 (C-BRAWH2017250). The unique exons for each isoform are shaded, or crossed.
Fig. 14 shows the result of analysis of the expression level of ERRγ3 (C-BRAWH2017250; lane 4) , and ERRγ2 (lane 3) in skeletal muscle and adipocytes. Lane 1 shows a 100 base pair ladder, and lane 2 shows the expression level of GAPDH.
Fig. 15 shows the results of analysis of the transcription-controlling activity of ERRγ3 (C-BRAWH2017250) alone, and the controlling function of ERRγ3 on the transcription-controlling activity of ERRγ2. The concentration of 4-hydroxytamoxifen is shown on the X-axis, and the corrected luciferase activity values are shown on the Y-axis.
Fig. 16 shows the enhancing function of ERRγ3 on the transcriptional activation effect of ERα in the presence of a ligand. The concentration of 4-hydroxytamoxifen is shown on the X-axis, and the corrected luciferase activity values are shown on the Y-axis.
Fig. 17 shows the enhancing function of ERRγ3 on the transcriptional activation effect of ERβ in the presence of a ligand. The concentration of 4-hydroxytamoxifen is shown on the X-axis, and the corrected luciferase activity values are shown on the Y-axis.
Fig. 18 shows the enhancing function of ERRγ3 on the transcriptional activation effect of TR in the presence of ligand. The concentration of 4-hydroxytamoxifen is shown on the X-axis, and the corrected luciferase activity values are shown on the Y-axis.
Fig. 19 shows the effect of 4-hydroxytamoxifen on the enhancing function of ERRγ3 on the transcriptional activation function of ERα, in the absence of β-estradiol. The concentration of 4-hydroxytamoxifen is shown on the X-axis, and the corrected luciferase activity values are shown on the Y-axis.
Fig. 20 shows the effect of 4-hydroxytamoxifen on the enhancing function of ERRγ3 on the transcriptional activation function of ERα, in the presence of β-estradiol. The concentration of 4-hydroxytamoxifen is shown on the X-axis, and the corrected luciferase activity values are shown on the Y-axis.
Fig. 21 schematically shows the enhancement of ERα's transcriptional activation function by ERRγ3.
Fig. 22 shows the controlling function of ERRγ3 on the transcriptional stimulation activity of RAR in the presence of retinoic acid. The concentration of 4-hydroxytamoxifen is shown on the X-axis, and the corrected luciferase activity values are shown on the Y-axis.
Fig. 23 shows the controlling function of ERRγ3 on the transcriptional stimulation activity of VDR in the presence of calcitriol. The concentration of 4-hydroxytamoxifen is shown on the X-axis, and the corrected luciferase activity values are shown on the Y-axis.
Fig. 24 shows the controlling function of ERRγ3 on the transcriptional stimulation activity of PPARα, β, and γ in the presence of bezafibrate, carbacyclin, and rosiglitazone. The concentration of 4-hydroxytamoxifen is shown on the X-axis, and the corrected luciferase activity values are shown on the Y-axis.
Fig. 25 shows the inhibitory function of ERRγ3 on the transcriptional activation function of GR in the presence of dexamethasone. The concentration of 4-hydroxytamoxifen is shown on the X-axis, and the corrected luciferase activity values are shown on the Y-axis.
Fig. 26 shows the results of distribution analysis using TaqMan PCR of the expression of ERRγ3 in mouse tissues.

### Best Mode for Carrying Out the Invention

This invention will be explained in further detail below with reference to Examples, but it is not to be construed as being limited thereto. In addition, all reference to prior art cited in the present invention is comprised herein by reference.

### [Example 1] Construction of a cDNA library by oligo-capping

### (1) Extraction of mRNA

mRNA, extracted from human brain (the whole brain tissue) as total RNA, was purchased (CLONTECH # 64020-1).

### (2) Construction of cDNA libraries

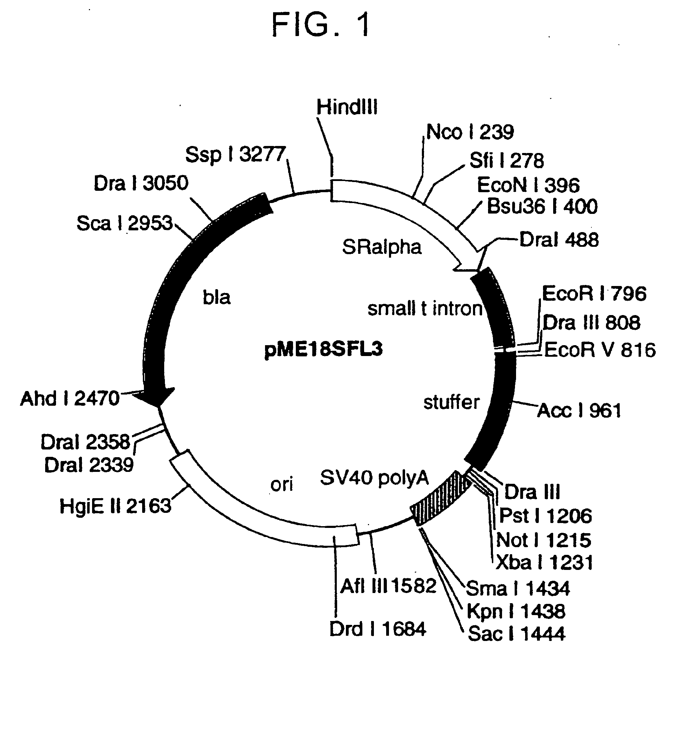

cDNA libraries were constructed from RNA using a method (WO 01/04286) modified from oligo-capping (M. Maruyama and S. Sugano, Gene 138: 171-174 (1994)). Using an oligo-cap linker (SEQ ID NO: 7), and an oligo-dT primer (SEQ ID NO: 8), BAP (bacterial alkaline phosphatase) treatment, TAP (tobacco acid pyrophosphatase) treatment, RNA ligation, first strand synthesis, and RNA removal were performed as described in WO 01/04286. Then, the cDNA was converted to double-stranded cDNA by PCR (polymerase chain reaction) using 5'-(SEQ ID NO: 9) , and 3'- (SEQ ID NO: 10) PCR primers, and then digested with SfiI. Then, cDNA fragments were typically fractioned to obtain cDNAs of 2 kb or longer (3 kb or longer in some case), which were unidirectionally cloned into Drain-digested pME18SFL3 vectors (Fig. 1) (GenBank AB009864, Expression vector) to construct cDNA libraries (BRAWH).

cDNA libraries containing a high percentage of full-length clones (the average percentage of full-length 5'-ends for each library was 90%, calculated using the coding regions of known mRNAs as indicators) were constructed using a method modified from the oligo-capping method using the pME18SFL3 expression vector, which enables expression in eukaryotic cells. The pME18SFL3 vector contains a SRα promoter and an SV40 small t intron upstream of the cloning sites, and a SV40 polyA addition signal downstream. As the cloning sites contain asymmetric Drain sites, and the cDNA fragments contain SfiI sites complementary to the DraIII sites at their ends, cloned cDNA fragments are unidirectionally inserted downstream of the SRα promoter. Therefore, clones containing full-length cDNA can be expressed transiently by introducing obtained plasmid into cells such as COS cells. Thus, it is very easy to experimentally analyze the gene products as a protein, or their biological activity.

### [Example 2] Analysis of the terminal sequence of cDNA clones and selection of clones for analyzing the full-length nucleotide sequence

The nucleotide sequences of the 5'-end of the cDNA clones obtained from cDNA libraries were analyzed on a DNA sequencer (ABI PRISM 3700, PE Biosystems). Sequencing reactions were performed according to the manuals and by using DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, PE Biosystems). The data obtained was stored in a database.

The analyzed 5'-end sequences of the cDNA clones were searched for homology using BLAST, targeting recorded data of complete coding regions (CDS) in GenBank and UniGene. Sequences identical to the human mRNA sequences were removed. The sequences were then clustered into groups, whereby clones comprising homology of 90% or more and a consensus sequence of 50 bases or longer were deemed to be in the same group. Clones with a longer 5'-terminus were selected from the groups, and, as necessary, the 3'-end sequences of these selected clones were analyzed and obtained in the same way as for the 5'-end sequences. By analyzing these obtained terminal sequences, clones making contig at the 5'- and 3'- ends were removed. BLAST homology searches were performed again, as described above, to remove sequences identical to human mRNA sequences (including sequences patented or in patent applications). Clones whose full-length sequence was to be analyzed were obtained from the selected clones.

### [Example 3] Analysis of full-length nucleotide sequences

The nucleotide sequence of each full-length cDNA was determined for those clones selected for full-length sequence analysis. The nucleotide sequences were determined mainly by primer walking with dideoxy terminator methods using custom-synthesized DNA primers. Specifically, sequence reactions were performed using custom-synthesized DNA primers, and DNA sequencing reagents, according to the manual (PE Biosystems). Nucleotide sequences were analyzed on a sequencer from the same company (PE Biosystems). Some clones were also analyzed using a DNA sequencer from Licor.

Some clones were subjected to the shotgun method, which randomly cuts plasmids comprising cDNAs without using custom primers, to determine the nucleotide sequence with a DNA sequencer in the same way. Full-length sequences were finally obtained by overlapping the partial nucleotide sequences determined as above.

These full-length nucleotide sequences were used to predict the regions translated into proteins, and to deduce amino acid sequences. BLAST homology searches were performed on databases containing the determined nucleotide sequences. The clone C-BRAWH2017250, which showed high homology to nuclear receptor ERRγ, was discovered.

### [Example 4] Analysis of the ERRγ3 (C-BRAWH2017250) cDNA nucleotide sequence, and comparison with the cDNA sequences of known ERRγ isoforms

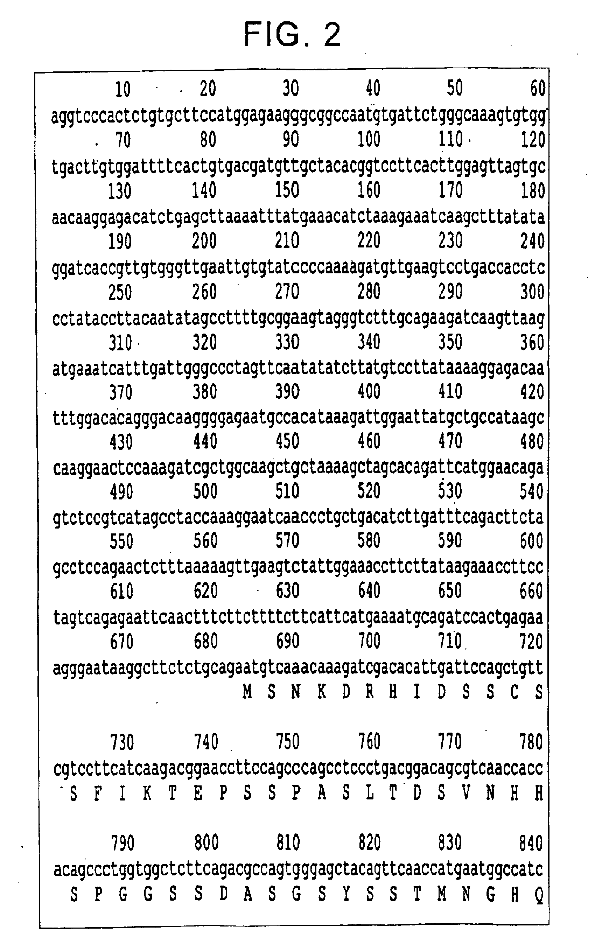

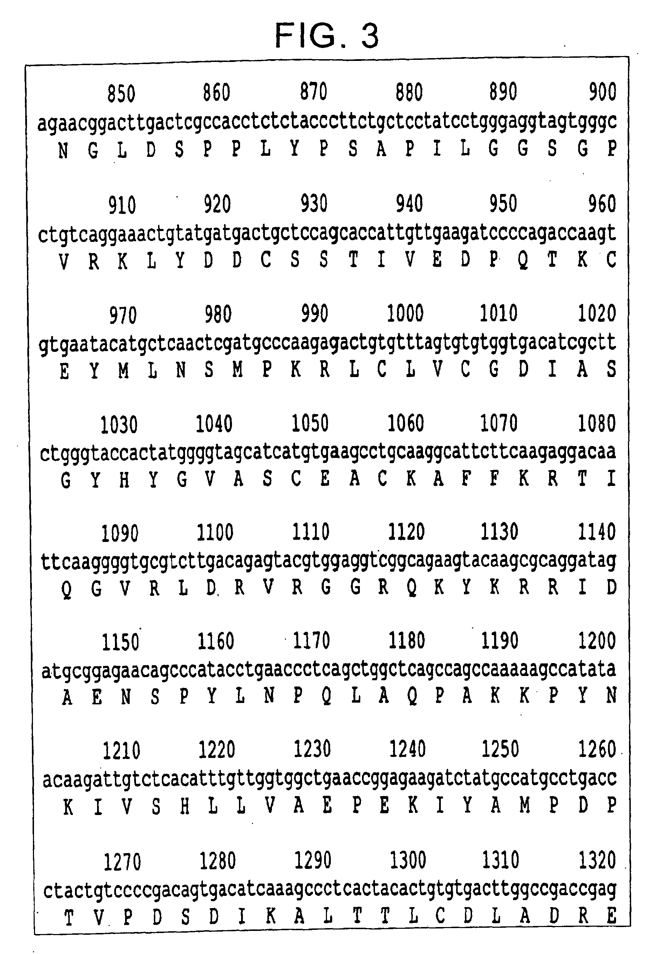

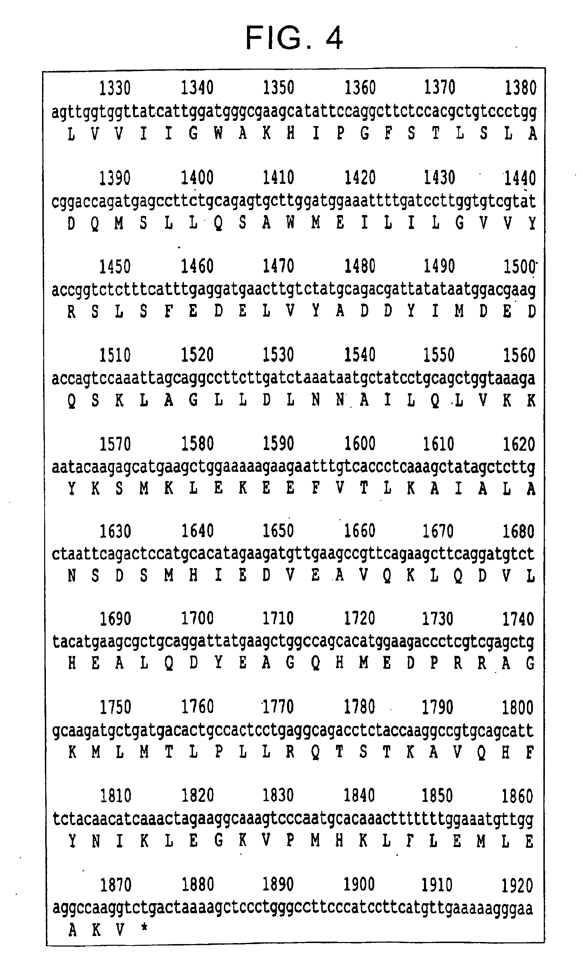

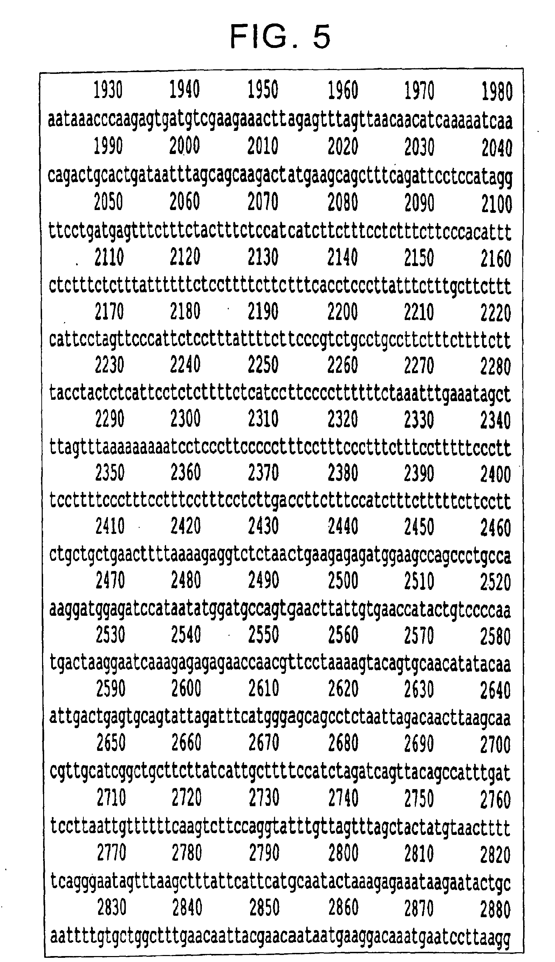

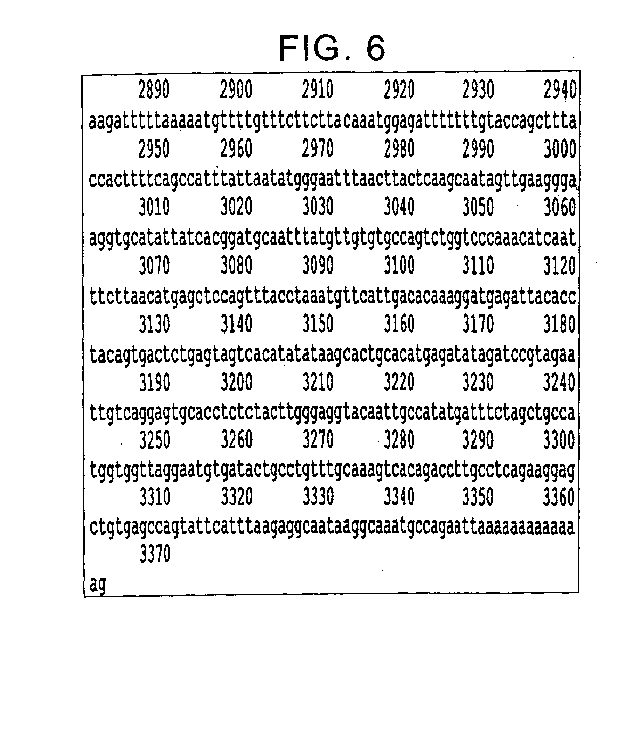

The ERRγ3 (C-BRAWH2017250) cDNA of 3362 base pairs (Figs. 2-6; SEQ ID NO: 1) was subjected to a sequence homology search as well as a search for functional motifs using the DNA sequence analysis software bioSCOUT (Lion) in the databases of GenBank, EMBL, and SWISS-PROT. Furthermore, the sequence was compared with those of the previously reported ERRγ isoforms based on the information in the literature (Figs. 7-10).

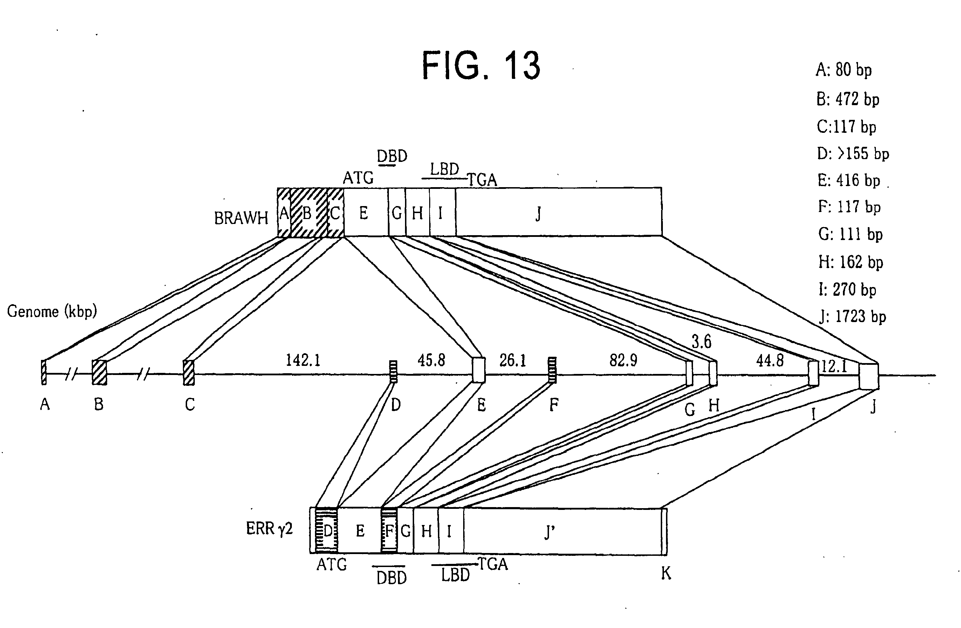

ERRγ3 contains a region of 1188 base pairs (684-1874) that encodes a protein of 396 amino acid residues, containing the DNA binding domain (DBD) and ligand-binding domain (LBD) that are common to the nuclear receptor family. The sequences revealing the highest homology with the cDNA were those of the two ERRγ isoforms: ERRγ1 (the short form) and ERRγ2 (the long form). Comparison of the respective amino acid sequences revealed that ERRγ3 has an N-terminal sequence that is 23 amino acids shorter than that of ERRγ2, and that the DBD common to ERRγ1 and ERRγ2 has a 39 amino acid deletion in ERRγ3. This deletion occurs in a region corresponding to the downstream zinc finger of the two zinc finger structures in the DBD (Fig. 13).

### [Example 5] In silico mapping of the ERRγ3 (C-BRAWH2017250) cDNA in human chromosomes

The ERRγ3 (C-BRAWH2017250) cDNA nucleotide sequence was searched against sequences derived from the GenBank and Sanger Centre human genomic sequence databases. With the exception of the short sequences at the 5'- and 3'-ends, which did not show any hits, the cDNA sequence matched sequences of multiple clones of the human chromosome 1q41. Thus, the exon sequences were determined (Fig. 13).

These results indicated that the ERRγ3 gene comprises at least nine exons. Furthermore, the differences between the cDNA of ERRγ3 (C-BRAWH2017250) and those of the known ERRγ isoforms were not clearly artifacts. This was clearly demonstrated since 1) the boundary sequences of the intron sequences, which were unambiguously determined from these exons, fulfill the GT-AT rule, and 2) the deleted sequence corresponding to the DBD perfectly matches an exon of the known ERRγ2 isoform.

### [Example 6] Evaluation of ERRγ3 cDNA expression in human tissues

The tissue distribution of ERRγ3 or ERRγ2 expression was examined by PCR, using commercially available cDNA from human tissues as a template, and isoform-specific primer sets. 20 µL of PCR reaction mixture contains 1 µg of cDNA from human heart, kidney, liver, spleen, brain, or skeletal muscle, 2 µl of 10x Ex Taq Buffer, 1.6 µl of dNTP mixture, 1 µl of Ex Taq (TAKARA SHUZO), 1 µl of Perfect Match PCR Enhancer (STRATAGENE) , 1 µl of Forward primer, 1 µl of Reverse primer, and 11.4 µl of sterile water.

The nucleotide sequence of the primers is as follows:
For ERRγ3 amplification: 
exerrg3s (CCACTGAGAAAGGAATAAGGCT / SEQ ID NO: 11)
exerrg3a (TGTTATATGGCTTTTTGGCTGGCT /SEQ ID NO: 12)
For ERRα2 amplification:

exerrg2s (CTTTTTCCCTGCACTACGA / SEQ ID NO: 13)
exerrg2a (GACCTCCACGTACTCTGTC / SEQ ID NO: 14)

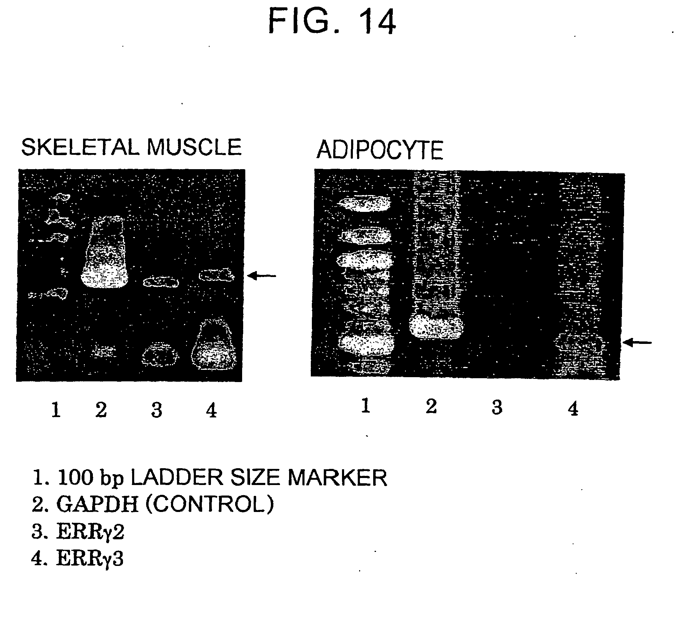

PCR amplification was performed for 35 cycles of 94°C for 1 minute, 55°C for 2 minutes, and 72°C for 3 minutes. Glyceraldehyde-3-phosphate dehydrogenase (GAPDH) was also amplified by PCR from each tissue under the same conditions, and used as an expression level control. ERRγ2 expression was detected in heart, kidney, spleen, skeletal muscle, placenta, and prostate; expression of ERRγ3 was limited to skeletal muscle, prostate, and adipocytes (Fig. 14) .

### [Example 7] Construction of a reporter gene assay system for evaluation of the transcription-controlling activity of ERRγ3

In order to construct a reporter gene assay system for evaluation of the transcription-controlling activity of ERRγ3, a reporter plasmid into which ERE (estrogen response element) was inserted was prepared along with expression plasmids for ERRγ3 and ERRγ2, as follows: A reporter gene assay was performed by transfecting the reporter plasmid and expression plasmids into cultured cells by lipofection, followed by incubating the cells for a certain time, treating the cells with cell lysis solution, and measuring reporter activity with a luminometer using a Dual luciferase assay.

ERE reporter plasmid was constructed by inserting multiple synthetic DNAs, corresponding to an ERE sequence, into the BglII site of the luciferase reporter vector PGVP2 (Wako Pure Chemical Industries, Ltd.) in tandem. Synthetic DNAs of 21 mer (5'-GATCTAGGTCACAGTGACCTA - 3'/SEQ ID NO: 15; 5'-GATCTAGGTCACTGTGACCTA -3'/ SEQ ID NO: 16), corresponding to ERE (estrogen receptor response element) and complementary to each other, were phosphorylated at the 5'-end with T4 polynucleotide kinase. The complementary strands were mixed at a 1:1 ratio, and annealed by heat denaturation at 65°C for 15 minutes, then slowly cooled to prepare the insert DNA fragment. The fragment was mixed with BglII-digested PGVP2 vector, ligated with T4 DNA ligase, and used to transform *E*.*coli* DH5α.

Transformation was performed using commercial *E.coli* DH5α competent cells (TOYOBO) and standard methods, and transformants were selected for ampicillin resistance. The obtained transformants were examined by colony PCR for the presence or absence and size of the insert fragment in the recombinant plasmid. The nucleotide sequences of clones containing multiple inserts was then examined, and the number of inserted response elements and their orientation was confirmed. From these, a clone containing four response elements, in tandem, was selected as a reporter plasmid.

Expression plasmids for ERRγ3 and ERRγ2 were constructed by inserting sequences comprising coding regions into the multi-cloning site of expression vector pcDNA3.1(+). The insert fragments were prepared by PCR amplification using pME18SFL3-C- BRAWH2017250 as a template for ERRγ3 (C-BRAWH2017250). The control ERRγ2 was prepared by PCR using cDNA from human skeletal muscle as a template.. The primers used for PCR were as follows:
For ERRγ3: 
5'-CGCGGATCCTCTGCAGAATGTCAAAC-3'/ SEQ ID NO: 17
5'-CGCCTCGAGGAAAGAAACTCAGG-3'/ SEQ ID NO: 18
For ERRγ2:

5'-TTTGGATCCTCGCAC ATGGATTCGGTA-3'/ SEQ ID NO: 19
5'-GGGCTCGAGAAGAAAGAGGAAAGAAGA-3'/ SEQ ID NO: 20

PCR amplification was performed on 35 cycles of 94°C for 1 minute, 55°C for 2 minutes, and 72°C for 3 minutes. The resulting insert fragments and expression vectors were digested with BamHI and XhoI, separated and purified by electrophoresis on an agarose gel, and then ligated with T4 DNA ligase. The reaction mixture was used to transform *E.coli* DH5α cells, and transformants were selected for ampicillin resistance. The obtained transformants were checked by colony PCR for the presence of inserts, and the nucleotide sequence was determined to confirm the sequence. Nucleotide substitution due to PCR was observed. Additional recombination experiments were conducted to eliminate this, whereby restriction enzyme fragments comprising mutations were replaced by fragments without mutations, which were derived from other clones.

The reporter plasmid and expression plasmid obtained as above were prepared by large-scale culture of bacterial strains carrying the respective plasmid. The DNA was then prepared using a commercial plasmid DNA preparation kit (Qiagen), and subjected to a reporter gene assay.

### [Example 8] The transcription-controlling activity of ERRγ3 and its function in ERRγ2 transcription control

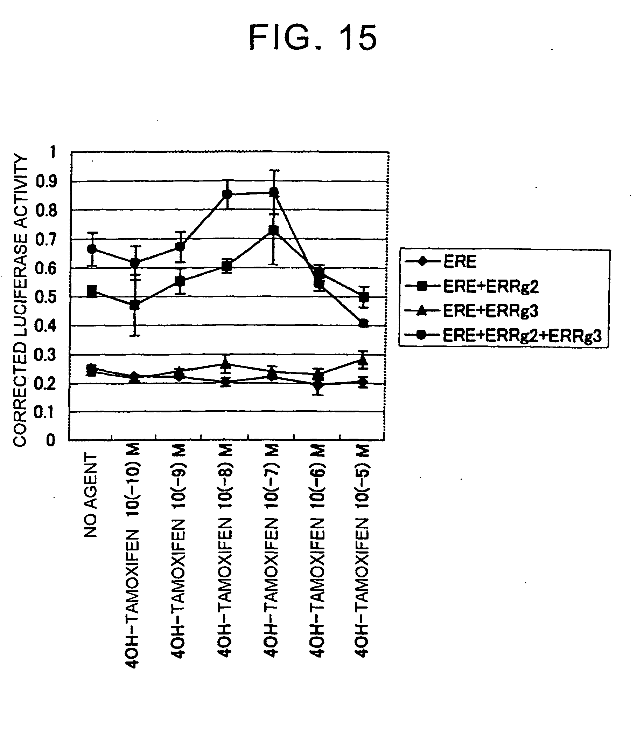

The transcription-controlling activity of ERRγ3 was evaluated using a reporter gene assay. The transcription-controlling activity of ERRγ3 by itself, and ERRγ3's function in ERRγ2 transcription control, were examined using the former ERRγ3 transcription-controlling activity as a control. In addition, since 4-hydroxytamoxifen is known to inhibit ERRγ2 activity, the response of the above controlling activities to 4-hydroxytamoxifen was evaluated (Fig. 15).

The reporter gene assay was performed using CV-1 cells (African green monkey kidney-derived cell line) as hosts. Host cells were transfected with luciferase reporter plasmids and expression plasmids for ERRγ3, ERRγ2, or both. They were then exposed to 4-hydroxytamoxifen at different concentrations (0, 10⁻¹⁰ to -10⁻⁵ M) for 24 hours, whereupon luciferase activity within the cells was measured.

Transfection was performed as follows: CV-1 cells were suspended at 2 x10⁵ cells/2.5 ml in DMEM medium supplemented with 10% fetal bovine serum, 2000 U/l of penicillin G, and 100 mg/l streptomycin. These cells were seeded to a 6-well plate at 2.5 ml/well, and cultured for 18 to 24 hours in a CO₂ incubator at 37°C in the presence of 5% CO₂. 1 µg of each of the transcription factor expression plasmid, the luciferase reporter plasmid, and another luciferase reporter plasmid for normalizing the measured value (pRL-TK) , were dissolved in Opti-MEM (Gibco BRL) to total of 150 µl. 7 µl of the cell transfection reagent LipofectAMINE 2000 (Gibco BRL) was diluted in 143 µl of Opti-MEM, and this was added to the mixture drop by drop while stirring. The mixture was incubated for 20 minutes.

The LipofectAMINE-DNA mixture was diluted in 1.5 ml of Opti-MEM, and added to cells that had been twice washed with 1 ml of PBS(-). The cells were cultured for four hours. The transfection reagent was removed from the culture, and 200 µl of trypsin/EDTA was added to the cells, which were then incubated at 37°C for five minutes. 1 µl of DMEM was added to suspend the cells, which were transferred to a centrifuge tube, and centrifuged for three minutes at 1000 rpm. After centrifugation, the cells were resuspended in DMEM to a concentration of 1.6 x10⁴ cells/100 µl, and 100 µl samples were seeded to a 96-well plate. When evaluating agents, a two-fold concentrated solution was made by pre-diluting a 1000 times concentrated agent DMSO solution with culture medium. 50 µl of the solution was mixed with 50 µl of a cell suspension at 1.6 x10⁴ cells/50 µl, and the culture was incubated in a CO₂ incubator at 37°C and 5% CO₂ for 24 hours.

Luciferase activity was measured using the Dual-Luciferase™ Reporter Assay System (Promega). The culture supernatant was removed from each well of the 96-well plate and washed once with 100 µl of PBS(-). 20 µl of x 1 PLB (Passive Lysis Buffer) was added, and the mixture was incubated at room temperature for 15 minutes, and then stored at -20°C in the freezer until use. The luminometer used was a ARVO™ HTS 1420 multi-labeling counter (Wallac). The protocol for measurement was as follows: 50 µl of Luciferase Assay Reagent II was added to each well, stirred for 0.3 seconds and measured for one second. 50 ml of Stop&Glo™ Reagent was then added, stirred for 0.3 seconds, and measured for one second. The measured values for firefly luciferase activity were corrected by dividing by the value for Renilla luciferase activity, the internal control. Average values and standard errors were calculated from triplicate samples.

This clarified that although ERRγ3 by itself does not comprise the transcription-controlling activity as predicted from that DNA deletion, it does however, comprise the function of markedly enhancing (53-68%) the transcriptional activation function of ERRγ2 when coexisted with ERRγ2. This enhancing function was reduced in the presence of 4-hydroxytamoxifen at high concentrations (10⁻⁶ M or higher).

### [Example 9] The transcription-controlling function of ERRγ3 on ERα, ERβ, and TR

To confirm whether or not ERRγ3 can regulate the transcription-controlling function of nuclear receptors other than ERRγ2, ERRγ3's function of regulating the transcription-controlling activity of nuclear receptors related to ERRγ (ERα, ERβ, and TR) was evaluated using a reporter gene assay. The particulars of the reporter gene assay protocol are the same as described in Example 8, except for the use of expression plasmids for ERα, ERβ, and TR. In addition to 4-hydroxytamoxifen, the cells were also contacted with 10⁻⁵ M of (R)-estradiol in experiments related to ERα and ERβ, and 10⁻⁵ M of thyroxine in the experiment related to TR.

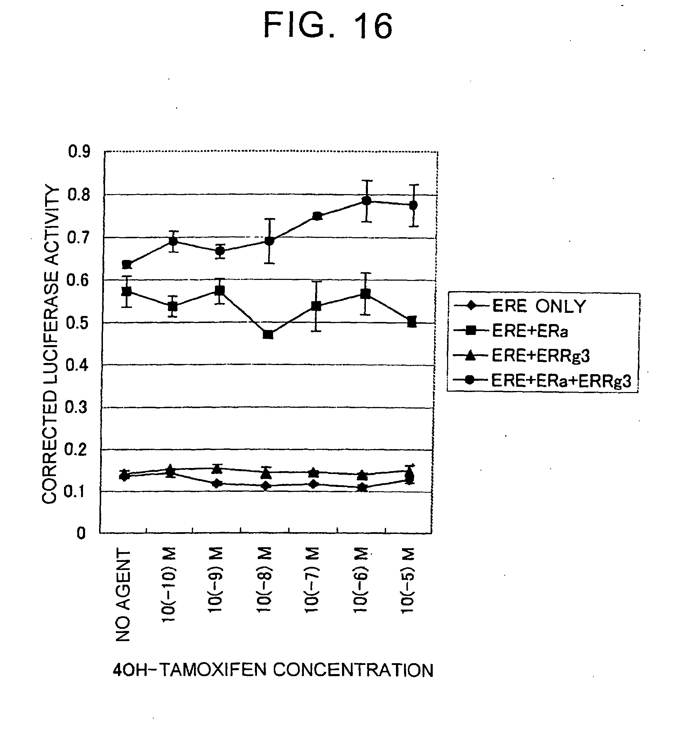

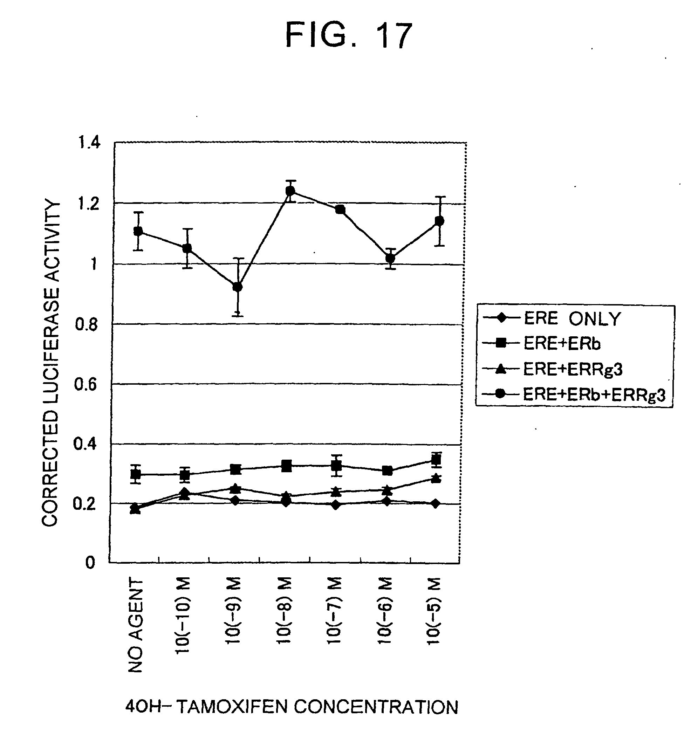

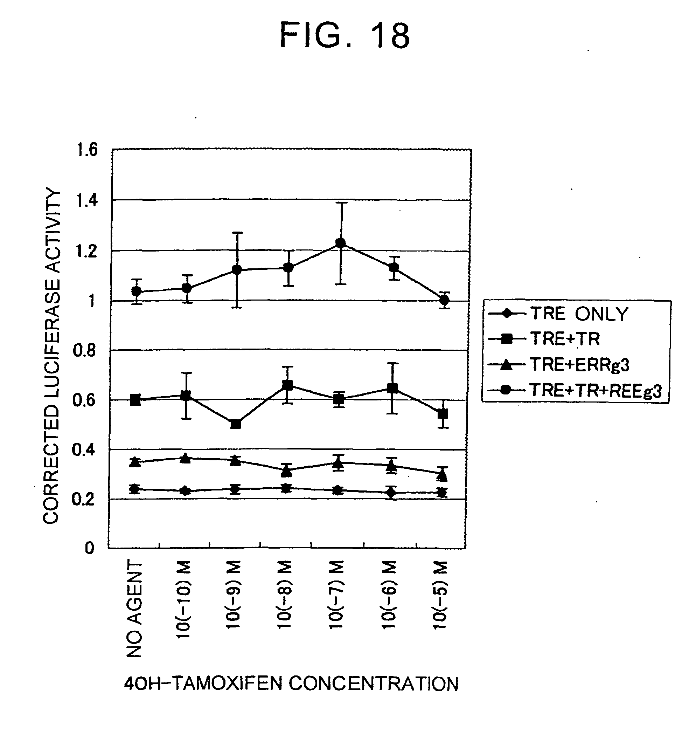

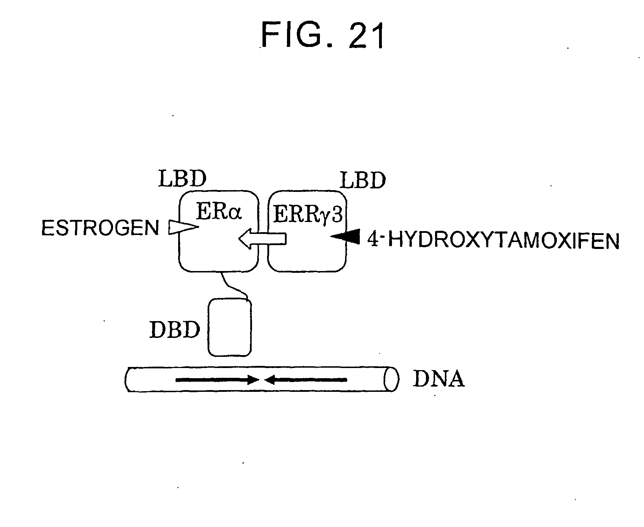

This clarified the fact that ERRγ3 comprises the function of significantly enhancing the transcription-controlling activity of ERα, ERβ, and TR in the presence of ligands for the respective receptors. Function enhancement was calculated as 13-74% for ERα (Fig. 16) , 600-650% for ERβ (Fig. 17) , and 128-229% for TR (Fig. 18). In addition, although 4-hydroxytamoxifen had no function-enhancing effect on ERβ and TR, the function of ERRγ3 on ERα increased dependent on the concentration of 4-hydroxytamoxifen, in the presence of the ligand. Thus, it was confirmed that ERRγ2 is capable of forming a complex with other nuclear receptor having the DBL such as ERα, as shown in Fig. 21, and enhancing the transcriptional activation function of the nuclear receptor.

### [Example 10] Comparison of ERRγ3 and ERRγ2 on the transcription-controlling function for ERα

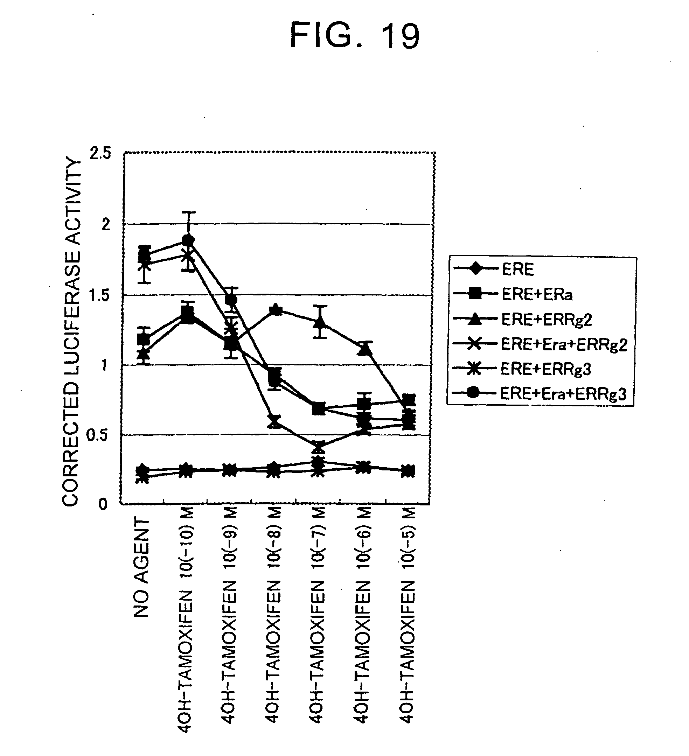

The effect of the ERα ligand (R)-estradiol on ERRγ3's function of enhancing the transcriptional activation function of ERα, and whether or not ERRγ2 comprises a similar transcription-controlling function to ERRγ3, were evaluated using the reporter gene assay (Figs. 19-20). The details of the reporter gene assay protocol are the same as described in Example 8. The transfected cells were contacted with varying concentrations of 4-hydroxytamoxifen in the absence of (R)-estradiol or presence of 10⁻⁷ M of β-estradiol.

The result indicated that in the absence of 4-hydroxytamoxifen, ERRγ3 comprises the function of enhancing the transcriptional activation function of ERα. This effect was calculated to be 64% in the absence of β-estradiol, and 18% in the presence of 10⁻⁷ M of β-estradiol. The effect of 4-hydroxytamoxifen on the enhancing function of ERRγ3 was shown to vary depending on the concentration of β-estradiol. Over the concentrations of β-estradiol tested in this experiment, the transcription enhancing function of ERRγ3 was reduced in the presence of 4-hydroxytamoxifen at high concentrations. In addition, ERRγ2 also showed the function of enhancing transcription, similar to the effect of ERRγ3, when coexisted with ERα. This observation was first demonstrated in the present Example.

### [Example 11] The effect of ERRγ3 on the transcriptional stimulation activity of RARα

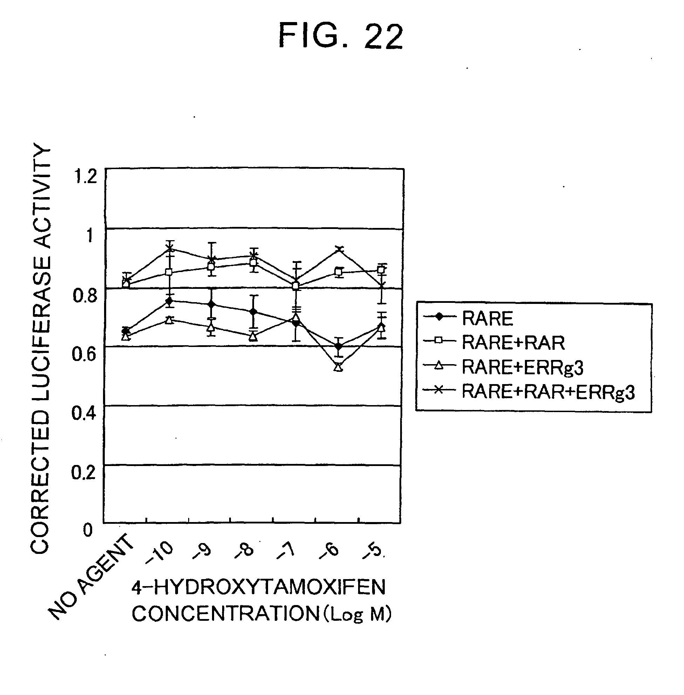

The reporter gene assay was used to evaluate the effect of ligand 4-hydroxytamoxifen and 5x 10⁻⁸ M of retinoic acid on the function of ERRγ3 in enhancing the transcriptional activation function of retinoic acid receptor (RAR) (Fig. 22). RAR is a nuclear receptor belonging to group B of subfamily 1.

CV-1 cells (a cell line derived from the kidney of African green monkey) were used as hosts in the reporter gene assay. The luciferase reporter plasmid pGVP2-RARE and the expression plasmids for ERRγ3 and RARα: pcDNA3.1-ERRγ3 and pcDNA3.1-RARα, respectively, or alternatively the expression plasmid for both nuclear receptors, were transfected into the host cells. The cells were then exposed to different concentrations of 4-hydroxytamoxifen (0, 10⁻¹⁰ to 10⁻⁵ M) and 5x 10⁻⁸ M of retinoic acid for 24 hours. Luciferase activity in the cells was then examined.

Transfection was performed as follows: CV-1 cells were resuspended in DMEM supplemented with 10% fetal bovine serum, 2000 U/l of penicillin G, and 100 mg/l of streptomycin to a concentration of 2x 10⁵ cells/2.5 ml. This was then seeded to a 6-well plate at 2.5 ml per well, and incubated for 18-24 hours in a CO₂ incubator at 37°C and 5% CO₂. 1 µg of each of the expression plasmids for the transcription factors, the luciferase reporter plasmid, and another luciferase reporter plasmid used for normalization of the measured values (pRL-TK), were dissolved in Opti-MEM (Gibco BRL) , to a total of 150 µl. 7 µl of the transfection reagent LipofectAMINE 2000 (Gibco BRL) was diluted in 143 µl of Opti-MEM, and this was added to the mixture drop by drop while stirring. The mixture was incubated for 20 minutes. The LipofectAMINE-DNA mixture was diluted in 1.5 ml of Opti-MEM, and added to cells that had been washed twice with 1 ml of PBS(-). The cells were cultured for four hours. After removing the transfection media, 200 µl of trypsin/EDTA was added to the cells, which were then incubated at 37°C for five minutes. Then, 1 ml of DMEM was added to suspend the cells, and this was transferred to a centrifuge tube, and centrifuged for three minutes at 1000 rpm. The cells were then resuspended in DMEM to a concentration of 1.6x 10⁴ cells/100 µl, and seeded to a 96-well plate at 100 µl per well. When evaluating agents, a two-fold concentrated solution was prepared by pre-diluting a 1000 times concentrated agent DMSO solution with culture medium. 50 µl of the solution was mixed with 50 µl of a cell suspension at 1. 6x 10⁴ cells per 50 µl. The cells were then incubated for 24 hours in a CO₂ incubator at 37°C and 5% CO₂. Luciferase activity was measured using the Dual-Luciferase™ Reporter Assay System (Promega). The culture supernatant was removed from each well of the 96-well plate and washed once with 100 µl of PBS (-). 20 µl of 1x PLB (Passive Lysis Buffer) was added, and the mixture was incubated at room temperature for 15 minutes, and then stored at -20°C in the freezer until use. The luminometer used was an ARVO™ HTS 1420 multi-labeling counter (Wallac). The protocol for measurement was as follows: 50 µl of Luciferase Assay Reagent II was added to each well, stirred for 0.3 seconds, and measured for one second. 50 µl of Stop&Glo™ Reagent was then added, stirred for 0.3 seconds, and measured for one second. The measured values for firefly luciferase activity were corrected by dividing by the value for the *Renilla* luciferase activity, the internal control. Average values and standard errors were calculated from triplicate samples.

In the presence of 5x 10⁻⁸ M retinoic acid, RAR stimulated transcription controlled by RARE, however, ERRγ3 did not show a significant modifying effect on RAR's transcriptional stimulation activity (Fig. 22).

### [Example 12] The effect of ERRγ3 on VDR's transcriptional stimulation activity

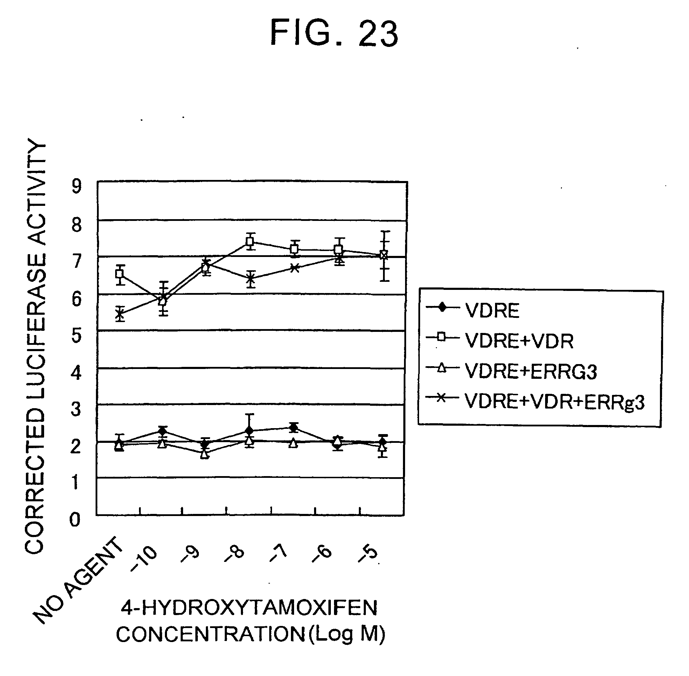

The reporter gene assay was used to evaluate the effect of calcitriol, a vitamin D receptor (VDR) ligand, on ERRγ3's function in enhancing VDR transcriptional activation function (Fig. 23). VDR is a nuclear receptor belonging to group I of subfamily 1.

CV-1 cells (a cell line derived from the kidney of African green monkeys) were used as hosts in the reporter gene assay. The host cells were transferred with the luciferase reporter plasmid pGVP2-VDRE, and expression plasmids for ERRγ3 and VDR: pcDNA3.1-ERRγ3 and pcDNA3.1-rVDR, respectively, or alternatively the expression plasmid for both nuclear receptors. Then, the cells were exposed to different concentrations of 4-hydroxytamoxifen (0, 10⁻¹⁰ to 10⁻⁵ M) and 5x 10⁻⁸ M of calcitriol for 24 hours. Luciferase activity in the cells was then examined. The detailed experimental protocol is as described in Example 11.

In the presence of 5x 10⁻⁸ M calcitriol, VDR stimulated transcription controlled by VDRE, however, ERRγ3 did not show a significant modifying effect on the transcriptional stimulation activity of RAR (Fig. 23).

### [Example 13] The effect of ERRγ3 on the transcriptional stimulation activity of PPARα, β, and γ

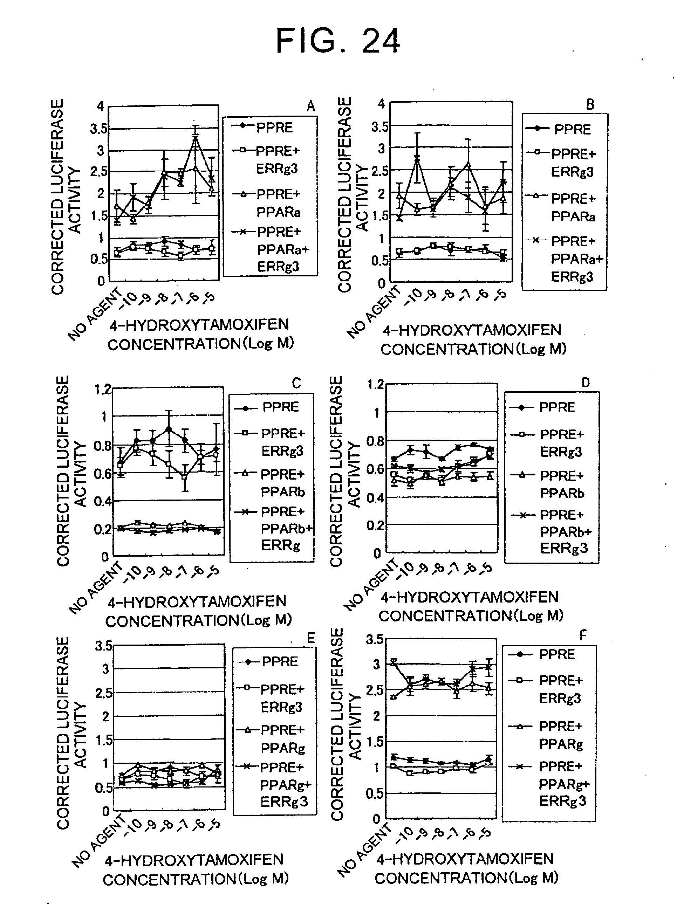

The effect of ligands on the function of ERRγ3 in enhancing the transcriptional activation function of peroxisome proliferator activated receptor (PPAR) α, β, or γ was evaluated using the reporter gene assay (Fig. 24). The ligands tested were 4-hydroxytamoxifen and either bezafibrate (for PPARα), carbacyclin, or rosiglitazone. PPARα, β, and γ are nuclear receptors belong to the group C of the subfamily 1.

CV-1 cells (a cell line derived from the kidney of African green monkeys) were used as hosts in the reporter gene assay. Host cells were transfected with the luciferase reporter plasmid pGVP2-PPRE1 and the expression plasmids for ERRγ3 and PPARα, β, or γ: pcDNA3.1-ERRγ3 and pcDNAI-hPPARα, pCDM8-hPPARβ2, or pCDM8-hPPARγ1-3, respectively, or alternatively with the expression plasmid for both ERRγ3 and PPAR. Then, the cells were exposed to different concentrations of 4-hydroxytamoxifen (0, 10⁻¹⁰ to 10⁻⁵ M) and 10⁻⁵ M of bezafibrate (in case of PPARα), 10⁻⁵ M of carbacyclin, or 10⁻⁶ M of rosiglitazone for 24 hours, and the luciferase activity of the cells was examined. The detailed experimental protocol is as for Example 11.

PPARα, β, and γ stimulated transcription controlled by PPRE in the presence of 10⁻⁵ M bezafibrate, 10⁻⁵ M carbacyclin, and 10⁻⁶ M rosiglitazone respectively (Fig. 24A to F). However, ERRγ3 did not show any significant modifying effect on PPAR transcriptional .stimulation activity (Fig. 24B, D, and F).

### [Example 14] The function of ERRγ3 in suppressing GR transcriptional stimulation activity

The reporter gene assay was used to evaluate the effect of ligands 4-hydroxytamoxifen and dexamethasone on the function of ERRγ3 in enhancing the transcriptional activation function of glucocorticoid receptor (GR (Fig. 25).

CV-1 cells (a cell line derived from the kidney of African green monkeys) were used as hosts in the reporter gene assay. The luciferase reporter plasmid pGRE-luc and the expression plasmids for ERRγ3 and GR: pcDNA3.1-ERRγ3 and pcDNA3.1-GR, respectively, or alternatively the expression plasmid for both nuclear receptors were transfected into host cells. The cells were then exposed to different concentrations of 4-hydroxytamoxifen (0, 10⁻¹⁰ to 10⁻⁵ M) and 10⁻⁵ M of dexamethasone for 24 hours, and their luciferase activity was then examined. The detailed experimental protocol was as described in Example 11.

While GR stimulated transcription controlled by GRE in the presence of dexamethasone at 10⁻⁵ M (Fig. 25A and B) , ERRγ3 inhibited GR's transcriptional stimulation activity. Furthermore, the presence of 10⁻⁵ M 4-hydroxytamoxifen reduced ERRγ3's inhibitory function (Fig. 25 B) .

### [Example 15] Expression of ERRγ3 in mice

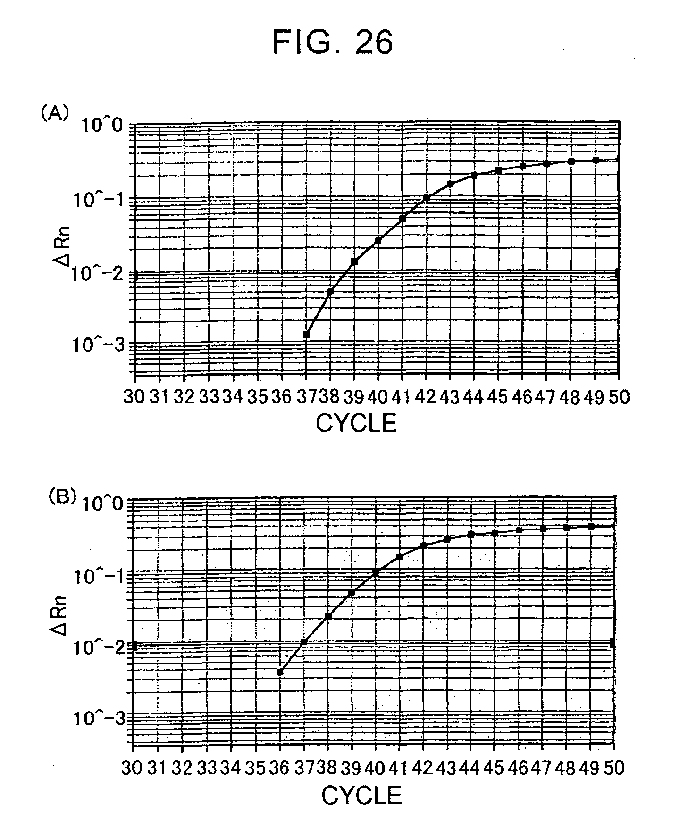

The tissue distribution of ERRγ3 expression in mice was examined by TaqMan PCR (PE Applied Biosystems) using a commercially available cDNA derived from mouse tissues as a template. The nucleotide sequence of mouse ERRγ3 was predicted based on the sequence of mouse ERRγ2. Based on the mouse ERRγ3 nucleotide sequence, ERRγ3-selective PCR primers (AAGCCTGCAAGGCATTCTTC/ SEQ ID NO: 21; CGACCTCCACGCACTCTG/SEQ ID NO: 22) and a probe (FAM-AGGACGATTCAAGGGGTCCGTCTTGA-TAMRA/SEQ ID NO: 23) were designed for TaqMan PCR. The probe was designed to encompass splicing sites at both ends of ERRγ2's exon F (the exon missing in ERRγ3). The probe is therefore considered to specifically detect ERRγ3, without hybridizing to ERRγ2. A total volume of 50 µl of PCR reaction composition composes: commercial cDNA derived from mouse tissues (CLONTECH; embryo and lung; 40 pg and 10 pg, respectively) as a template, 1x TaqMan EZ buffer, 300 µM dATP, 300 µM dGTP, 300 µM dCTP, 600 µM dUTP, 200 nM forward primer, 200 nM reverse primer, 100 nM probe, 5 U of rTth DNA polymerase, 0.5 U of AmpErase UNG, and 4 mM Mn(OAc)₂. PCR amplification was performed by initial incubation at 50°C for 2 minutes, 60°C for 30 minutes, and 95°C for 5 minutes, followed by a cycle of 95°C for 20 seconds, and 65°C for 1 minute. ERRγ3 expression was detected in both embryo (Fig. 26 A), and lung (Fig. 26 B).

### Industrial Applicability

The present invention provided a novel nuclear receptor ERRγ3. ERRγ3 is a protein comprising the function of enhancing the transcriptional activation function of arbitrary nuclear receptors. Furthermore, the present invention revealed that the known proteins ERRγ1 and ERRγ2, as well as ERRγ3 protein, comprise the function of enhancing the transcriptional activation function of other nuclear receptors.

The function of these proteins in enhancing the transcriptional activation function of other nuclear receptors is a novel finding, discovered in the present invention. Regulating the transcription-controlling activity of nuclear receptors should open the way to controlling cellular activities by signal transduction. This kind of idea has been the basis for the functional analyses of a variety of nuclear receptors in the past. Attempts to regulate cellular activities by regulating the functions revealed in this way are continuing. However, many of these attempts are geared towards targeting specific nuclear receptors. However, the research of the present inventors has shown that the activity of nuclear receptors is controlled not only by a single nuclear receptor, but by interaction with other nuclear receptors. In other words, the study revealed that regulation of signal transduction pathways by targeting nuclear receptors requires consideration of not only the function of a specific nuclear receptor, but also of its interactions with other nuclear receptors that regulate the transcription-controlling activity of the nuclear receptor.

The present invention has provided a method for evaluating the function of ERRγ3, ERRγ1 or ERRγ2 in enhancing the transcriptional activation function of other nuclear receptors. Furthermore, the present invention has provided a method of evaluating the effect of test substances on the above enhancing function. Based on these evaluation methods, test substances can be evaluated for their activity in controlling the function of ERRγ3, ERRγ1 or ERRγ2 in enhancing the transcriptional activation function of other nuclear receptors. Accordingly, based on the present invention, compounds capable of controlling the above function of enhancing transcriptional activation function can be screened. Compounds identified by a screening method of the present invention are compounds capable of controlling the function of ERRγ3 of the present invention, or that of ERRγ1 or ERRγ2, in enhancing the transcriptional activation function of other nuclear receptors. Such compounds may be useful in controlling cellular functions as agents directed at novel targets.

In addition, ERRγ3, ERRγ1 or ERRγ2 themselves, or compounds capable of controlling the activity or expression of these proteins, may be useful as therapeutic agents for diseases caused by abnormalities in the transcription-controlling activities of nuclear receptors.

The ERRγ3, ERRγ1, or ERRγ2 protein may be useful for the treatment of diseases characterized by abnormal nuclear receptors. More specifically, by controlling the expression level or activity of ERRγ1 or ERRγ2, therapeutic effects can be achieved for breast cancer, cervix cancer, osteoporosis, hyperlipidemia, arteriosclerosis, angina, myocardial infarction, stroke, systemic lupus erythematosus, or Alzheimer's disease (these diseases related to ER), or attention deficit hyperactivity disorder (ADHD).

Moreover, the present invention provides antisenses of the polynucleotides encoding ERRγ3, ERRγ1 or ERRγ2, as well as double-stranded RNAs, or proteins conferring dominant negative effect on these proteins. Such antisenses or proteins conferring dominant negative effect may be useful for the treatment of diseases characterized by the abnormalities in the transcription-controlling activity of nuclear receptors. More specifically, such polynucleotides or proteins encoded by the polynucleotides can achieve therapeutic effects by regulating the transcription-controlling activity of disease-causing nuclear receptors, such as ER.

## Claims

1. A polynucleotide of any one of the following (A) to (E):
(A) a polynucleotide comprising a coding region of the nucleotide sequence of SEQ ID NO: 1;
(B) a polynucleotide encoding a protein comprising the amino acid sequence of SEQ ID NO: 2;
(C) a polynucleotide comprising an amino acid sequence wherein one or more amino acids are replaced, deleted, inserted, and/or added to the amino acid sequence of SEQ ID NO: 2, encoding a protein that:
(a) controls the transcriptional activation function of a nuclear receptor when co-existed with the nuclear receptor; and
(b) lacks at least part of a DNA binding domain;
(D) a polynucleotide that hybridizes under stringent conditions with a polynucleotide comprising the nucleotide sequence of SEQ ID NO: 1, encoding a protein that:
(a) controls the transcriptional activation function of a nuclear receptor when co-existed with the nuclear receptor; and
(b) lacks at least part of a DNA binding domain;
(E) a polynucleotide comprising a nucleotide sequence in which a sequence corresponding to nucleotides 599 to 715 of SEQ ID NO: 5 is deleted or replaced with another nucleotide sequence, and comprising a nucleotide sequence that comprises homology of 70% or more to the above nucleotide sequence in which the sequence corresponding to nucleotides 599 to 715 is deleted.

2. The polynucleotide of claim 1, wherein the nuclear receptor in (a) belongs to a member selected from the group consisting of group A of subfamily 3, group B of subfamily 3, group C of subfamily 3, and group C of subfamily 1.

3. The polynucleotide of claim 2, wherein the nuclear receptor in (a) is a nuclear receptor selected from the group consisting of estrogen receptor related receptor, estrogen receptor, and thyroid hormone receptor, wherein the polynucleotide encodes a protein that enhances the transcriptional activation function of the nuclear receptor.

4. The polynucleotide of claim 2 that encodes a protein that reduces the transcriptional activation effect of a nuclear receptor, wherein the nuclear receptor is a glucocorticoid receptor.

5. A protein encoded by the polynucleotide of claim 1.

6. A vector comprising the polynucleotide of claim 1.

7. A transformant carrying the polynucleotide of claim 1 or the vector of claim 5.

8. A method for producing the protein of claim 5, comprising a step of culturing the transformant of claim 7, and recovering an expression product.

9. A method of detecting the activity of a test substance in regulating the transcription-controlling activity of a nuclear receptor complex, wherein the complex comprises an arbitrary nuclear receptor and a protein encoded by the polynucleotide of any one of the following (A) to (E):
(A) a polynucleotide comprising the coding region of the nucleotide sequence of any of SEQ ID NO: 1, 3, or 5;
(B) a polynucleotide encoding a protein comprising the amino acid sequence of any of SEQ ID NO: 2, 4, or 6;
(C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
(D) a polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
(E) a polynucleotide comprising homology of 80% or more with the nucleotide sequence of any of SEQ ID NO: 1, 3, or 5, encoding a protein that is functionally equivalent to a protein comprising the amino acid sequence of any of SEQ ID NO: 2, 4, or 6;
wherein the method comprises the following steps:
(1) contacting the test substance with a cell expressing the arbitrary nuclear receptor and the protein encoded by the polynucleotide of any one of (A) to (E) , where the cell carries an expression cassette in which a reporter gene is inserted downstream of a response element for the nuclear receptor;
(2) culturing the above cell under conditions that allow expression of the nuclear receptor and the protein encoded by the polynucleotide of any one of (A) to (E), and measuring the expression level of the reporter gene in the cell; and
(3) detecting the activity of the test substance in controlling the transcription-controlling activity of the above nuclear receptor, using the measurement result of step (2) as an index.

10. The method of claim 9, wherein the nuclear receptor belongs to a member selected from the group consisting of group A of subfamily 3, group B of subfamily 3, group C of subfamily 3, and group C of subfamily 1.

11. The method of claim 10, wherein the nuclear receptor is a nuclear receptor selected from the group consisting of estrogen receptor related receptor, estrogen receptor, and thyroid hormone receptor.

12. The method of claim 10, wherein the nuclear receptor is a glucocorticoid receptor.

13. A method of evaluating a substance having an activity of regulating the transcription-controlling activity of a nuclear receptor complex, comprising the followings steps:
(1) detecting the activity of a test substance in regulating the transcription-controlling activity of the nuclear receptor complex using the method of claim 9; and
(2) selecting a test substance capable of suppressing or enhancing the transcriptional activation function of the nuclear receptor complex, by comparison with a control.

14. A method for detecting the activity of a test substance in binding to a nuclear receptor, wherein the nuclear receptor is a protein that is encoded by the polynucleotide of any one of the following (A) to (E) :
(A) a polynucleotide comprising a coding region of the nucleotide sequence of SEQ ID NO: 1;
(B) a polynucleotide encoding a protein comprising the amino acid sequence of SEQ ID NO: 2;
(C) a polynucleotide comprising an amino acid sequence wherein one or more amino acids are replaced, deleted, inserted, and/or added to the amino acid sequence of SEQ ID NO: 2, encoding a protein that:
(a) controls the transcriptional activation function of a nuclear receptor when co-existed with the nuclear receptor; and
(b) lacks at least part of a DNA binding domain;
(D) a polynucleotide that hybridizes under stringent conditions with a polynucleotide comprising the nucleotide sequence of SEQ ID NO: 1, encoding a protein that:
(a) controls the transcriptional activation function of a nuclear receptor when co-existed with the nuclear receptor; and
(b) lacks at least part of a DNA binding domain;
(E) a polynucleotide comprising a nucleotide sequence in which a sequence corresponding to nucleotides 599 to 715 of SEQ ID NO: 5 is deleted or replaced with another nucleotide sequence, and comprising a nucleotide sequence that comprises homology of 70% or more to the above nucleotide sequence in which the sequence corresponding to nucleotides 599 to 715 is deleted;
wherein the method comprises the following steps:
(1) contacting the nuclear receptor, its ligand, and the test substance in any of the following orders i) to iii):
i) contacting the nuclear receptor and the test substance first, and then contacting them with the ligand;
ii) contacting the nuclear receptor and the ligand in the presence of the test substance; or
iii) contacting the nuclear receptor and the ligand first, and then contacting them with the test substance;
(2) measuring the amount of the ligand or the test substance bound to the nuclear receptor; and
(3) detecting the activity of binding to the nuclear receptor, using the measurement result according step (2) as an index.

15. The method of claim 14, wherein the ligand is a compound selected from the group consisting of ligands, agonists, and antagonists of estrogen receptor-related receptors.

16. The method of claim 14, wherein the nuclear receptor coexists with another arbitrary second nuclear receptor.

17. The method of claim 16, wherein the second nuclear receptor belongs to a member selected from the group consisting of group A of subfamily 3, group B of subfamily 3, group C of subfamily 3, and group C of subfamily 1.

18. The method of claim 17, wherein the second nuclear receptor is a nuclear receptor selected from the group consisting of estrogen receptor-related receptors, estrogen receptors, and thyroid hormone receptors.

19. The method of claim 17, wherein the nuclear receptor is a glucocorticoid receptor.

20. A method of evaluating a substance capable of binding to a nuclear receptor, comprising the following steps:
(1) detecting the activity of a test substance in binding to a nuclear receptor, using the method of claim 14; and
(2) selecting a test substance capable of binding to the nuclear receptor.

21. An agent for controlling the activity of a nuclear receptor, comprising as an effective ingredient a substance selected by the method of claim 13 or 20.

22. An agent for controlling the activity of a nuclear receptor, comprising as an effective ingredient a polynucleotide of any one of the following (A) to (E), or a protein encoded by the polynucleotide:
(A) a polynucleotide comprising a coding region of the nucleotide sequence of any one of SEQ ID NO: 1, 3, or 5;
(B) a polynucleotide encoding the amino acid sequence of any one of SEQ ID NO: 2, 4, or 6;
(C) a polynucleotide comprising an amino acid sequence wherein one or more amino acids are replaced, deleted, inserted, and/or added to the amino acid sequence of any one of SEQ ID NO: 2, 4, or 6, encoding a protein functionally equivalent to a protein comprising the amino acid sequence of any one of SEQ ID NO: 2, 4, or 6;
(D) a polynucleotide that hybridizes under stringent conditions with a polynucleotide comprising a nucleotide sequence of any one of SEQ ID NO: 1, 3, or 5, encoding a protein functionally equivalent to a protein comprising the amino acid sequence of any one of SEQ ID NO: 2, 4, or 6;
(E) a polynucleotide comprising a sequence with homology of 80% or more to the nucleotide sequence of any one of SEQ ID NO: 1, 3, or 5, encoding a protein functionally equivalent to a protein comprising the amino acid sequence of any one of SEQ ID NO: 2, 4, or 6.

23. An agent for controlling the activity of a nuclear receptor, comprising as an effective ingredient any of the following (1) to (4) :
(1) An antisense polynucleotide of the polynucleotide of the above (A) to (E);
(2) An antibody capable of recognizing a protein encoded by the polynucleotide of the above (A) to (E);
(3) A protein conferring a dominant negative effect on a protein encoded by a polynucleotide of the above (A) to (E); and
(4) A double-stranded RNA of 21 to 23 base pairs, comprising a sense RNA corresponding to a partial sequence of a polynucleotide of the above (A) to (E), and its antisense RNA.

24. A model animal in which the activity of a nuclear receptor is controlled, where the animal is a transgenic non-human animal in which the expression of a protein of any one of the following (A) to (D) is controlled:
(A) A protein comprising the amino acid sequence of any of SEQ ID NO: 2, 4, or 6;
(B) A protein encoded by a DNA that hybridizes under stringent conditions with a DNA comprising the nucleotide sequence of any of SEQ ID NO: 1, 3, or 5, and which is functionally equivalent to a protein comprising the amino acid sequence of any one of SEQ ID NO: 2, 4, or 6;
(C) A protein comprising an amino acid sequence wherein one or more amino acids are replaced, deleted, inserted, and/or added to the amino acid sequence of any one of SEQ ID NO: 2, 4, or 6, and which is functionally equivalent to a protein comprising the amino acid sequence of any one of SEQ ID NO: 2, 4, or 6; and
(D) A protein comprising an amino acid comprises a sequence with homology of 80% or more to the amino acid sequence of any one of SEQ ID NO: 2, 4, or 6, and which is functionally equivalent to a protein comprising the amino acid sequence of any one of SEQ ID NO: 2, 4, or 6.

25. A method for diagnosing a disease that is caused by an abnormal activity of a nuclear receptor, comprising the steps of measuring the expression level of a polynucleotide in a biological sample collected from a subject, and determining that the subject is developing a disease caused by abnormal nuclear receptor activity when the expression level of that polynucleotide is elevated or decreased compared with a healthy subject, wherein the polynucleotide is any of the following (A) to (E) :
(A) A polynucleotide comprising a coding region of the nucleotide sequence of any of SEQ ID NO: 1, 3, or 5;
(B) A polynucleotide encoding a protein comprising the amino acid sequence of any of SEQ ID NO: 2, 4, or 6;
(C) A polynucleotide encoding a protein comprising an amino acid sequence wherein one or more amino acids are replaced, deleted, inserted, and/or added to the amino acid sequence of any of SEQ ID NO: 2, 4, or 6, encoding a protein functionally equivalent to a protein comprising the amino acid sequence of any of SEQ ID NO: 2, 4, or 6;
(D) A polynucleotide that hybridizes under stringent conditions with a polynucleotide comprising the nucleotide sequence of any of SEQ ID NO: 1, 3, or 5, encoding a protein functionally equivalent to a protein comprising the amino acid sequence of any of SEQ ID NO: 2, 4, or 6; and
(E) a polynucleotide comprising a sequence with homology of 80% or more to a nucleotide sequence of any of SEQ ID NO: 1, 3, or 5, encoding a protein functionally equivalent to a protein comprising the amino acid sequence of any of SEQ ID NO: 2, 4, or 6.


SEQUENCE LISTING

<110> FUJISAWA PHARMACEUTICAL CO., LTD.

<120> NUCLEAR RECEPTOR ERRγ3

<130> F3-A0202P

<140>
<141>

<150> JP 2002-084560
<151> 2002-03-25

<160> 23

<170> PatentIn Ver. 2.1

<210> 1
<211> 3362
<212> DNA
<213> Homo sapiens

<220>
<221> CDS
<222> (684)..(1874)

<400> 1
aggtcccact ctgtgcttcc atggagaagg gcggccaatg tgattctggg caaagtgtgg 60
tgacttgtgg attttcactg tgacgatgtt gctacacggt ccttcacttg gagttagtgc 120
aacaaggaga catctgagct taaaatttat gaaacatcta aagaaatcaa gctttatata 180
ggatcaccgt tgtgggttga attgtgtatc cccaaaagat gttgaagtcc tgaccacctc 240
cctatacctt acaatatagc cttttgcgga agtagggtct ttgcagaaga tcaagttaag 300
atgaaatcat ttgattgggc cctagttcaa tatatcttat gtccttataa aaggagacaa 360
tttggacaca gggacaaggg gagaatgcca cataaagatt ggaattatgc tgccataagc 420
caaggaactc caaagatcgc tggcaagctg ctaaaagcta gcacagattc atggaacaga 480
gtctccgtca tagcctacca aaggaatcaa ccctgctgac atcttgattt cagacttcta 540
gcctccagaa ctctttaaaa agttgaagtc tattggaaac cttcttataa gaaaccttcc 600
tagtcagaga attcaacttt cttcttttct tcattcatga aaatgcagat ccactgagaa 660
agggaataag gcttctctgc aga atg tca aac aaa gat cga cac att gat tcc 713
agc tgt tcg tcc ttc atc aag acg gaa cct tcc agc cca gcc tcc ctg 761
acg gac agc gtc aac cac cac agc cct ggt ggc tct tca gac gcc agt 809
ggg agc tac agt tca acc atg aat ggc cat cag aac gga ctt gac tcg 857
cca cct ctc tac cct tct gct cct atc ctg gga ggt agt ggg cct gtc 905
agg aaa ctg tat gat gac tgc tcc agc acc att gtt gaa gat ccc cag 953
acc aag tgt gaa tac atg ctc aac tcg atg ccc aag aga ctg tgt tta 1001
gtg tgt ggt gac atc gct tct ggg tac cac tat ggg gta gca tca tgt 1049
gaa gcc tgc aag gca ttc ttc aag agg aca att caa ggg gtg cgt ctt 1097
gac aga gta cgt gga ggt cgg cag aag tac aag cgc agg ata gat gcg 1145
gag aac agc cca tac ctg aac cct cag ctg gct cag cca gcc aaa aag 1193
cca tat aac aag att gtc tca cat ttg ttg gtg gct gaa ccg gag aag 1241
atc tat gcc atg cct gac cct act gtc ccc gac agt gac atc aaa gcc 1289
ctc act aca ctg tgt gac ttg gcc gac cga gag ttg gtg gtt atc att 1337
gga tgg gcg aag cat att cca ggc ttc tcc acg ctg tcc ctg gcg gac 1385
cag atg agc ctt ctg cag agt gct tgg atg gaa att ttg atc ctt ggt 1433
gtc gta tac cgg tct ctt tca ttt gag gat gaa ctt gtc tat gca gac 1481
gat tat ata atg gac gaa gac cag tcc aaa tta gca ggc ctt ctt gat 1529
cta aat aat gct atc ctg cag ctg gta aag aaa tac aag agc atg aag 1577
ctg gaa aaa gaa gaa ttt gtc acc ctc aaa gct ata gct ctt gct aat 1625
tca gac tcc atg cac ata gaa gat gtt gaa gcc gtt cag aag ctt cag 1673
gat gtc tta cat gaa gcg ctg cag gat tat gaa gct ggc cag cac atg 1721
gaa gac cct cgt cga gct ggc aag atg ctg atg aca ctg cca ctc ctg 1769
agg cag acc tct acc aag gcc gtg cag cat ttc tac aac atc aaa cta 1817
gaa ggc aaa gtc cca atg cac aaa ctt ttt ttg gaa atg ttg gag gcc 1865
aag gtc tga ctaaaagctc cctgggcctt cccatccttc atgttgaaaa 1914
agggaaaata aacccaagag tgatgtcgaa gaaacttaga gtttagttaa caacatcaaa 1974
aatcaacaga ctgcactgat aatttagcag caagactatg aagcagcttt cagattcctc 2034
cataggttcc tgatgagttt ctttctactt tctccatcat cttctttcct ctttcttccc 2094
acatttctct ttctctttat tttttctcct tttcttcttt cacctccctt atttctttgc 2154
ttctttcatt cctagttccc attctccttt attttcttcc cgtctgcctg ccttctttct 2214
tttctttacc tactctcatt cctctctttt ctcatccttc cccttttttc taaatttgaa 2274
atagctttag tttaaaaaaa aatcctccct tccccctttc ctttcccttt ctttcctttt 2334
tccctttcct tttccctttc ctttcctttc ctcttgacct tctttccatc tttctttttc 2394
ttccttctgc tgctgaactt ttaaaagagg tctctaactg aagagagatg gaagccagcc 2454
ctgccaaagg atggagatcc ataatatgga tgccagtgaa cttattgtga accatactgt 2514
ccccaatgac taaggaatca aagagagaga accaacgttc ctaaaagtac agtgcaacat 2574
atacaaattg actgagtgca gtattagatt tcatgggagc agcctctaat tagacaactt 2634
aagcaacgtt gcatcggctg cttcttatca ttgcttttcc atctagatca gttacagcca 2694
tttgattcct taattgtttt ttcaagtctt ccaggtattt gttagtttag ctactatgta 2754
actttttcag ggaatagttt aagctttatt cattcatgca atactaaaga gaaataagaa 2814
tactgcaatt ttgtgctggc tttgaacaat tacgaacaat aatgaaggac aaatgaatcc 2874
ttaaggaaga tttttaaaaa tgttttgttt cttcttacaa atggagattt ttttgtacca 2934
gctttaccac ttttcagcca tttattaata tgggaattta acttactcaa gcaatagttg 2994
aagggaaggt gcatattatc acggatgcaa tttatgttgt gtgccagtct ggtcccaaac 3054
atcaatttct taacatgagc tccagtttac ctaaatgttc attgacacaa aggatgagat 3114
tacacctaca gtgactctga gtagtcacat atataagcac tgcacatgag atatagatcc 3174
gtagaattgt caggagtgca cctctctact tgggaggtac aattgccata tgatttctag 3234
ctgccatggt ggttaggaat gtgatactgc ctgtttgcaa agtcacagac cttgcctcag 3294
aaggagctgt gagccagtat tcatttaaga ggcaataagg caaatgccag aattaaaaaa 3354
aaaaaaag 3362

<210> 2

<211> 396

<212> PRT

<213> Homo sapiens

<400> 2

<210> 3
<211> 5221
<212> DNA
<213> Homo sapiens

<220>

<221> CDS

<222> (219)..(1529)

<400> 3

cggccgcccg ggcaggtttt gtagactttc atagccaaag aaaccggctt cggcttcttt 60

aaaatccccg acgactcacc tgattaacct gctgcagttc tgaccctgcc aagagctgac 120

aatttactgg ttcatcaatg aaacaatatt aaattatgaa gatgtaagga aaaaatccta 180

cgctaacact gtcgcagttt gaaaggcttc tctgcaga atg tca aac aaa gat cga 236

Met Ser Asn Lys Asp Arg

1               5

cac att gat tcc agc tgt tcg tcc ttc atc aag acg gaa cct tcc agc 284

His Ile Asp Ser Ser Cys Ser Ser Phe Ile Lys Thr Glu Pro Ser Ser

            10                  15                  20

cca gcc tcc ctg acg gac agc gtc aac cac cac agc cct ggt ggc tct 332

Pro Ala Ser Leu Thr Asp Ser Val Asn His His Ser Pro Gly Gly Ser

        25                  30                  35

tca gac gcc agt ggg agc tac agt tca acc atg aat ggc cat cag aac 380

Ser Asp Ala Ser Gly Ser Tyr Ser Ser Thr Met Asn Gly His Gln Asn

    40                  45                  50

gga ctt gac tcg cca cct ctc tac cct tct gct cct atc ctg gga ggt 428

Gly Leu Asp Ser Pro Pro Leu Tyr Pro Ser Ala Pro Ile Leu Gly Gly
55                  60                  65                  70

agt ggg cct gtc agg aaa ctg tat gat gac tgc tcc agc acc att gtt 476
Ser Gly Pro Val Arg Lys Leu Tyr Asp Asp Cys Ser Ser Thr Ile Val
                75                  80                  85

gaa gat ccc cag acc aag tgt gaa tac atg ctc aac tcg atg ccc aag 524
Glu Asp Pro Gln Thr Lys Cys Glu Tyr Met Leu Asn Ser Met Pro Lys
            90                  95                  100

aga ctg tgt tta gtg tgt ggt gac atc gct tct ggg tac cac tat ggg 572
Arg Leu Cys Leu Val Cys Gly Asp Ile Ala Ser Gly Tyr His Tyr Gly
        105                 110                 115

gta gca tca tgt gaa gcc tgc aag gca tct ttc aag agg aaa ata caa 620
Val Ala Ser Cys Glu Ala Cys Lys Ala Ser Phe Lys Arg Lys Ile Gln
    120                 125                 130

gcc aat ata gaa tac agc tgc cct gcc acg aat gaa tgt gaa atc aca 668
Ala Asn Ile Glu Tyr Ser Cys Pro Ala Thr Asn Glu Cys Glu Ile Thr
135                 140                 145                 150

aag cgc aga cgt aaa tcc tgc cag gct tgc cgc ttc atg aag tgt tta 716
Lys Arg Arg Arg Lys Ser Cys Gln Ala Cys Arg Phe Met Lys Cys Leu
                155                 160                 165

aaa gtg ggc atg ctg aaa gaa ggg gtg cgt ctt gac aga gta cgt gga 764
Lys Val Gly Met Leu Lys Glu Gly Val Arg Leu Asp Arg Val Arg Gly
            170                 175                 180

ggt cgg cag aag tac aag cgc agg ata gat gcg gag aac agc cca tac 812
Gly Arg Gln Lys Tyr Lys Arg Arg Ile Asp Ala Glu Asn Ser Pro Tyr
        185                 190                 195

ctg aac cct cag ctg gtt cag cca gcc aaa aag cca tat aac aag att 860
Leu Asn Pro Gln Leu Val Gln Pro Ala Lys Lys Pro Tyr Asn Lys Ile
    200                 205                 210

gtc tca cat ttg ttg gtg gct gaa ccg gag aag atc tat gcc atg cct 908
Val Ser His Leu Leu Val Ala Glu Pro Glu Lys Ile Tyr Ala Met Pro
215                 220                 225                 230

gac cct act gtc ccc gac agt gac atc aaa gcc ctc act aca ctg tgt 956
Asp Pro Thr Val Pro Asp Ser Asp Ile Lys Ala Leu Thr Thr Leu Cys
                235                 240                 245

gac tgt gcc gac cga gag ttg gtg gtt atc att gga tgg gcg aag cat 1004
Asp Cys Ala Asp Arg Glu Leu Val Val Ile Ile Gly Trp Ala Lys His
            250                 255                 260

atc cca ggc ttc tcc acg ctg tcc ctg gcg gac cag atg agc ctt ctg 1052

Ile Pro Gly Phe Ser Thr Leu Ser Leu Ala Asp Gln Met Ser Leu Leu
        265                 270                 275

cag agt gct tgg atg gaa att ttg atc ctt ggt ttc gta tac cgg tct 1100
Gln Ser Ala Trp Met Glu Ile Leu Ile Leu Gly Phe Val Tyr Arg Ser
    280                 285                 290

ctt tcg ttt gag gat gaa ctt gtc tat gca gac gat tat ata atg gac 1148
Leu Ser Phe Glu Asp Glu Leu Val Tyr Ala Asp Asp Tyr Ile Met Asp
295                 300                 305                 310

gaa gac cag tcc aaa tta gca ggc ctt ctt gat cta aat aat gct atc 1196
Glu Asp Gln Ser Lys Leu Ala Gly Leu Leu Asp Leu Asn Asn Ala Ile
                315                 320                 325

ctg cag ctg gta aag aaa tac aag agc atg aag ctg gaa aaa gaa gaa 1244
Leu Gln Leu Val Lys Lys Tyr Lys Ser Met Lys Leu Glu Lys Glu Glu
            330                 335                 340

ttt gtc acc ctc aaa gct ata gct ctt gct aat tca gac tcc atg cac 1292
Phe Val Thr Leu Lys Ala Ile Ala Leu Ala Asn Ser Asp Ser Met His
        345                 350                 355

ata gaa gat gtt gaa gcc gtt cag aag ctt cag gat gtc tta cat gaa 1340
Ile Glu Asp Val Glu Ala Val Gln Lys Leu Gln Asp Val Leu His Glu
    360                 365                 370

gcg ctg cag gat tat gaa gct ggc cag cac atg gaa gac cct cgt cga 1388
Ala Leu Gln Asp Tyr Glu Ala Gly Gln His Met Glu Asp Pro Arg Arg
375                 380                 385                 390

gct ggc aag atg ctg atg aca ctg cca ctc ctg agg cag acc tct acc 1436
Ala Gly Lys Met Leu Met Thr Leu Pro Leu Leu Arg Gln Thr Ser Thr
                395                 400                 405

aag gcc gtg cag cat ttc tac aac atc aaa cta gaa ggc aaa gtc cca 1484
Lys Ala Val Gln His Phe Tyr Asn Ile Lys Leu Glu Gly Lys Val Pro
            410                 415                 420

atg cac aaa ctt ttt ttg gaa atg ttg gag gcc aag gtc tgc taa 1529
Met His Lys Leu Phe Leu Glu Met Leu Glu Ala Lys Val Cys
        425                 430                 435

aagctccctg ggccttccat ccttcattgt tgaaaagggg aaataaaccc aagagtgatg 1589
tcgaagaaac ttagagttta gttaacaaca tcaaaaatca acagactgca ctgataattt 1649
agcagcaaga ctatgaagca gctttcagat tcctccatag gttcctgatg agtttctttc 1709
tactttctcc atcatcttct ttcctctttc ttcccacatt tctctttctc tttatttttt 1769
atccttttct tctttcacct cccttatttc tttgcttctt tcattcctag ttcccattct 1829
cctttatttt cttcccgtct gcctgccttc tttcttttct ttacctactc tcattcctct 1889
cttttctcat ccttcccctt ttttctaaat ttgaaatagc tttagtttaa aaaaaaatcc 1949
tcccttcccc ctttcctttc cctttctttc ctttttccct gtccttttcc ctttcctttc 2009
ctttcctctt gaccttcttt ccatctttct ttttcttcct tctgctgctg aacttttaaa 2069

agaggtctct aactgaagag agatggaagc cagccctgcc aaaggatgga gatccataat 2129

atggatgcca gtgaacttat tgtgaaccat accgtcccca atgactaagg aatcaaagag 2189

agagaaccaa cgttcctaaa agtacagtgc acatatacaa attgactgag tgcagtatta 2249

gatttcatgg gagcagcctc taattagaca acttaagcaa cgttgcatcg gctgcttctt 2309

atcattgctt ttccatctag agcagttaca gccatttgat cccttaattg tttttcaag 2369

tctcccaggt atttgttagt ttagctacta tgtaactttt tcagggaata gtttaagctt 2429

tattcagtca tgcaatacta aagagaaata agaatactgc aattttgtgc tggctttgaa 2489

caattacgaa caataatgaa ggacaaatga atcctgaagg aagattttta aaaatgtttt 2549

gtttcttctt acaaatggag atttttttgt accagcttta ccacttttca gccatttatt 2609

aatatgggaa tttaacttac tcaagcaata gttgaaggga aggtgcatat tatcacggtt 2669

gcaatttatg gttgtgtgcc cagtctggtc cccaaacatc aatttcttaa catgagctcc 2729

agtttaccta aatgttcact gacacaaagg atgagattac acctacagtg actctgagta 2789

gtcacatata taagcactgc acatgagata tagatccgta gaattgtcag gagtgcacct 2849

ctctacttgg gaggtacaat tgccatatga tttctagctg ccatggtggt taggaatgtg 2909

atacatgcct gtttgcaaag tcacagacca ttgcctcaga aggagctgtg agccagtatt 2969

catttaagag gcaataaggc aaatgccaga attaaaaaaa aaaaatcatc aaagacagaa 3029

aatgcctgac caaattctaa aacctaatcc atataagttt attcatttag gaatgttcgt 3089

ttaaattaat ctgcagtttt taccaagagc taagccaata tatgtgcttt tcaaccagta 3149

ttgtcacagc atgaaagtca cagtcaggtt ccagactgtt aagaggtgta atctaatgaa 3209

gaaatcaatt agatgccccg aaatctacag tcgctgaata accaataaac agtaacctcc 3269

atcaaatgct ataccaatgg accagtgtta gtagctgctc cctgtattat gtgaacagtc 3329

ttattctatg tacacagatg taattaaaat tgtaatccta acaaacaaaa gaaatgtagt 3389

tcagcttttc aatgtttcat gtttgctgtg cttttctgaa ttttatgttg cattcaaaga 3449

ctgttgtctt gttcttgtgg tgtttggatt cttgtggtgt gtgcttttag acacagggta 3509

gaattagaga caatattgga tgtacaattc ctcaggagac tacagtagta tattctattc 3569

cttaccagta ataaggttct tcctaataat aattaagaga ttgagactcc aaacaagtat 3629

tcattatgaa cagatacaca tcaaaatcat aataatattt tcagaacaag gaataatttc 3689

tctaatggtt tattatagaa taccaatgta tagcttagaa ataaaacttt gaatatttca 3749

agaatataga taagtctaat ttttaaatgc tgtatatatg gctttcactc aatcatctct 3809

cagatgttgt tattaactcg ctctgtgttg ttgcaaaact ttttggtgca gattcgtttc 3869

caaaactatt gctactttgt gtgctttaaa caaaatacct tgggttgatg aaacatcaac 3929

ccagtgctag gaatactgtg tatctatcat tagctatatg ggactatatt gtagattgtg 3989

gtttctcagt agagaagtga ctgtagtgtg attcttgata aatcatcatt agcaattcat 4049

tcagatggtc aataacttga aatttatagc tgtgatagga gttcagaaat tggcacatcc 4109

ctttaaaaat aacaacagaa aatacaactc ctgggaaaaa aaggtgctga ttctataaga 4169

ttatttatat atgtgagtgt ttaaaaagat tattttccag aaagtttgtg cagggtttaa 4229

gttgctacta ttcaactaca ctatatataa ataagatata tacaatatat acattgtttt 4289

cactgtatca cattaaagta cttgggcttc agaagtaaga agccaaccaa ctgaaaacct 4349

gagatggaga tatgttcaaa gaatgagata caatttttta gttttcagtt taagtaactc 4409

tcagcattac aaaagagtaa gtatctcaca aataggaaat aaaactaaaa cgtagattta 4469

aaaaagaact gcacgggctt tagggtaaat gctcatctta aacctcacta gagggaagtc 4529

ttctcaagtt tcaagcaaga ccatttactt aatgtgaagt tttggaaagt tataaaggtg 4589

tatgttttag ccatatgatc ctaaatttaa ttttgctctt ttaggttcgt tcttatttaa 4649

agcaatatga ttgtgtgact ccttgtagtt acacttgtgt ttcaatcaga tcagattgtt 4709

gtatttattc cactattttg catttaaatg ataacataac agatataaaa aatttaaaac 4769

tgctattttt cttatagaag agaaaatggg tgttggtgat tgtattttaa ttatttaagc 4829

gtctctgttt acctgcctag gaaaacattt tatggcagtc ttatgtgcaa agatcgtaaa 4889

aggacaaaaa atttaaactg cttataataa tccaggagtt gcattatagc cagtagtaaa 4949

aaaaataata ataataataa taaaaccatg tctatagctg tagatgggct tcacatctgt 5009

aaagcaatca attgtatatt tttgtgatgt gtaccatact gtgtgctcca gcaaatgtcc 5069

atttgtgtaa atgtatttat tttatattgt atatattgtt aaatgcaaaa aggagatatg 5129

attctgtaac tccaatcagt tcagatgtgt aacccaaata ttatgccttt caggatgatg 5189

gtagagcaat attaaacaag cttccacttt tg                                    5221

<210> 4

<211> 436

<212> PRT

<213> Homo sapiens

<400> 4

Met Ser Asn Lys Asp Arg His Ile Asp Ser Ser Cys Ser Ser Phe Ile

1               5                   10                  15

Lys Thr Glu Pro Ser Ser Pro Ala Ser Leu Thr Asp Ser Val Asn His

            20                  25                  30

His Ser Pro Gly Gly Ser Ser Asp Ala Ser Gly Ser Tyr Ser Ser Thr

        35                  40                  45

Met Asn Gly His Gln Asn Gly Leu Asp Ser Pro Pro Leu Tyr Pro Ser

    50                  55                  60

Ala Pro Ile Leu Gly Gly Ser Gly Pro Val Arg Lys Leu Tyr Asp Asp

65                  70                  75                  80

Cys Ser Ser Thr Ile Val Glu Asp Pro Gln Thr Lys Cys Glu Tyr Met

                85                  90                  95

Leu Asn Ser Met Pro Lys Arg Leu Cys Leu Val Cys Gly Asp Ile Ala

            100                 105                 110

Ser Gly Tyr His Tyr Gly Val Ala Ser Cys Glu Ala Cys Lys Ala Ser

        115                 120                 125

Phe Lys Arg Lys Ile Gln Ala Asn Ile Glu Tyr Ser Cys Pro Ala Thr

    130                 135                 140
Asn Glu Cys Glu Ile Thr Lys Arg Arg Arg Lys Ser Cys Gln Ala Cys
145                 150                 155                 160
Arg Phe Met Lys Cys Leu Lys Val Gly Met Leu Lys Glu Gly Val Arg
                165                 170                 175
Leu Asp Arg Val Arg Gly Gly Arg Gln Lys Tyr Lys Arg Arg Ile Asp
            180                 185                 190
Ala Glu Asn Ser Pro Tyr Leu Asn Pro Gln Leu Val Gln Pro Ala Lys
        195                 200                 205
Lys Pro Tyr Asn Lys Ile Val Ser His Leu Leu Val Ala Glu Pro Glu
    210                 215                 220
Lys Ile Tyr Ala Met Pro Asp Pro Thr Val Pro Asp Ser Asp Ile Lys
225                 230                 235                 240
Ala Leu Thr Thr Leu Cys Asp Cys Ala Asp Arg Glu Leu Val Val Ile
                245                 250                 255
Ile Gly Trp Ala Lys His Ile Pro Gly Phe Ser Thr Leu Ser Leu Ala
            260                 265                 270
Asp Gln Met Ser Leu Leu Gln Ser Ala Trp Met Glu Ile Leu Ile Leu
        275                 280                 285
Gly Phe Val Tyr Arg Ser Leu Ser Phe Glu Asp Glu Leu Val Tyr Ala
    290                 295                 300
Asp Asp Tyr Ile Met Asp Glu Asp Gln Ser Lys Leu Ala Gly Leu Leu
305                 310                 315                 320
Asp Leu Asn Asn Ala Ile Leu Gln Leu Val Lys Lys Tyr Lys Ser Met
                325                 330                 335
Lys Leu Glu Lys Glu Glu Phe Val Thr Leu Lys Ala Ile Ala Leu Ala

340                 345                 350

Asn Ser Asp Ser Met His Ile Glu Asp Val Glu Ala Val Gln Lys Leu

        355                 360                 365

Gln Asp Val Leu His Glu Ala Leu Gln Asp Tyr Glu Ala Gly Gln His

    370                 375                 380

Met Glu Asp Pro Arg Arg Ala Gly Lys Met Leu Met Thr Leu Pro Leu

385                 390                 395                 400

Leu Arg Gln Thr Ser Thr Lys Ala Val Gln His Phe Tyr Asn Ile Lys

                405                 410                 415

Leu Glu Gly Lys Val Pro Met His Lys Leu Phe Leu Glu Met Leu Glu

            420                 425                 430

Ala Lys Val Cys

        435

<210> 5

<211> 5216

<212> DNA

<213> Homo sapiens

<220>

<221> CDS

<222> (155)..(1531)

<400> 5

aagtccttga ttaggagagt gtgagagctt tggtcccaac tggctgtgcc tataggcttg 60

tcactaggag aacatttgtg ttaattgcac tgtgctctgt caaggaaact ttgatttata 120

gctggggtgc acaaataatg gttgccggtc gcac atg gat tcg gta gaa ctt tgc 175
Met Asp Ser Val Glu Leu Cys
1               5

ctt cct gaa tct ttt tcc ctg cac tac gag gaa gag ctt ctc tgc aga 223
Leu Pro Glu Ser Phe Ser Leu His Tyr Glu Glu Glu Leu Leu Cys Arg
        10                  15                  20

atg tca aac aaa gat cga cac att gat tcc agc tgt tcg tcc ttc atc 271
Met Ser Asn Lys Asp Arg His Ile Asp Ser Ser Cys Ser Ser Phe Ile
    25                  30                  35

aag acg gaa cct tcc agc cca gcc tcc ctg acg gac agc gtc aac cac 319
Lys Thr Glu Pro Ser Ser Pro Ala Ser Leu Thr Asp Ser Val Asn His
40                  45                  50                  55

cac agc cct ggt ggc tct tca gac gcc agt ggg agc tac agt tca acc 367
His Ser Pro Gly Gly Ser Ser Asp Ala Ser Gly Ser Tyr Ser Ser Thr
                60                  65                  70

atg aat ggc cat cag aac gga ctt gac tcg cca cct ctc tac cct tct 415
Met Asn Gly His Gln Asn Gly Leu Asp Ser Pro Pro Leu Tyr Pro Ser
            75                  80                  85

gct cct atc ctg gga ggt agt ggg cct gtc agg aaa ctg tat gat gac 463
Ala Pro Ile Leu Gly Gly Ser Gly Pro Val Arg Lys Leu Tyr Asp Asp
        90                  95                  100

tgc tcc agc acc att gtt gaa gat ccc cag acc aag tgt gaa tac atg 511
Cys Ser Ser Thr Ile Val Glu Asp Pro Gln Thr Lys Cys Glu Tyr Met
    105                 110                 115

ctc aac tcg atg ccc aag aga ctg tgt tta gtg tgt ggt gac atc gct 559
Leu Asn Ser Met Pro Lys Arg Leu Cys Leu Val Cys Gly Asp Ile Ala
120                 125                 130                 135

tct ggg tac cac tat ggg gta gca tca tgt gaa gcc tgc aag gca ttc 607
Ser Gly Tyr His Tyr Gly Val Ala Ser Cys Glu Ala Cys Lys Ala Phe
                140                 145                 150

ttc aag agg aca att caa ggc aat ata gaa tac agc tgc cct gcc acg 655
Phe Lys Arg Thr Ile Gln Gly Asn Ile Glu Tyr Ser Cys Pro Ala Thr
            155                 160                 165

aat gaa tgt gaa atc aca aag cgc aga cgt aaa tcc tgc cag gct tgc 703
Asn Glu Cys Glu Ile Thr Lys Arg Arg Arg Lys Ser Cys Gln Ala Cys
        170                 175                 180

cgc ttc atg aag tgt tta aaa gtg ggc atg ctg aaa gaa ggg gtg cgt 751
Arg Phe Met Lys Cys Leu Lys Val Gly Met Leu Lys Glu Gly Val Arg

    185                 190                 195

ctt gac aga gta cgt gga ggt cgg cag aag tac aag cgc agg ata gat 799
Leu Asp Arg Val Arg Gly Gly Arg Gln Lys Tyr Lys Arg Arg Ile Asp
200                 205                 210                 215

gcg gag aac agc cca tac ctg aac cct cag ctg gtt cag cca gcc aaa 847
Ala Glu Asn Ser Pro Tyr Leu Asn Pro Gln Leu Val Gln Pro Ala Lys
                220                 225                 230

aag cca tat aac aag att gtc tca cat ttg ttg gtg gct gaa ccg gag 895
Lys Pro Tyr Asn Lys Ile Val Ser His Leu Leu Val Ala Glu Pro Glu
            235                 240                 245

aag atc tat gcc atg cct gac cct act gtc ccc gac agt gac atc aaa 943
Lys Ile Tyr Ala Met Pro Asp Pro Thr Val Pro Asp Ser Asp Ile Lys
        250                 255                 260

gcc ctc act aca ctg tgt gac ttg gcc gac cga gag ttg gtg gtt atc 991
Ala Leu Thr Thr Leu Cys Asp Leu Ala Asp Arg Glu Leu Val Val Ile
    265                 270                 275

att gga tgg gcg aag cat att cca ggc ttc tcc acg ctg tcc ctg gcg 1039
Ile Gly Trp Ala Lys His Ile Pro Gly Phe Ser Thr Leu Ser Leu Ala
280                 285                 290                 295

gac cag atg agc ctt ctg cag agt gct tgg atg gaa att ttg atc ctt 1087
Asp Gln Met Ser Leu Leu Gln Ser Ala Trp Met Glu Ile Leu Ile Leu
                300                 305                 310

ggt gtc gta tac cgg tct ctt tcg ttt gag gat gaa ctt gtc tat gca 1135
Gly Val Val Tyr Arg Ser Leu Ser Phe Glu Asp Glu Leu Val Tyr Ala
            315                 320                 325

gac gat tat ata atg gac gaa gac cag tcc aaa tta gca ggc ctt ctt 1183
Asp Asp Tyr Ile Met Asp Glu Asp Gln Ser Lys Leu Ala Gly Leu Leu
        330                 335                 340

gat cta aat aat gct atc ctg cag ctg gta aag aaa tac aag agc atg 1231
Asp Leu Asn Asn Ala Ile Leu Gln Leu Val Lys Lys Tyr Lys Ser Met
    345                 350                 355

aag ctg gaa aaa gaa gaa ttt gtc acc ctc aaa gct ata gct ctt gct 1279
Lys Leu Glu Lys Glu Glu Phe Val Thr Leu Lys Ala Ile Ala Leu Ala
360                 365                 370                 375

aat tca gac tcc atg cac ata gaa gat gtt gaa gcc gtt cag aag ctt 1327
Asn Ser Asp Ser Met His Ile Glu Asp Val Glu Ala Val Gln Lys Leu
                380                 385                 390

cag gat gtc tta cat gaa gcg ctg cag gat tat gaa gct ggc cag cac 1375
Gln Asp Val Leu His Glu Ala Leu Gln Asp Tyr Glu Ala Gly Gln His

            395                 400                 405

atg gaa gac cct cgt cga gct ggc aag atg ctg atg aca ctg cca ctc 1423
Met Glu Asp Pro Arg Arg Ala Gly Lys Met Leu Met Thr Leu Pro Leu
        410                 415                 420

ctg agg cag acc tct acc aag gcc gtg cag cat ttc tac aac atc aaa 1471
Leu Arg Gln Thr Ser Thr Lys Ala Val Gln His Phe Tyr Asn Ile Lys
    425                 430                 435

cta gaa ggc aaa gtc cca atg cac aaa ctt ttt ttg gaa atg ttg gag 1519
Leu Glu Gly Lys Val Pro Met His Lys Leu Phe Leu Glu Met Leu Glu
440                 445                 450                 455

gcc aag gtc tga ctaaaagctc cctgggcctt cccatccttc atgttgaaaa 1571
Ala Lys Val

agggaaaata aacccaagag tgatgtcgaa gaaacttaga gtttagttaa caacatcaaa 1631
aatcaacaga ctgcactgat aatttagcag caagactatg aagcagcttt cagattcctc 1691
cataggttcc tgatgagttt ctttctactt tctccatcat cttctttcct ctttcttccc 1751
acatttctct ttctctttat tttttctcct tttcttcttt cacctccctt atttctttgc 1811
ttctttcatt cctagttccc attctccttt attttcttcc cgtctgcctg ccttctttct 1871
tttctttacc tactctcatt cctctctttt ctcatccttc cccttttttc taaatttgaa 1931
atagctttag tttaaaaaaa aatcctccct tccccctttc ctttcccttt ctttcctttt 1991
tccctttcct tttccctttc ctttcctttc ctcttgacct tctttccatc tttctttttc 2051
ttccttctgc tgctgaactt ttaaaagagg tctctaactg aagagagatg gaagccagcc 2111

ctgccaaagg atggagatcc ataatatgga tgccagtgaa cttattgtga accatactgt 2171
ccccaatgac taaggaatca aagagagaga accaacgttc ctaaaagtac agtgcaacat 2231
atacaaattg actgagtgca gtattagatt tcatgggagc agcctctaat tagacaactt 2291
aagcaacgtt gcatcggctg cttcttatca ttgcttttcc atctagatca gttacagcca 2351
tttgattcct taattgtttt ttcaagtctt ccaggtattt gttagtttag ctactatgta 2411
actttttcag ggaatagttt aagctttatt cattcatgca atactaaaga gaaataagaa 2471
tactgcaatt ttgtgctggc tttgaacaat tacgaacaat aatgaaggac aaatgaatcc 2531
tgaaggaaga tttttaaaaa tgttttgttt cttcttacaa atggagattt ttttgtacca 2591
gctttaccac ttttcagcca tttattaata tgggaattta acttactcaa gcaatagttg 2651
aagggaaggt gcatattatc acggatgcaa tttatgttgt gtgccagtct ggtcccaaac 2711
atcaatttct taacatgagc tccagtttac ctaaatgttc actgacacaa aggatgagat 2771
tacacctaca gtgactctga gtagtcacat atataagcac tgcacatgag atatagatcc 2831
gtagaattgt caggagtgca cctctctact tgggaggtac aattgccata tgatttctag 2891
ctgccatggt ggttaggaat gtgatactgc ctgtttgcaa agtcacagac cttgcctcag 2951
aaggagctgt gagccagtat tcatttaaga ggcaataagg caaatgccag aattaaaaaa 3011
aaaaatcatc aaagacagaa aatgcctgac caaattctaa aacctaatcc atataagttt 3071
attcatttag gaatgttcgt ttaaattaat ctgcagtttt taccaagagc taagccaata 3131
tatgtgcttt tcaaccagta ttgtcacagc atgaaagtca agtcaggttc cagactgtta 3191
agaggtgtaa tctaatgaag aaatcaatta gatgccccga aatctacagt cgctgaataa 3251
ccaataaaca gtaacctcca tcaaatgcta taccaatgga ccagtgttag tagctgctcc 3311
ctgtattatg tgaacagtct tattctatgt acacagatgt aattaaaatt gtaatcctaa 3371
caaacaaaag aaatgtagtt cagcttttca atgtttcatg tttgctgtgc ttttctgaat 3431
tttatgttgc attcaaagac tgttgtcttg ttcttgtggt gtttggattc ttgtggtgtg 3491
tgcttttaga cacagggtag aattagagac aatattggat gtacaattcc tcaggagact 3551
acagtagtat attctattcc ttaccagtaa taaggttctt cctaataata attaagagat 3611
tgaaactcca aacaagtatt cattatgaac agatacacat caaaatcata ataatatttt 3671

caaaacaagg aataatttct ctaatggttt attatagaat accaatgtat agcttagaaa 3731
taaaactttg aatatttcaa gaatatagat aagtctaatt tttaaatgct gtatatatgg 3791
ctttcactca atcatctctc agatgttgtt attaactcgc tctgtgttgt tgcaaaactt 3851
tttggtgcag attcgtttcc aaaactattg ctactttgtg tgctttaaac aaaatacctt 3911
gggttgatga aacatcaacc cagtgctagg aatactgtgt atctatcatt agctatatgg 3971
gactatattg tagattgtgg tttctcagta gagaagtgac tgtagtgtga ttctagataa 4031
atcatcatta gcaattcatt cagatggtca ataacttgaa atttatagct gtgataggag 4091
ttcagaaatt ggcacatccc tttaaaaata acaacagaaa atacaactcc tgggaaaaaa 4151
ggtgctgatt ctataagatt atttatatat gtaagtgttt aaaaagatta ttttccagaa 4211
agtttgtgca gggtttaagt tgctactatt caactacact atatataaat aaaatatata 4271
caatatatac attgttttca ctgtatcaca ttaaagtact tgggcttcag aagtaagagc 4331
caaccaactg aaaacctgag atggagatat gttcaaagaa tgagatacaa ttttttagtt 4391
ttcagtttaa gtaactctca gcattacaaa agagtaagta tctcacaaat aggaaataaa 4451
actaaaacgt ggatttaaaa agaactgcac gggctttagg gtaaatgctc atcttaaacc 4511
tcactagagg gaagtcttct caagtttcaa gcaagaccat ttacttaatg tgaagttttg 4571
gaaagttata aaggtgtatg ttttagccat atgattttaa ttttaatttt gcttctttta 4631
ggttcgttct tatttaaagc aatatgattg tgtgactcct tgtagttaca cttgtgtttc 4691
aatcagatca gattgttgta tttattccac tattttgcat ttaaatgata acataaaaga 4751
tataaaaaat ttaaaactgc tatttttctt atagaagaga aaatgggtgt tggtgattgt 4811
attttaatta tttaagcgtc tctgtttacc tgcctaggaa aacattttat ggcagtctta 4871
tgtgcaaaga tcgtaaaagg acaaaaaatt taaactgctt ataataatcc aggagttgca 4931
ttatagccag tagtaaaaat aataataata ataataaaac catgtctata gctgtagatg 4991
ggcttcacat ctgtaaagca atcaattgta tatttttgtg atgtgtacca tactgtgtgc 5051
tccagcaaat gtccatttgt gtaaatgtat ttattttata ttgtatatat tgttaaatgc 5111
aaaaaggaga tatgattctg taactccaat cagttcagat gtgtaactca aattattatg 5171
cctttcagga tgatggtaga gcaatattaa acaagcttcc acttt 5216

<210> 6

<211> 458

<212> PRT

<213> Homo sapiens

<400> 6

Met Asp Ser Val Glu Leu Cys Leu Pro Glu Ser Phe Ser Leu His Tyr
1               5                   10                  15

Glu Glu Glu Leu Leu Cys Arg Met Ser Asn Lys Asp Arg His Ile Asp
            20                  25                  30

Ser Ser Cys Ser Ser Phe Ile Lys Thr Glu Pro Ser Ser Pro Ala Ser
        35                  40                  45

Leu Thr Asp Ser Val Asn His His Ser Pro Gly Gly Ser Ser Asp Ala
    50                  55                  60

Ser Gly Ser Tyr Ser Ser Thr Met Asn Gly His Gln Asn Gly Leu Asp
65                  70                  75                  80

Ser Pro Pro Leu Tyr Pro Ser Ala Pro Ile Leu Gly Gly Ser Gly Pro
                85                  90                  95

Val Arg Lys Leu Tyr Asp Asp Cys Ser Ser Thr Ile Val Glu Asp Pro
            100                 105                 110

Gln Thr Lys Cys Glu Tyr Met Leu Asn Ser Met Pro Lys Arg Leu Cys
        115                 120                 125

Leu Val Cys Gly Asp Ile Ala Ser Gly Tyr His Tyr Gly Val Ala Ser
    130                 135                 140

Cys Glu Ala Cys Lys Ala Phe Phe Lys Arg Thr Ile Gln Gly Asn Ile
145                 150                 155                 160
Glu Tyr Ser Cys Pro Ala Thr Asn Glu Cys Glu Ile Thr Lys Arg Arg
                165                 170                 175
Arg Lys Ser Cys Gln Ala Cys Arg Phe Met Lys Cys Leu Lys Val Gly
            180                 185                 190
Met Leu Lys Glu Gly Val Arg Leu Asp Arg Val Arg Gly Gly Arg Gln
        195                 200                 205
Lys Tyr Lys Arg Arg Ile Asp Ala Glu Asn Ser Pro Tyr Leu Asn Pro
    210                 215                 220
Gln Leu Val Gln Pro Ala Lys Lys Pro Tyr Asn Lys Ile Val Ser His
225                 230                 235                 240
Leu Leu Val Ala Glu Pro Glu Lys Ile Tyr Ala Met Pro Asp Pro Thr
                245                 250                 255
Val Pro Asp Ser Asp Ile Lys Ala Leu Thr Thr Leu Cys Asp Leu Ala
            260                 265                 270
Asp Arg Glu Leu Val Val Ile Ile Gly Trp Ala Lys His Ile Pro Gly
        275                 280                 285
Phe Ser Thr Leu Ser Leu Ala Asp Gln Met Ser Leu Leu Gln Ser Ala
    290                 295                 300
Trp Met Glu Ile Leu Ile Leu Gly Val Val Tyr Arg Ser Leu Ser Phe
305                 310                 315                 320
Glu Asp Glu Leu Val Tyr Ala Asp Asp Tyr Ile Met Asp Glu Asp Gln
                325                 330                 335
Ser Lys Leu Ala Gly Leu Leu Asp Leu Asn Asn Ala Ile Leu Gln Leu
            340                 345                 350

Val Lys Lys Tyr Lys Ser Met Lys Leu Glu Lys Glu Glu Phe Val Thr
        355                 360                 365
Leu Lys Ala Ile Ala Leu Ala Asn Ser Asp Ser Met His Ile Glu Asp
    370                 375                 380
Val Glu Ala Val Gln Lys Leu Gln Asp Val Leu His Glu Ala Leu Gln
385                 390                 395                 400
Asp Tyr Glu Ala Gly Gln His Met Glu Asp Pro Arg Arg Ala Gly Lys
                405                 410                 415
Met Leu Met Thr Leu Pro Leu Leu Arg Gln Thr Ser Thr Lys Ala Val
            420                 425                 430
Gln His Phe Tyr Asn Ile Lys Leu Glu Gly Lys Val Pro Met His Lys
        435                 440                 445
Leu Phe Leu Glu Met Leu Glu Ala Lys Val
    450                 455

<210> 7
<211> 30
<212> RNA
<213> Artificial Sequence

<220>
<223> Description of Artificial Sequence:an artificially
synthesized oligo-cap linker sequence

<400> 7

agcaucgagu cggccuuguu ggccuacugg                                    30

<210> 8
<211> 42
<212> DNA
<213> Artificial Sequence

<220>
<223> Description of Artificial Sequence:an artificially
      synthesized oligo(dT) primer sequence

<400> 8
gcggctgaag acggcctatg tggccttttt tttttttttt tt                      42

<210> 9
<211> 21
<212> DNA
<213> Artificial Sequence

<220>
<223> Description of Artificial Sequence:an artificially
      synthesized primer sequence

<400> 9

agcatcgagt cggccttgtt g                                                21

<210> 10
<211> 21
<212> DNA
<213> Artificial Sequence

<220>
<223> Description of Artificial Sequence:an artificially
      synthesized primer sequence

<400> 10
gcggctgaag acggcctatg t                                                21

<210> 11
<211> 23
<212> DNA
<213> Artificial Sequence

<220>
<223> Description of Artificial Sequence:an artificially
      synthesized primer sequence

<400> 11

ccactgagaa agggaataag gct 23

<210> 12

<211> 24

<212> DNA

<213> Artificial Sequence

<220>

<223> Description of Artificial Sequence:an artificially

synthesized primer sequence

<400> 12

tgttatatgg ctttttggct ggct 24

<210> 13

<211> 19

<212> DNA

<213> Artificial Sequence

<220>

<223> Description of Artificial Sequence:an artificially

synthesized primer sequence

<400> 13
   ctttttccct gcactacga                                                    19

<210> 14

<211> 19

<212> DNA

<213> Artificial Sequence

<220>

<223> Description of Artificial Sequence:an artificially

      synthesized primer sequence

<400> 14

gacctccacg tactctgtc                                                    19

<210> 15

<211> 21

<212> DNA

<213> Artificial Sequence

<220>

<223> Description of Artificial Sequence:an artificially

      synthesized sequence

<400> 15

gatctaggtc acagtgacct a                                                    21

<210> 16

<211> 21

<212> DNA

<213> Artificial Sequence

<220>

<223> Description of Artificial Sequence:an artificially

      synthesized sequence

<400> 16

gatctaggtc actgtgacct a                                                    21

<210> 17

<211> 26

<212> DNA

<213> Artificial Sequence

<220>

<223> Description of Artificial Sequence:an artificially

      synthesized primer sequence

<400> 17

cgcggatcct ctgcagaatg tcaaac 26

<210> 18

<211> 23

<212> DNA

<213> Artificial Sequence

<220>

<223> Description of Artificial Sequence:an artificially

synthesized primer sequence

<400> 18

cgcctcgagg aaagaaactc agg 23

<210> 19

<211> 27

<212> DNA

<213> Artificial Sequence

<220>

<223> Description of Artificial Sequence:an artificially

synthesized primer sequence

<400> 19

tttggatcct cgcacatgga ttcggta 27

<210> 20

<211> 27

<212> DNA

<213> Artificial Sequence

<220>

<223> Description of Artificial Sequence:an artificially

synthesized primer sequence

<400> 20

gggctcgaga agaaagagga aagaaga 27

<210> 21

<211> 20

<212> DNA

<213> Artificial Sequence

<220>

<223> Description of Artificial Sequence:an artificially

synthesized primer sequence

<400> 21

aagcctgcaa ggcattcttc 20

<210> 22
<211> 18
<212> DNA
<213> Artificial Sequence

<220>
<223> Description of Artificial Sequence:an artificially
      synthesized primer sequence

<400> 22
cgacctccac gcactctg                                                    18

<210> 23
<211> 26
<212> DNA
<213> Artificial Sequence

<220>
<223> Description of Artificial Sequence:an artificially
      synthesized TaqMan probe sequence

<220>
<221> misc_binding

<222> (1)

<223> Label FAM(6-carboxy-fluorescein)

<220>

<221> misc_binding

<222> (26)

<223> Label TAMRA(6-carboxy-N,N,N',N'-tetramethylrhodamine)

<400> 23

aggacgattc aaggggtccg tcttga 26